# EUROPEAN PATENT APPLICATION

(11) **EP 4 357 351 A1**
(43) Date of publication of application: **24.04.2024**
(21) Application number: 22825110.4
(22) Date of filing: 24.01.2022
(51) Int. Cl.: C07F 9/54, A61K 31/662, A61K 31/675, A61P 35/00, A61K 31/665, A61P 27/02

(54) **TRAP1 INHIBITOR AND USE THEREOF**

(30) Priority: 15.06.2021 KR 20210077326; 15.06.2021 KR 20210077327
(71) Applicant: Smartin Bio Inc., Chungcheongbuk-do 28160 (KR); Ulsan National Institute of Science and Technology (UNIST), Eonyang-eup Ulju-gun Ulsan 44919 (KR)
(72) Inventor: KANG, Byoung Heon, Ulju-gun Ulsan 44920 (KR); KIM, So-Yeon, Ulju-gun Ulsan 44920 (KR); YOON, Nam Gu, Suwon-si Gyeonggi-do 16295 (KR)
(74) Representative: Schön, Christoph
(86) International application number: PCT/KR2022/001195
(87) International publication number: WO 2022/265178

(57) **Abstract**

The present application provides a compound that inhibits TRAP1 or a pharmaceutically acceptable salt thereof. In this case, the compound or the pharmaceutically acceptable salt thereof can inhibit the binding between TRAP1 and the client protein. In addition, the present application provides: a pharmaceutical composition for the treatment of cancer or ocular disease comprising a compound that inhibits TRAP1 or a pharmaceutically acceptable salt thereof; and use thereof.

## Description

### Technical Field

The present application relates to a compound for inhibiting TRAP1 or a pharmaceutically acceptable salt thereof and a use thereof, and specifically, to a TRAP1 inhibitor and a composition comprising the same for treating cancer or ocular diseases.

### Background Art

Tumor necrosis factor receptor-associated protein-1 (TRAP1) is a paralog of heat shock protein-90 (Hsp90), a chaperone protein, and is a mitochondrial protein present only in mitochondria. The inventors of the present disclosure synthesized compounds for inhibiting TRAP1 and recognized a use of the compound as a pharmaceutical composition for inhibiting TRAP1 and further treating ocular diseases using the compound.

### Disclosure

### Technical Problem

In one embodiment, provided herein is a compound for inhibiting TRAP1 or a pharmaceutically acceptable salt thereof.

In another embodiment, provided herein is a compound for inhibiting the binding between TRAP1 and the client protein or a pharmaceutically acceptable salt thereof.

In a further embodiment, provided herein is a pharmaceutical composition comprising a compound for inhibiting TRAP1 or a pharmaceutically acceptable salt thereof.

In yet another embodiment, provided herein is a method of treating a disease, the method including administering a pharmaceutical composition comprising a compound for inhibiting TRAP1 or a pharmaceutically acceptable salt thereof, to a subject in need thereof.

For example, provided herein is a therapeutic use of a compound for inhibiting TRAP1 or a pharmaceutically acceptable salt thereof for ocular diseases.

For example, provided herein is a composition for treating ocular diseases, the composition comprising a compound for inhibiting TRAP1 or a pharmaceutically acceptable salt thereof.

For example, provided herein is a use of a compound for inhibiting TRAP1 or a pharmaceutically acceptable salt thereof to prepare a composition for treating ocular diseases.

For example, provided herein is a method of treating ocular diseases, the method including administering a compound for inhibiting TRAP1 or a pharmaceutically acceptable salt thereof to a subject in need thereof.

### Technical Solution

Provided herein is a compound represented by following Formula 1 or a pharmaceutically acceptable salt thereof and a pharmaceutical composition comprising the same for treating cancer or ocular diseases:

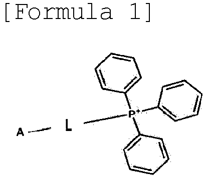

wherein, L comprises (CH₂)ₙ,
n is an integer of 7 or more and 40 or less, and
A is selected from methyl, unsubstituted or substituted aryl, unsubstituted or substituted cycloalkyl, and unsubstituted or substituted heterocyclyl.

In this case, wherein A is selected from aryl, cycloalkyl, and heterocyclyl that are unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

In this case, wherein A is unsubstituted or substituted aryl, and
wherein the unsubstituted or substituted aryl is phenyl unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

In this case, wherein A is selected from the group consisting of

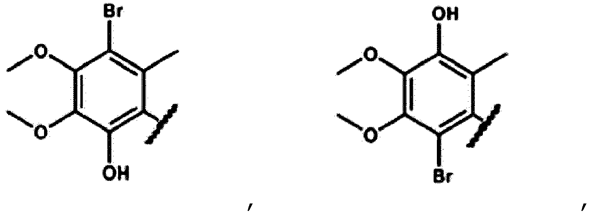

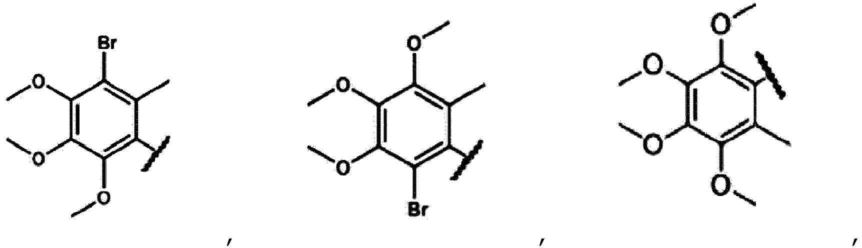

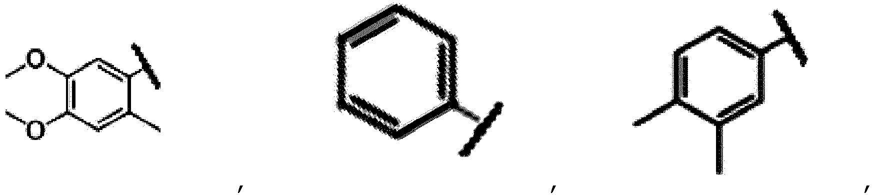

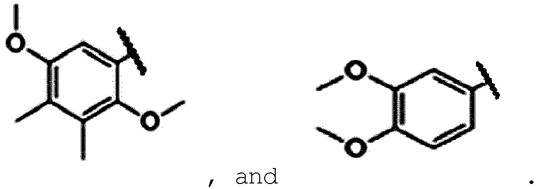

In this case, wherein A is unsubstituted or substituted aryl, and
wherein the unsubstituted or substituted aryl is naphthalene unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

In this case, wherein A is

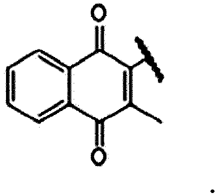

In this case, wherein A is unsubstituted or substituted aryl, and
wherein the unsubstituted or substituted aryl is benzodioxole unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

In this case, wherein A is 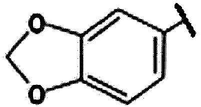 or

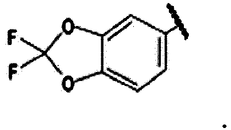

In this case, wherein A is unsubstituted or substituted cycloalkyl, and
wherein the unsubstituted or substituted cycloalkyl is cyclohexyl unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

In this case, wherein A is unsubstituted cyclohexyl.

In this case, wherein A is unsubstituted or substituted heterocyclyl, and
wherein the unsubstituted or substituted heterocyclyl is pyrrolidine or chromane unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

In this case, wherein A is pyrrolidine substituted with one or more selected from oxycarbonyl, C1-5 alkyl, and =O.

In this case, wherein A is

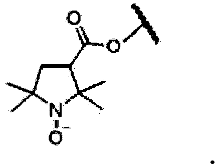

In this case, wherein A is chroman-2-yl substituted with one or more selected from C1-5 alkyl and hydroxyl.

In this case, wherein A is

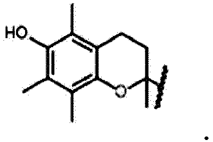

In this case, wherein n is an integer of 9 or more.

In this case, provided is a method of treating cancer or ocular disease, the method comprisiong administering the pharmaceutical composition to a subject in need thereof.

In this case, wherein the pharmaceutical composition is administered orally.

In this case, wherein the pharmaceutical composition is administered topically using an eye drop.

In this case, provided is a TRAP1 inhibitor comprising the compound represented by above Formula 1 or the pharmaceutically acceptable salt thereof.

In this case, wherein the TRAP1 inhibitor binds to CBS.

In this case, provided is a TRAP1-client protein binding inhibitor comprising the compound represented by above Formula 1 or the pharmaceutically acceptable salt thereof.

### Advantageous Effects

TRAP1 can be inhibited using a compound provided herein or a pharmaceutically acceptable salt thereof.

A binding between TRAP1 and client protein can be inhibited using the compound provided herein or the pharmaceutically acceptable salt thereof.

A pharmaceutical composition comprising the compound provided herein or the pharmaceutically acceptable salt thereof can be used to treat specific diseases related to the mechanism of TRAP1. For example, the pharmaceutical composition comprising the compound provided herein or the pharmaceutically acceptable salt thereof can be used to treat cancer or ocular diseases.

The compound provided herein is advantageous in enabling non-invasive treatments, such as eye drop administration, without showing side effects on normal cells by selectively inhibiting TRAP1.

### Description of Drawings

FIG. 1A shows an overall structure of the zTRAP1 dimer bound to MitoQ and AMPPNP, where protomers A and B are shown in light gray and dark gray, respectively.
FIG. 1B shows the recognition of MitoQ by TRAP1 in detail, showing that sidechains of residues interact with MitoQ, where Fo-Fc maps (grey mesh) are calculated in the absence of MitoQ.
FIG. 1C shows a MitoQ-binding pocket structure, where the images above show a Ub-binding pocket, and the images below show a TPP-binding pocket.
FIG. 2 shows a crystal structure in which TRAP1 and MitoQ are bound.
FIG. 3 illustrates a structure of alkyl-TPPs.
FIG. 4 shows analysis results of the binding capacity of alkyl-TPPs to client-binding sites (CBS) in TRAP1.
FIG. 5 illustrates structures of antioxidant-TPP conjugates.
FIG. 6 shows analysis results of the binding capacity of antioxidant-TPP conjugates to CBS in TRAP1.
FIG. 7 illustrates structures of other synthetic compounds.
FIGS. 8A and 8B each independently show analysis results of the binding capacity of other synthetic compounds to CBS in TRAP1.
FIG. 9A shows analysis results of the TRAP1 ATPase activity of alkyl-TPPs.
FIG. 9B shows analysis results of the binding capacity of alkyl-TPPs to the ATP-binding pocket of TRAP1, where mP stands for millipolarization.
FIG. 10 shows results of inhibiting TRAP1 and Hsp90 in cancer cells.
FIG. 11A shows analysis results of the TRAP1 ATPase activity depending on the length of linkers.
FIB. 11B shows analysis results of millipolarization (mP) depending on the length of linkers.
FIG. 12 shows effects of antioxidant-TPP conjugates on cells.
FIG. 13 shows effects of MitoQS, Mito-VitEL, and Mito-CPS on Hsp90 and TRAP1.
FIG. 14 shows analysis results of the TRAP1 ATPase activity of other synthetic compounds.
FIG. 15 shows results of inhibiting TRAP1 by other synthetic compounds.
FIG. 16 shows results of in vivo tumor growth inhibition effects of other synthetic compounds.
FIG. 17 shows western blot results of tumors.
FIGS. 18 and 19 show increases in TRAP1 expression in a diabetic retinopathy model.
FIG. 18A shows western blot analysis results of the OIR retina compared to the age-matched room-air retina, where TRAP1 protein levels are normalized to β-actin.
FIG. 18B shows qPCR analysis results of TRAP1 expression in the room-air and OIR retinas.
FIG. 19A shows western blot analysis results of the retina of STZ-DM compared to that of an age-matched control group.
FIG. 19B shows qPCR analysis results of TRAP1 expression in the retina of STZ-DM compared to that of a control group.

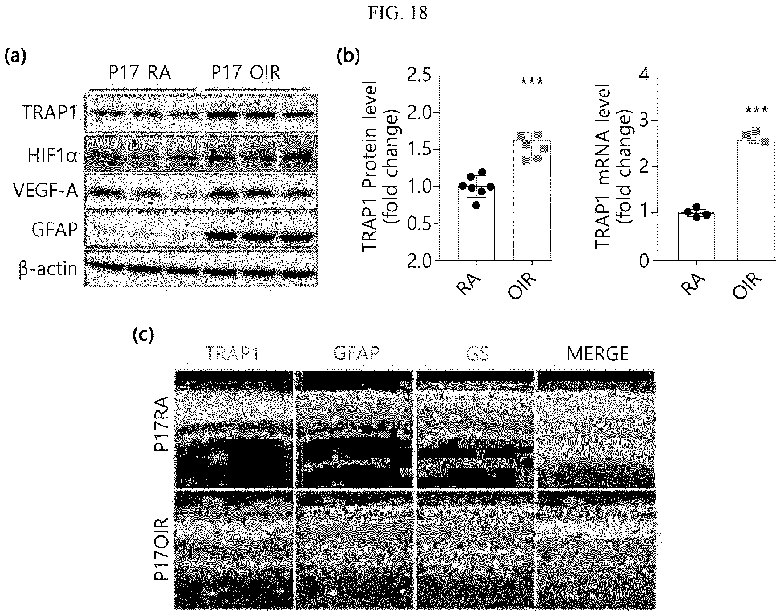

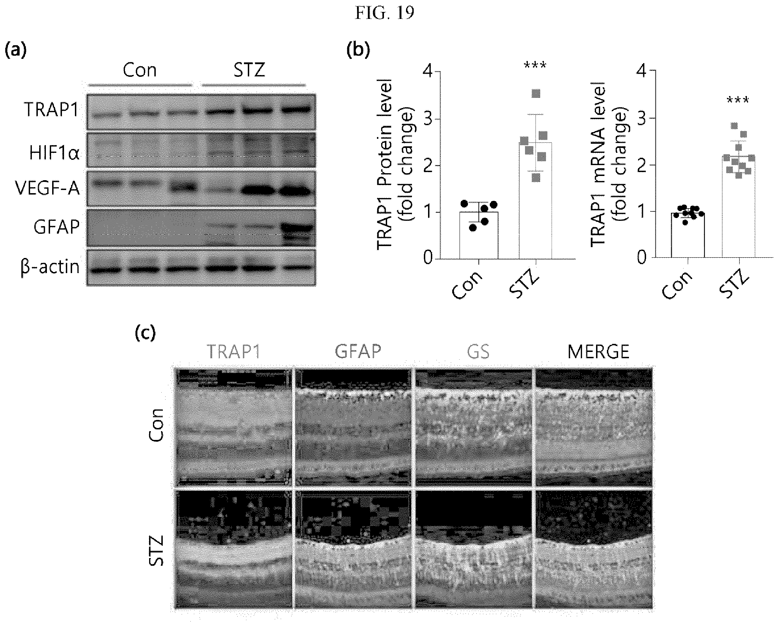

FIGS. 18C and 19C show TRAP1, glial fibrillary acidic protein (GFAP), glutamine synthetase (GS), and DAPI (gray in the middle of Merge images) staining results of the retinas of STZ-DM and age-matched control group or OIR and room-air retinas.

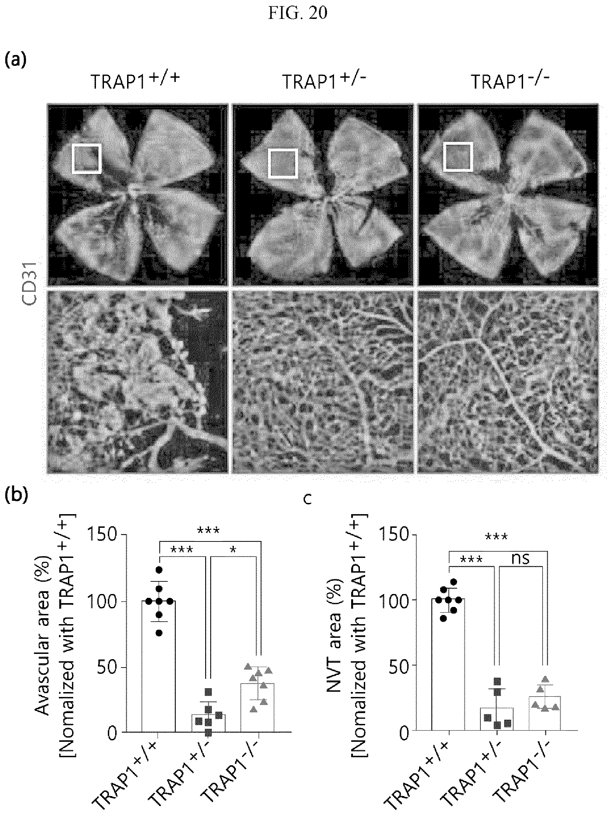

FIG. 20 shows results of TRAP1 knockout deteriorating pathological retinal neovascularization in an OIR model.
FIG. 20A shows whole-mount staining results of the P17 OIR retina using CD31.
FIGS. 20B and 20C show quantification results of new vascular bundles and avascular areas normalized to TRAP1+/+.

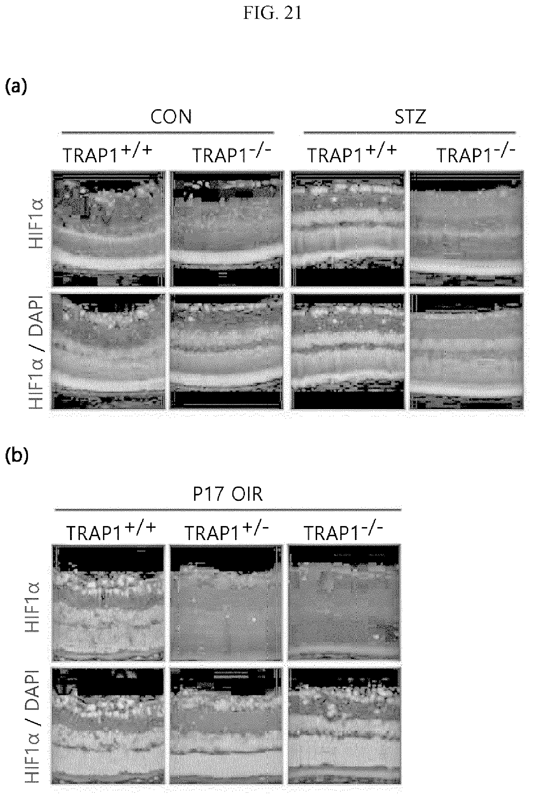

FIG. 21A shows HIF1α immunofluorescence staining results of the STZ mouse retina.
FIG. 21B shows HIF1α immunofluorescence staining results of the P17 OIR mouse retina.

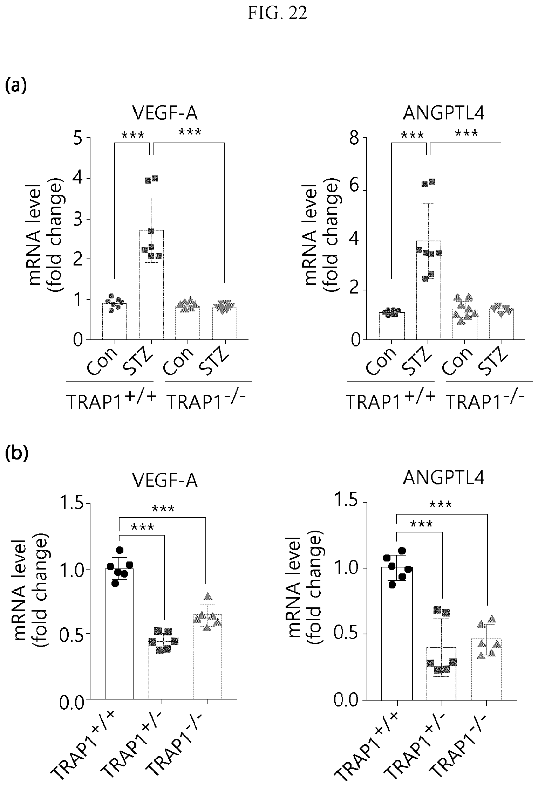

FIG. 22A shows qPCR analysis results of VEGF-A and ANGPTL4 expressions in the STZ-DM retina.
FIG. 22B shows qPCR analysis results of VEGF-A and ANGPTL4 expressions in the P17 OIR retina.

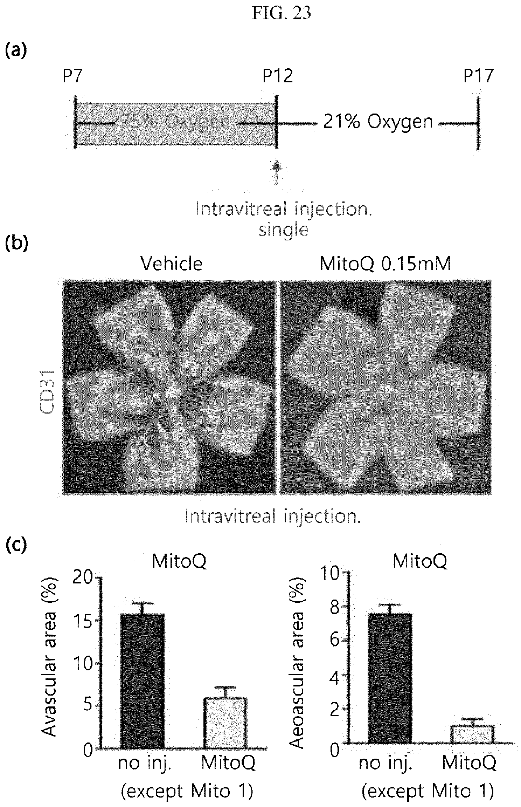

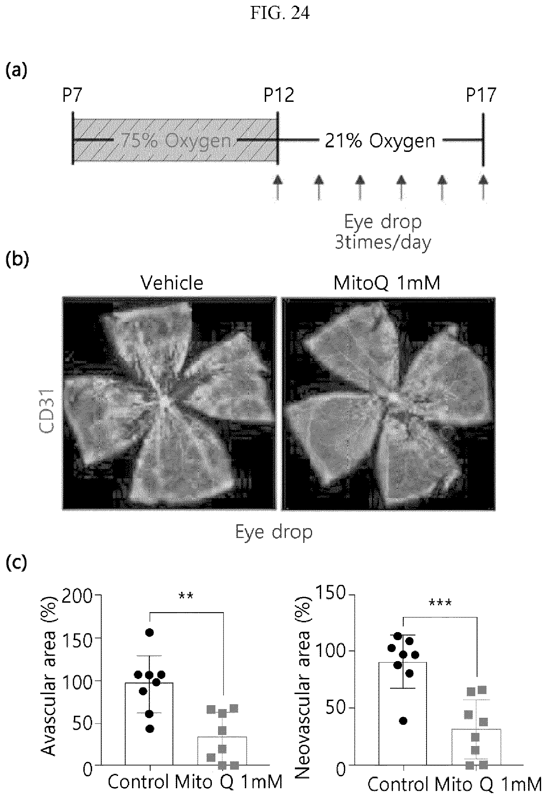

FIGS. 23A and 24A illustrate intravitreal injection or eye drop administration of MitoQ to OIR mice at P12 to examine the HIF1α inhibitory activity of MitoQ in vivo.
FIGS. 23B and 24B show whole-mount staining results of the retina using CD31.
FIGS. 23C and 24C show the ratio of avascular and neovascular areas in the OIR retina according to MitoQ administration.

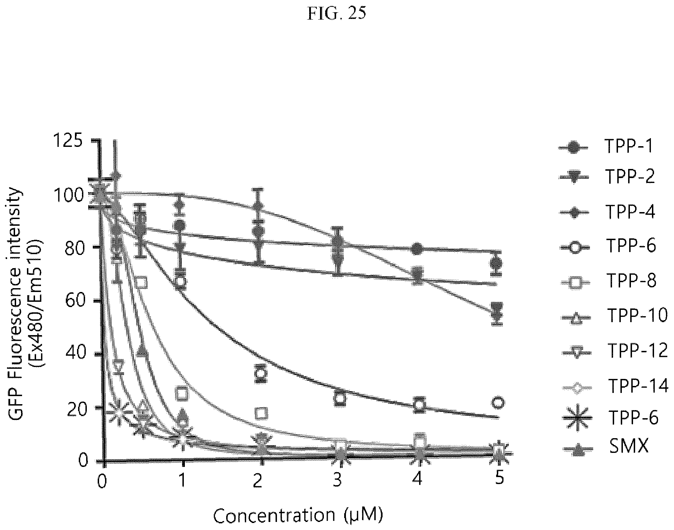

FIG. 25 shows analysis results of the HIF1α inhibitory activity of alkyl-TPPs using an MIO-MI HRE GFP cell line.

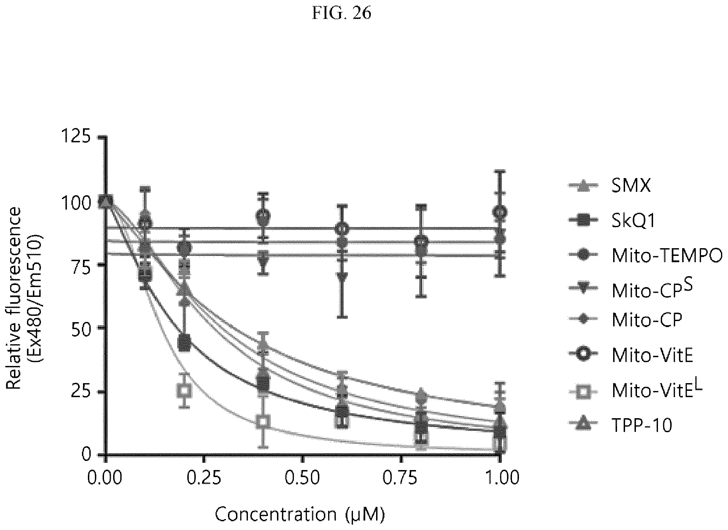

FIG. 26 shows analysis results of the HIF1α inhibitory activity of TPP-antioxidant conjugates using an MIO-MI HRE GFP cell line.

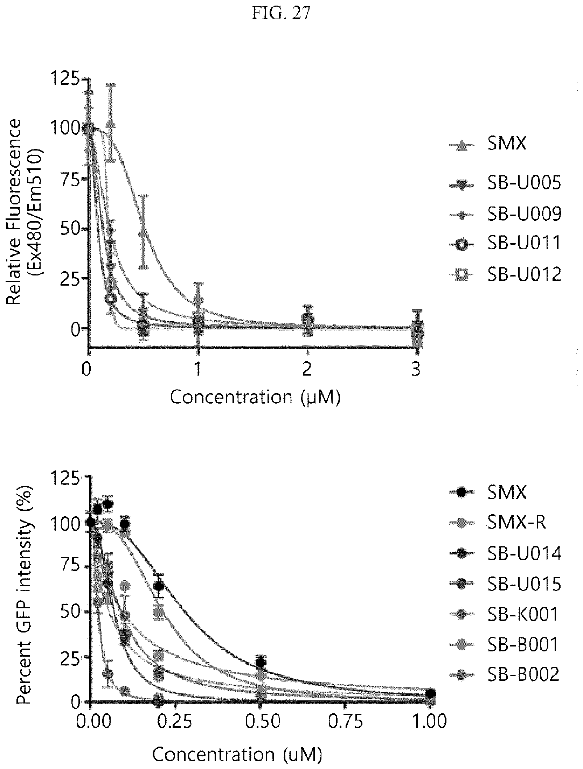

FIG. 27 shows analysis results of the HIF1α inhibitory activity of other synthetic compounds using an MIO-MI HRE GFP cell line.

### Best Mode

### Definition

Unless otherwise defined, all technical and scientific terms used herein have the same meaning as commonly understood by those skilled in the art to which the present disclosure pertains. All publications, patent documents, and other references mentioned herein are incorporated by reference in their entirety.

Hereinbelow, specific details of the present disclosure are to be disclosed.

### TRAP1

TRAP1, one of the paralogs of Hsp90, is a mitochondrial protein present only in mitochondria. Hsp90, including the TRAP1, has a structure in which two protomers are combined. In this case, the respective protomers are called first and second promoters. When bound to the client protein specific to Hsp90, the protomers departing from each other come closer. Additionally, when ATP binds thereto, Hsp90 is cleaved and thus functions to form a three-dimensional structure of the client protein. Each protomer is composed of an N-terminal domain, a middle domain, and a C-terminal domain. The N-terminal domain is a site where ATP is bound to produce energy for the activity of Hsp90s. The middle domain is a site where the client proteins specific to the respective Hsp90s bind. The C-terminal domain is a site where two protomers are linked. Hsp90s are characterized in that homology between paralogs is significantly high in the N-terminal domain but is low in the middle domain. Accordingly, while compounds targeting the N-terminal domain may act non-selectively on Hsp90 paralogs, compounds targeting the domain with low homology may act selectively on Hsp90 paralogs. Unless otherwise specified, the Hsp90 includes Hsp90s present in the cytoplasm, such as Hsp90-α1, Hsp90-α2, and Hsp90-β.

Additionally, it is known that when the TRAP1 is inhibited, the expression of SIRT3 and SDHB is reduced (see Interplay between TRAP1 and Sirtuin-3 Modulates Mitochondrial Respiration and Oxidative Stress to Maintain Sternness of Glioma Stem Cells. Cancer Res 79, 1369-1382). Accordingly, to confirm whether TRAP1 is inhibited, the expression of SIRT3 and SDHB may be examined herein.

### Aryl

The term "aryl" used herein includes unsubstituted or substituted monocyclic aromatic groups where each atom of the ring is carbon. The ring preferably is a 5- to 7-membered ring, and more preferably is a 6-membered ring. The term "aryl" also includes a polycyclic ring system having two or more cyclic rings where two adjacent rings share two or more carbons and at least one of the rings is aromatic. Additionally, the other cyclic rings may, for example, be a cycloalkyl, a cycloalkenyl, a cycloalkynyl, an aryl, a heteroaryl, and/or a heterocyclyl. Aryl groups include benzene, naphthalene, phenanthrene, phenol, aniline, and the like.

### Cycloalkyl

A "cycloalkyl" group is a fully saturated cyclic hydrocarbon. "Cycloalkyl" includes monocyclic and bicyclic rings. Unless otherwise defined, a monocyclic cycloalkyl group typically has 3 to about 10 carbon atoms, and more typically has 3 to 8 carbon atoms. The second ring of a bicyclic cycloalkyl may be selected from saturated, unsaturated, and aromatic rings. The cycloalkyl, a bicyclic molecule, includes compounds where 1, 2, 3, or more atoms are shared between two rings.

### Heterocyclyl

The term "heterocyclyl" means an unsubstituted or substituted non-aromatic ring structure, which preferably is a 3- to 10-membered ring and more preferably is a 3- to 7-membered ring. This ring structure contains at least one heteroatom, preferably contains 1 to 4 heteroatoms, and more preferably contains 1 or 2 heteroatoms. The terms "heterocyclyl" and "heterocyclic" also include a polycyclic ring system having two or more cyclic rings where two adjacent rings share two or more carbons and at least one of the rings is heterocyclic. Additionally, the other cyclic rings may, for example, be a cycloalkyl, a cycloalkenyl, a cycloalkynyl, an aryl, a heteroaryl, and/or a heterocyclyl. Heterocyclyl groups include, for example, piperidine, piperazine, pyrrolidine, morpholine, lactone, lactam, and the like.

### Hydroxyalkyl

"Hydroxyalkyl", an alkyl group having at least one hydroxy substituent, means, for example, a straight-chain monovalent hydrocarbon radical containing 1 to 6 carbon atoms substituted with one or two hydroxy groups, or a branched-chain monovalent hydrocarbon radical containing 3 to 6 carbon atoms. However, in the presence of two hydroxy groups, both the two are not present on the same carbon atom. Specifically, examples thereof include hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl, 2-(hydroxymethyl)-3-hydroxypropyl, and the like.

### Ocular diseases

"Ocular diseases" used herein include ocular neovascularization diseases characterized by choroidal neovascularization diseases, retinal neovascularization diseases, subretinal neovascularization diseases, corneal neovascularization diseases, iris neovascularization diseases, or neovascular glaucoma. In addition, the ocular neovascularization diseases may refer to retinal neovascularization diseases, and the retinal neovascularization diseases may mean ocular neovascularization diseases characterized by diabetic retinopathy, retinopathy of prematurity, or retinal vein occlusion. Additionally, the choroidal neovascularization diseases may mean wet age-related macular degeneration (wet AMD).

### Pharmaceutically acceptable

The term "pharmaceutically acceptable" used herein refers to a compound, substance, composition, and/or dosage form being suitable for making contact with the target's tissue within a scope of reasonable medical judgment without causing excessive toxicity, irritation, allergic reaction, other problems, or side effects, and having a reasonable benefit/risk ratio.

### I. Compound of Formula 1

### 1. Formula 1

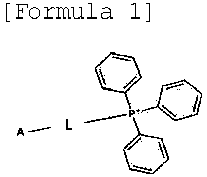

Provided herein is a compound having a structure of Formula 1 described above.

A may be selected from methyl, an unsubstituted or substituted aryl, an unsubstituted or substituted cycloalkyl, and an unsubstituted or substituted heterocyclyl.
L comprises (CH₂)ₙ, and
in (CH₂)ₙ, n may be an integer of 7 or more and 10 or less, 7 or more and 20 or less, 7 or more and 30 or less, 7 or more and 40 or less, or 7 or more and 50 or less. In a specific example, n of the (CH₂)ₙ may be an integer of 7 or more and 40 or less, but is not limited thereto. L herein may be understood as a concept corresponding to a connector or linker.

### 2. Structure of A

### 1) Methyl

In one example, A may be methyl.

### 2) Unsubstituted or substituted aryl

In one example, A may be an unsubstituted or substituted aryl. In a specific example, A may be an aryl unsubstituted or substituted with one or more selected from a halogen, =O, hydroxy, oxycarbonyl, hydroxy C1-5 alkyl, C1-5 alkyl, C1-5 alkenyl, C1-5 alkynyl, and C1-5 alkoxy. In a more specific example, A may be a phenyl unsubstituted or substituted with one or more selected from halogen, =O, hydroxy, oxycarbonyl, hydroxy C1-5 alkyl, C1-5 alkyl, C1-5 alkenyl, C1-5 alkynyl, and C1-5 alkoxy. In a more specific example, A may be selected from the group consisting of

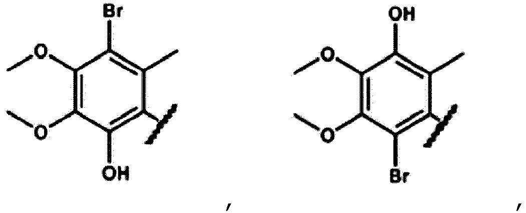

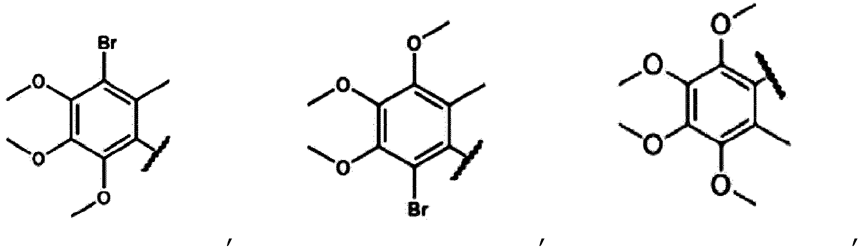

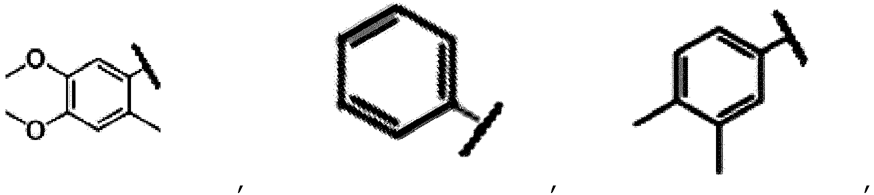

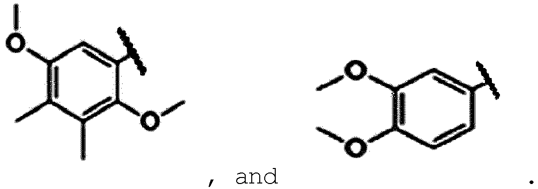

In another specific example, A is naphthalene unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy. In a more specific example, A may be naphthalene-2-yl substituted with one or more selected from C1-5 alkyl and =O. In an even more specific example, A may be

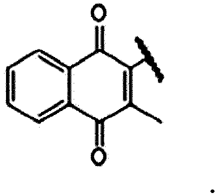

In a further specific example, A may be benzodioxole unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy. In an even further specific example, A may be 1,3-benzodioxole or 2,2-difluoro-1,3-benzodioxole. In a specific example, A may be

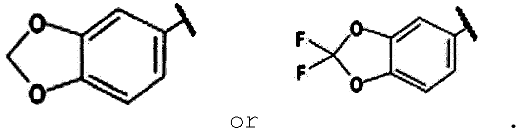

However, A is not limited thereto.

### 3) Unsubstituted or substituted cycloalkyl

In one example, A may be unsubstituted or substituted cycloalkyl. In a specific example, A may be a monocyclic cycloalkyl having 3 to 10 carbon atoms, the monocyclic cycloalkyl unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy. In a more specific example, A may be cyclohexyl unsubstituted or substituted with one or more selected from halogen, =O, hydroxyl, oxycarbonyl, the hydroxy C1-5 alkyl, the C1-5 alkyl, the C1-5 alkenyl, the C1-5 alkynyl, and the C1-5 alkoxy. However, A is not limited thereto.

### 4) Unsubstituted or substituted heterocyclyl

In one example, A may be an unsubstituted or substituted heterocyclyl. In a specific example, A may be pyrrolidine or chroman unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy. In a more specific example, A may be pyrrolidine substituted with one or more selected from oxycarbonyl, C1-5 alkyl, and =O. In another specific example, A may be chroman-2-yl substituted with one or more selected from C1-5 alkyl and hydroxyl. In a specific example, A may be

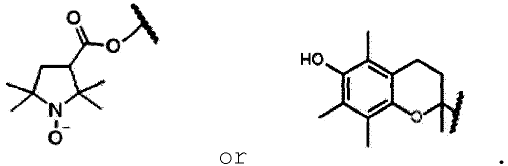

### 3. Structure of L

In Formula 1, L may have a structure with a specific length. In one example, the structure may comprise an alkyl, an alkenyl, an alkynyl, and/or an ethyleneoxy. In a specific example, L may have a structure comprising (CH₂)ₙ. In this case, in one example, n may be an integer of 7 or more and 40 or less. Additionally, n, a factor that determines the length of a connector or linker in the compound of the present application, may play an important role in binding the compound having the structure of Formula 1 to TRAP1.

However, the structure of L is not limited thereto. In this case, in one example, L may have a structure with a length of 10, 15, 20, 25 angstroms, or more. In this case, in one example, L may have a structure with a maximum length of 50, 60, 70, 80, or 90 angstroms. However, the maximum length of L is not limited thereto. In one example, L may have a structure comprising an alkyl, an alkenyl, an alkynyl, and/or an ethyleneoxy with the length described above. In a specific example, L may have an alkyl structure with the length described above.

### 1) Binding capacity depending on distance from TPP

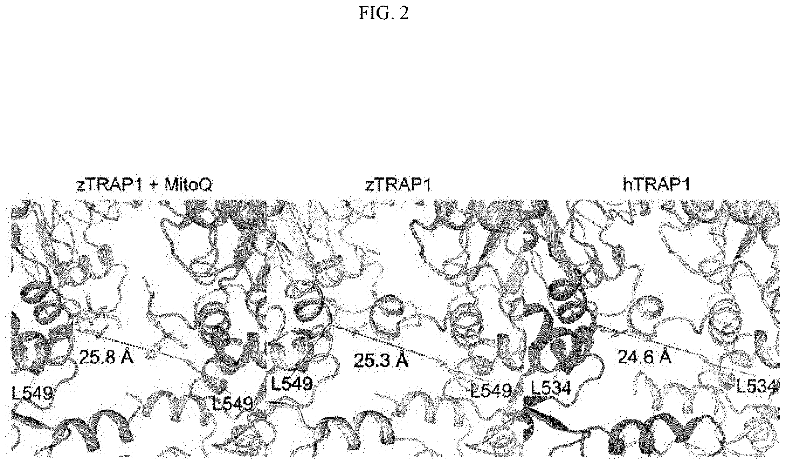

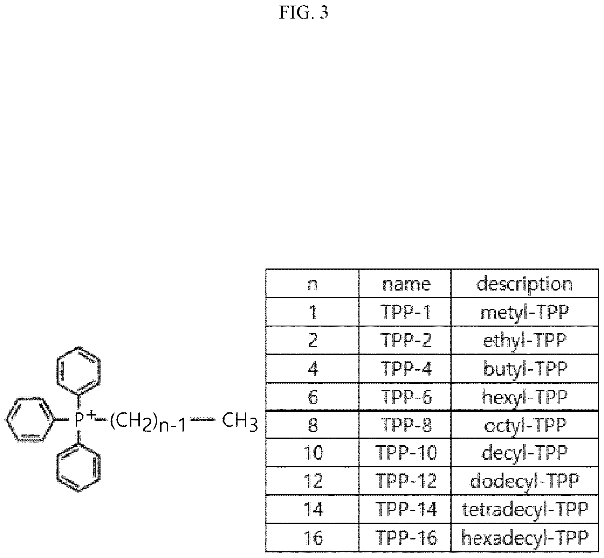

Referring to a crystal structure where TRAP1 and MitoQ (used interchangeably with SMX herein) are bound in FIG. 2, the distance between the two protomers of TRAP1 is about 25 Å, confirming that effective binding is enabled when the distance between the TPP moiety and the Ub moiety of MitoQ is appropriate. Additionally, referring to the experiment results of alkyl-TPPs (FIG. 3 illustrates a structure of alkyl-TPPs), competitive binding to SB-TM2 is confirmed to be enabled from octyl-TPP.

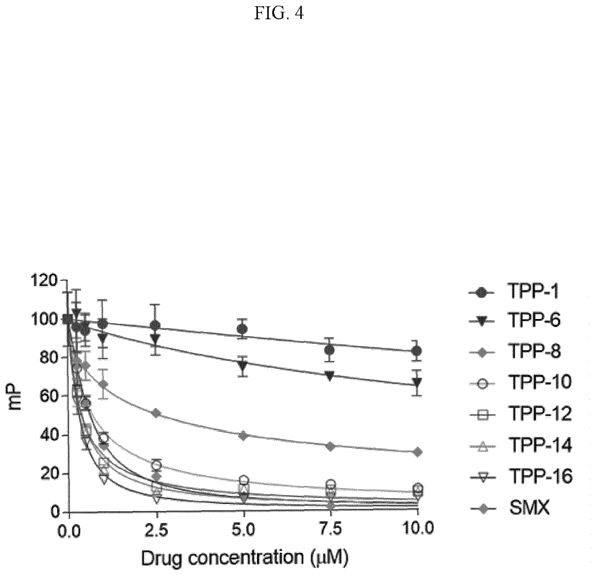

In other words, in the compound structure herein, when the alkyl chain (CH₂)ₙ bound to TPP has a length equal to or greater than the size of C8, the compound may effectively bind to TRAP1. More specifically, when the alkyl chain bound to TPP has a length equal to or greater than the size of C8, the compound may bind to a client-binding site (CBS) of TRAP1. Additionally, Such binding capacity increases as the distance from TPP increases, for example, as the length of the alkyl chain increases. In one example, when additionally compared to the binding capacity of SMX (MitoQ), the binding capacity of dodecyl-TPP, tetradecyl-TPP, and hexadecyl-TPP appears to be superior to that of SMX. In particular, in one example, it was confirmed that the binding capacity of hexadecyl-TPP, especially having the longest alkyl chain, was twice as strong as that of SMX (see Table 1 and FIG. 4) .

**[Table 1] Analysis results of TRAP1 binding capacity of alkyl-TPPs**

| IC₅₀ (µM), FP analysis using SB-TM2 | | |
|---|---|---|
| Drug | IC₅₀ | Activity compared to that of SMX |
| TPP-1 | N.D. | N.D. |
| TPP-2 | N.D. | N.D. |
| TPP-4 | N.D. | N.D. |
| TPP-6 | 18.85 ± 5.39 | 0.03 |
| TPP-8 | 2.572 ± 0.258 | 0.24 |
| TPP-10 | 0.6931 ± 0.0578 | 0.90 |
| TPP-12 | 0.3769 ± 0.0354 | 1.65 |
| TPP-14 | 0.3835 ± 0.0392 | 1.62 |
| TPP-16 | 0.3091 ± 0.0260 | 2.01 |
| SMX | 0.6227 ± 0.0375 | 1 |

### 2) Antioxidant-TPP conjugate

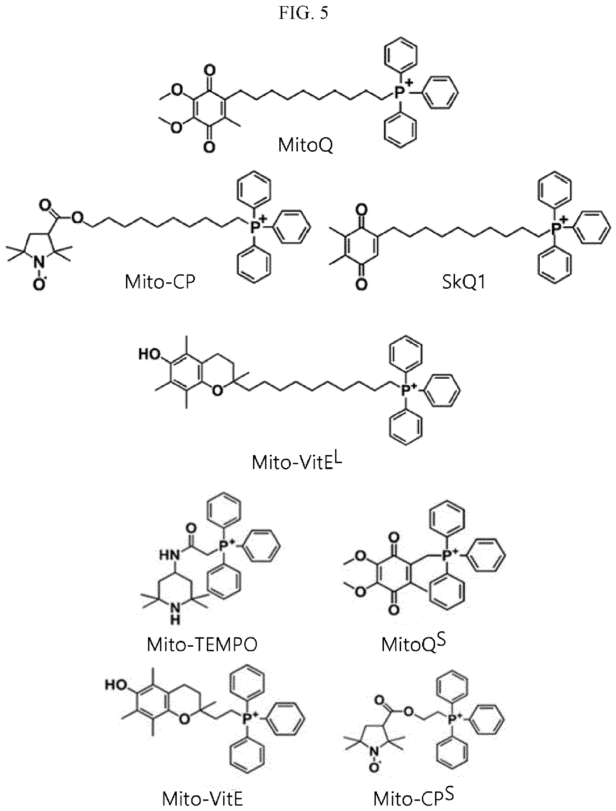

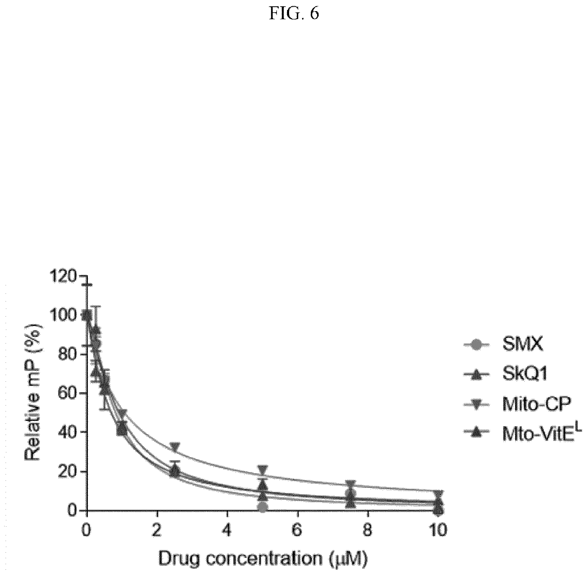

Referring to the experiment results using conjugates in which an antioxidant and TPP are bound (FIG. 5 illustrates structures of MitoQ, Mito-CP, SkQ1, Mito-VitE^{L}, Mito-TEMPO, MitoQ^{s}, Mito-VitE, and Mito-CP^{s}), it is confirmed that Mito-TEMPO, Mito-CP^{s}, and Mito-VitE, having short linkers, do not exhibit TRAP1 binding capacity (see Table 2 and FIG. 6) . In other words, it is confirmed that the appropriate distance of the linker is essential for binding to TRAP1.

**[Table 2] Analysis results of TRAP1 binding capacity of antioxidant-TPP conjugates**

| IC₅₀ (µM), FP analysis using SB-TM2 | | |
|---|---|---|
| Drug | IC₅₀ | Activity compared to that of SMX |
| SkQ1 | 0.6959 ± 0.0554 | 1.18 |
| Mito-CP | 1.074 ± 0.0818 | 0.77 |
| Mito-VitE^{L} | 0.9187 ± 0.0714 | 0.90 |
| Mito-TEMPO | N.D. | N.D. |
| Mito-CP^{S} | N.D. | N.D. |
| Mito-VitE | N.D. | N.D. |
| SMX | 0.824 ± 0.0512 | 1 |

### 3) Other synthetic compounds

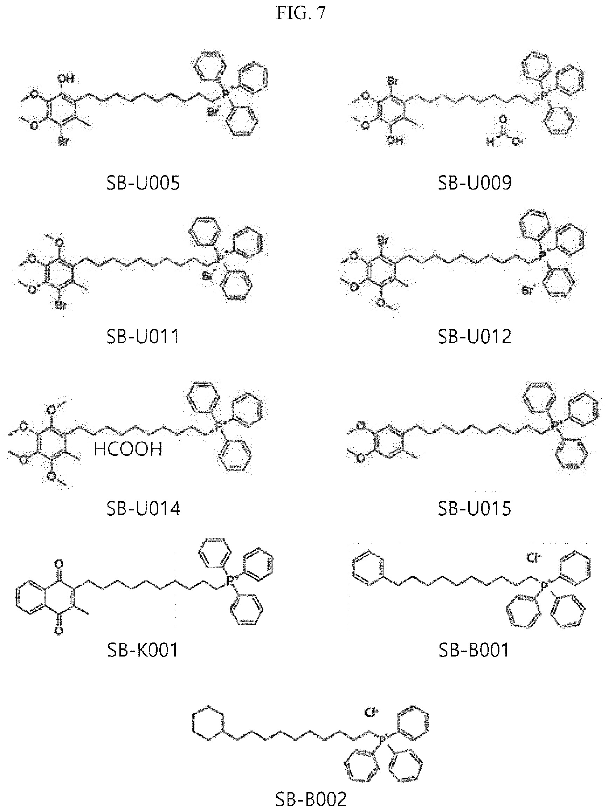

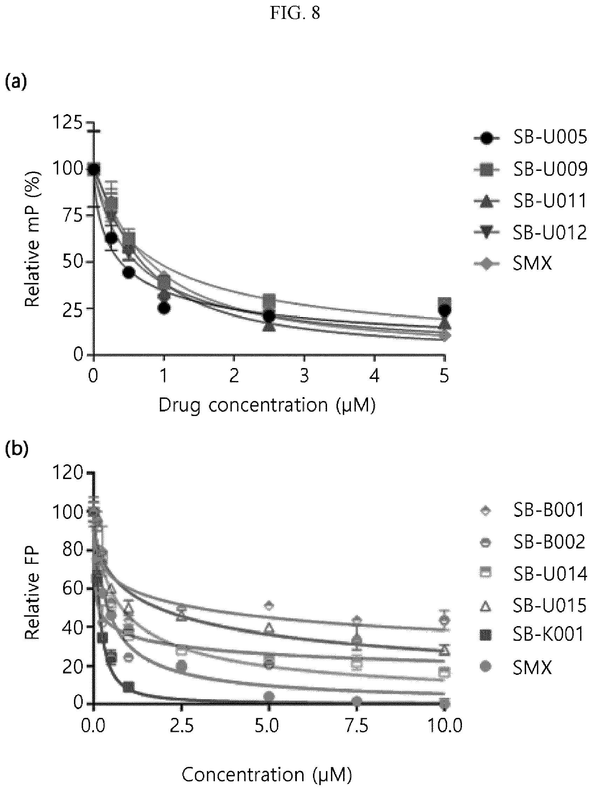

Referring to the experiment results of the binding using other compounds where n is 10 (FIG. 7 illustrates structures of synthetic compounds), it is confirmed that when TPP and various compound structures are linked through a hydrocarbon with a predetermined length, the binding capacity to TRAP1 is exhibited (see Tables 3 and 4 and FIG. 8).

**[Table 3] Analysis results of TRAP1 binding capacity of other synthetic compounds**

| IC₅₀ (µM), FP analysis using SB-TM2 | | |
|---|---|---|
| Drug | IC₅₀ | Activity compared to that of SMX |
| SB-U005 | 0.3853 ± 0.0835 | 2.051907604 |
| SB-U009 | 0.9086 ± 0.1363 | 0.87012987 |
| SB-U011 | 0.6838 ± 0.0729 | 1.156186019 |
| SB-U012 | 0.6010 ± 0.0906 | 1.31547421 |
| SMX | 0.7906 ± 0.0512 | 1 |

**[Table 4] Analysis results of TRAP1 binding capacity of other synthetic compounds**

| IC₅₀ (µM), FP analysis using SB-TM2 | | |
|---|---|---|
| Drug | IC₅₀ | Activity compared to that of SMX |
| SB-U014 | 0.8046 ± 0.0994 | 0.50 |
| SB-U015 | 1.835 ± 0.2849 | 0.22 |
| SB-K001 | 0.1658 ± 0.0230 | 2.45 |
| SB-B001 | 2.966 ± 1.055 | 0.14 |
| SB-B002 | 0.1451 ± 0.13 | 2.80 |
| SMX | 0.4063 ± 0.0389 | 1 |

In other words, what is important for the compound having the structure of Formula 1 to bind to TRAP1 is that n is an integer of at least 7. In addition, it is confirmed that the higher the n value, the higher the TRAP1 binding capacity. Accordingly, in one example, n may be an integer having a maximum value of 20, 30, 40, or 50. However, the maximum value of n is not limited thereto.

### 4. Salt of Formula 1

A salt form of the compound disclosed herein may be considered. In this case, the salt includes pharmaceutically acceptable salts. The salt disclosed herein includes acid addition salts or base addition salts. Examples of acids to form the salt include hydrochloric acid, sulfuric acid, phosphoric acid, glycolic acid, lactic acid, pyruvic acid, citric acid, succinic acid, glutaric acid, and the like, and examples of bases to form the salt include lithium, sodium, potassium, calcium, magnesium, methylamine, trimethylamine, and the like. However, these are not limited thereto and may be easily selected by those skilled in the art.

### 5. Examples of specific compounds

**[Table 5] Specific examples of compounds disclosed herein**

| No. | Compound name |
|---|---|
| 1 | 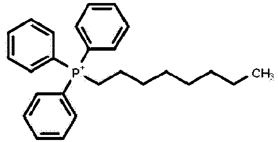 Octyltriphenylphosphonium |
| 2 | 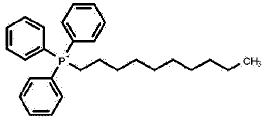 Decyltriphenylphosphonium |
| 3 | 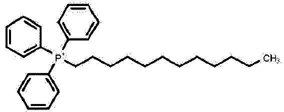 Dodecyltriphenylphosphonium |
| 4 | 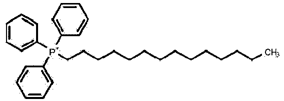 Tetradecyltriphenylphosphonium |
| 5 | 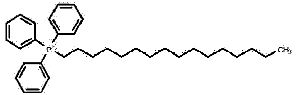 Hexadecyltriphenylphosphonium |
| 6 | 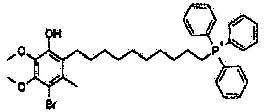 (10-(3-Bromo-4,5,6-trimethoxy-2-methylphenyl)decyl)triphenylphosphonium |
| 7 | 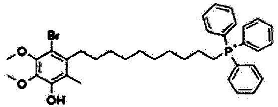 (10-(2-Bromo-5-hydroxy-3,4-dimethoxy-6-methylphenyl)decyl)triphenylphosphonium |
| 8 | 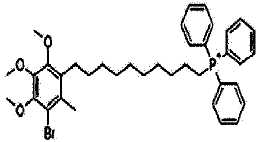 (10-(3-Bromo-4,5,6-trimethoxy-2-methylphenyl)decyl)triphenylphosphonium |
| 9 | 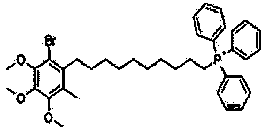 (10-(2-Bromo-3,4,5-trimethoxy-6-methylphenyl)decyl)triphenylphosphonium |
| 10 | 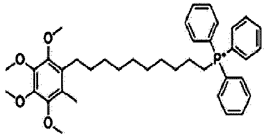 Triphenyl(10-(2,3,4,5-tetramethoxy-6-methylphenyl) decyl) phosphonium |
| 11 | 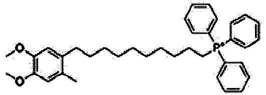 (10-(4,5-Dimethoxy-2-methylphenyl)decyl)triphenylphosphonium |
| 12 | 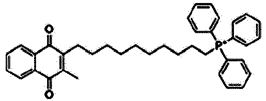 (10-(3-Methyl-1,4-dioxo-1,4-dihydronaphthalen-2-yl)decyl)triphenylphosphonium |
| 13 | 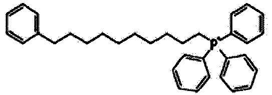 Triphenyl(10-phenyldecyl)phosphonium |
| 14 | 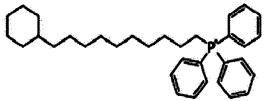 (10-Cyclohexyldecyl)triphenylphosphonium chloride |
| 15 | 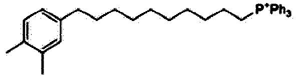 (10-(3,4-Dimethylphenyl)decyl)triphenylphosphonium |
| 16 | 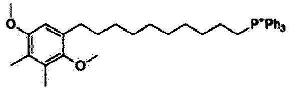 10-(2,5-Dimethoxy-3,4-demethyl-phenyl)decyl-triphenyl-phosphonium |
| 17 | 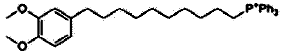 (10-(3,4-Dimethoxyphenyl)decyl)triphenylphosphonium |
| 18 | 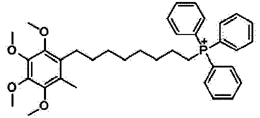 Triphenyl(8-(2,3,4,5-tetramethoxy-6-methylphenyl) octyl) phosphonium |
| 19 | 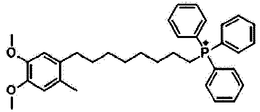 (8-(4,5-Dimethoxy-2-methylphenyl)octyl)triphenylphosphonium |
| 20 | 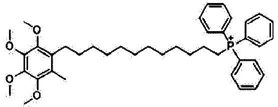 Triphenyl(12-(2,3,4,5-tetramethoxy-6-methylphenyl) dodecyl) phosphonium |
| 21 | 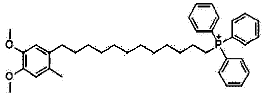 (12-(4,5-Dimethoxy-2-methylphenyl)dodecyl)triphenylphosphonium |
| 22 | 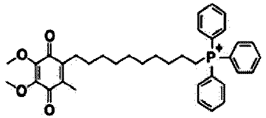 (10-(4,5-Dimethoxy-2-methyl-3,6-dioxocyclohexadien-1-yl)decyl)triphenylphosphonium |
| 23 | 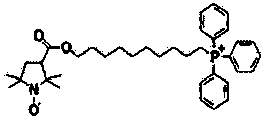 2,2,5,5-Tetramethyl-3-(((10-(triphenylphosphonio)decyl)oxy)carbonyl)pyrrolidine-1-oleate |
| 24 | 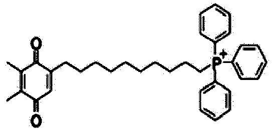 (10-(4,5-Dimethyl-3,6-dioxocyclohexa-1,4-diene-1-yl)decyl)triphenylphosphonium |
| 25 | 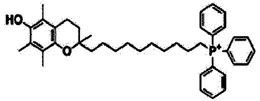 (10-(6-Hydroxy-2,5,7,8-tetramethylchroman-2-yl)decyl)triphenylphosphonium |

### II. Use of compound of Formula 1

### 1. TRAP1 inhibition

One invention disclosed herein provides a use of the compound described above for inhibiting TRAP1.

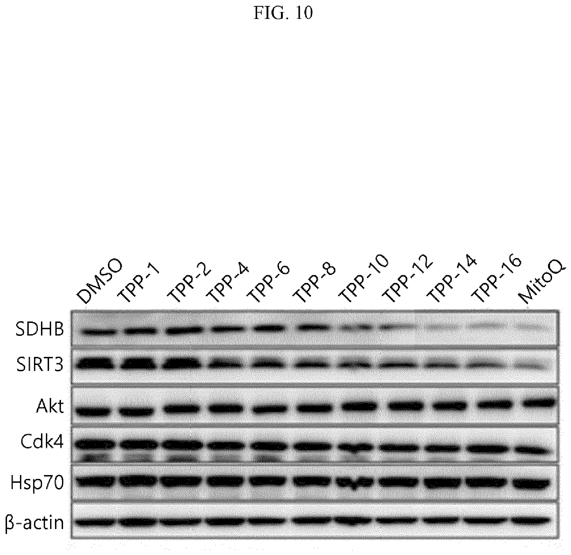

The compound disclosed herein binds to TRAP1 to inhibit the functions of TRAP1, thereby reducing the expression of SDHB and SIRT3, and also promotes p-AMPK and CHOP (see Control of tumor bioenergetics and survival stress signaling by mitochondrial Hsp90s. Cancer cell 22, 331-344, Mitochondrial Hsp90s suppress calcium-mediated stress signals propagating from mitochondria to the ER in cancer cells. Molecular cancer 13, 148), known as markers of TRAP1 inhibition (see FIGS. 10, 12, 13, 15, and 17).

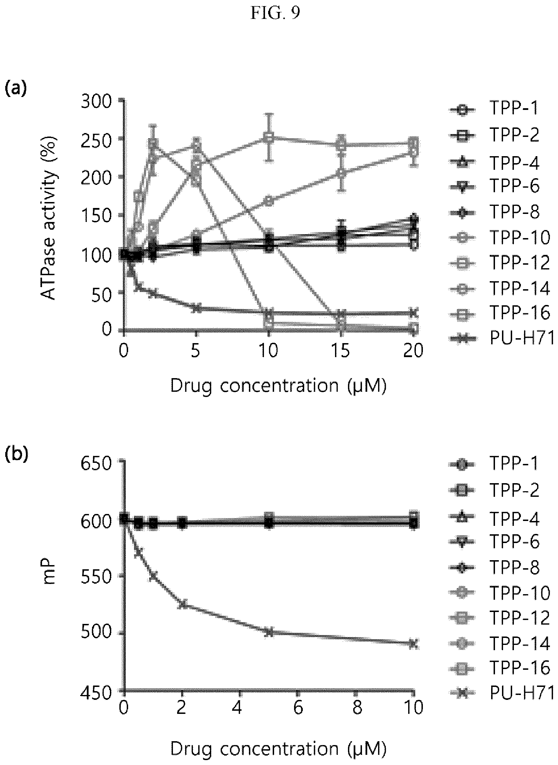

Unlike PU-H71, known to bind to ATP pocket-binding site, the compound provided herein does not inhibit ATPase activity in a concentration-dependent manner in the case of using TPP-alkyls. In other words, the compound provided herein is not related to ATPase activity inhibition. Additionally, the compound provided herein also does not bind to the ATP-binding site. This was confirmed by analyzing ATP pocket-binding capacity in one example of the present disclosure (see FIGS. 9, 11, and 14).

Accordingly, the compound provided herein may bind to and inhibit TRAP1. Even more specifically, the compound provided herein may inhibit TRAP1 without binding to the ATP-binding site of TRAP1.

In other words, provided herein may be a TRAP1 inhibitor comprising the compound having the structure of Formula 1 or the pharmaceutically acceptable salt thereof. In this case, the compound or the pharmaceutically acceptable salt thereof may be characterized by not binding to the ATP-binding site.

Additionally, the compound having the structure of Formula 1 or the pharmaceutically acceptable salt thereof herein may be used to prepare the TRAP1 inhibitor.

### 2. Inhibition of binding between TRAP1 and client protein

The compound may inhibit TRAP1 activity by binding to the middle unit of TRAP1. More specifically, the compound binds to the client-binding site of TRAP1 and inhibits TRAP1 activity without binding to the ATP-binding site.

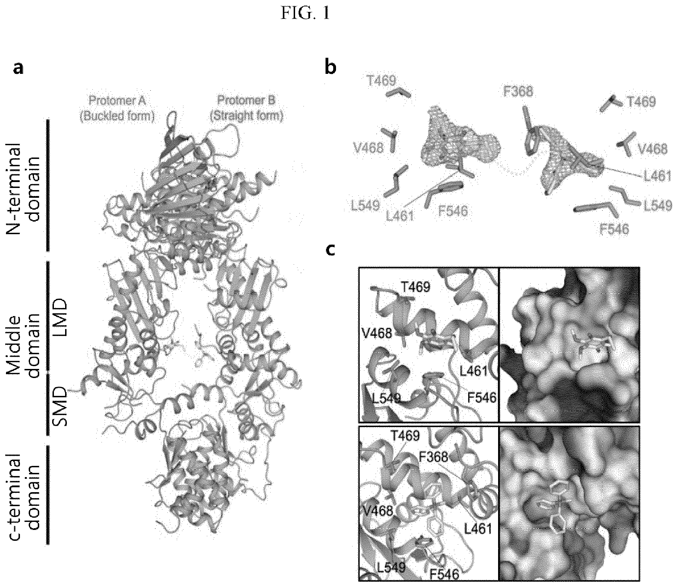

To confirm this, the inventors of the present disclosure analyzed the binding structure of TRAP1 and MitoQ, constructed a fluorescent probe bound to the client-binding site, and then analyzed the binding capacity of the compounds using the probe (see FIGS. 1, 2, 4, 6, and 8). Referring to the experiment results, it is confirmed that all TPP-alkyls, antioxidant-TPP conjugates, and other compounds bind to the client-binding site of TRAP1. Additionally, as a result of analyzing the ATPase activity, examining the presence of binding to the ATP-binding site, and determining whether cytoplasmic Hsp90 was inhibited, it was confirmed that the compound herein inhibits TRAP1 activity without binding to the ATP-binding site (see FIGS. 9, 11, and 14).

Furthermore, specifically, the compound herein selectively inhibits only TRAP1 without affecting the client protein of cytoplasmic Hsp90 and Hsp70 (see Evidence for Efficacy of New Hsp90 Inhibitors Revealed by Ex Vivo Culture of Human Prostate Tumors. Clinical Cancer Research 18, 3562-3570.), a marker of Hsp90 inhibition (see FIGS. 10, 12, 13, 15, and 17).

In other words, the compound herein binds to the middle unit of TRAP1 and does not affect the N-terminal domain where ATP is bound to produce energy for the activity of Hsp90s.

Accordingly, the compound provided herein binds to the middle unit of TRAP1 and may thus inhibit the binding of the client protein known to bind to the middle unit. In other words, the compound herein may be used to inhibit the binding between TRAP1 and the client protein.

Provided herein may be a TRAP1-client protein binding inhibitor comprising the compound having the structure of Formula 1 or the pharmaceutically acceptable salt thereof.

The compound having the structure of Formula 1 or the pharmaceutically acceptable salt thereof herein may be used to prepare the TRAP1-client protein binding inhibitor.

### 3. Pharmaceutical composition

The compound provided herein or the pharmaceutically acceptable salt thereof may be used as a pharmaceutical composition. In other words, provided herein is a pharmaceutical composition comprising the compound or the pharmaceutically acceptable salt thereof. The compound or the pharmaceutically acceptable salt thereof herein may be used to prepare the pharmaceutical composition.

The pharmaceutically acceptable salts include salts of compounds derived from various physiologically acceptable organic and inorganic counterions. The counterions are well-known in the art and include, for example, sodium, potassium, calcium, magnesium, aluminum, lithium, and ammonium, such as tetraalkylammonium and the like (when a molecule comprises an acidic functional group). Additionally, when a molecule comprises a basic functional group, the counterions include salts of organic or inorganic acids, such as hydrochloride, sulfate, phosphate, diphosphate, nitrate hydrobromide, tartrate, mesylate, acetate, malate, maleate, fumarate, tartrate, succinate, citrate, lactate, pamoate, salicylate, stearate, methanesulfonate, p-toluenesulfonate, oxalate, and the like. Suitable pharmaceutically acceptable salts thereof include those listed in the literature [Remington's Pharmaceutical Sciences, 17th Edition, pg. 1418 (1985) and P. Heinrich Stahl, Camille G. Wermuth (Eds.), Handbook of Pharmaceutical Salts Properties, Selection, and Use; 2002]. Examples of acid addition salts include acids such as hydroiodic acid, phosphoric acid, metaphosphoric acid, nitric acid, and sulfuric acid, and organic acids, such as alginic acid, ascorbic acid, anthranilic acid, benzoic acid, camphor sulfuric acid, citric acid, embonic acid (pamoic acid), ethanesulfonic acid, formic acid, fumaric acid, furoic acid, galacturonic acid, gentisic acid, gluconic acid, glucuronic acid, glutamic acid, glycolic acid, isonicotinic acid, isothionic acid, lantacid, malic acid, mandelic acid, methanesulfonic acid, mucic acid, pantothenic acid, phenylacetic acid, propionic acid, saccharic acid, salicylic acid, stearic acid, succinic acid, sulfinic acid, trifluoroacetic acid, and aryl sulfonic acid, including salts formed from benzenesulfonic acid and p-toluenesulfonic acid. Examples of base addition salts formed with alkali, alkaline earth metals, and organic bases include chloroprocaine, choline, N,N-dibenzylethylenediamine, diethanolamine, ethylenediamine, lysine, meglumine (N-methylglucamine), and crocaine, as well as salts formed internally. Salts comprising non-physiologically acceptable anions or cations are useful intermediate products for use in preparing physiologically acceptable salts and/or in non-therapeutic situations, for example, in vitro, and fall within the scope of the present disclosure. The pharmaceutically acceptable salt, according to the present application, includes halogen salts, that is, fluorine salts, bromine salts, iodine salts, and the like.

The pharmaceutical composition may be used to treat diseases capable of being treated through TRAP1 inhibition.

In one example, the disease includes cancer. TRAP1 is widely known to be related to cancer and may be a target for cancer treatment (see Regulation of Tumor Cell Mitochondrial Homeostasis by an Organelle-Specific Hsp90 Chaperone Network, 2007, Kang et al; Control of Tumor Bioenergetics and Survival Stress Signaling by Mitochondrial Hsp90s, 2012, Chae et al; The mitochondrial chaperone TRAP1 as a candidate target of oncotherapy, 2001, Xie et al; TRAP1:a viable therapeutic target for future cancer treatments, 2017, Lettini et al.). Additionally, referring to the experiment results, it is confirmed that the compound provided herein inhibits TRAP1 in cancer cells and thus reduces the size of cancer.

Accordingly, the compound herein may be used in preparing pharmaceutical compositions for cancer treatment.

Provided herein may be a pharmaceutical composition for treating cancer, the composition comprising the compound having the structure of Formula 1 or the pharmaceutically acceptable salt thereof.

In this case, the cancer may include thyroid cancer, stomach cancer, colon cancer, lung cancer, breast cancer, liver cancer, prostate cancer, pancreatic cancer, gallbladder cancer, biliary tract cancer, and the like. However, the disease is not limited to cancer and includes all diseases known to be related to TRAP1.

Additionally, in one example, the disease includes an ocular disease. In this case, the ocular diseases include ocular neovascularization diseases characterized by choroidal neovascularization diseases, retinal neovascularization diseases, subretinal neovascularization diseases, corneal neovascularization diseases, iris neovascularization diseases, or neovascular glaucoma. In addition, the ocular neovascularization diseases may refer to retinal neovascularization diseases, and the retinal neovascularization diseases may mean ocular neovascularization diseases characterized by diabetic retinopathy, retinopathy of prematurity, or retinal vein occlusion. Additionally, the choroidal neovascularization disease may mean wet age-related macular degeneration (wet AMD).

### 1) Ocular diseases

Ocular diseases described herein may be classified according to the ocular structure where abnormalities occur. In this case, the ocular structure may be an eye component including the conjunctiva, sclera, cornea, iris, ciliary body, lens, choroid, retina, vitreous body, optic nerve, or eye muscle. In this case, ocular diseases occurring in the retina among the structure described above are called retinosis or retinopathy.

Additionally, ocular diseases may be classified according to the presence or absence of neovascularization. The neovascularization refers to a physical process of forming new blood vessels around malformed blood vessels. The neovascularization may occur abnormally due to abnormal blood vessel weakening, ischemia, or excessive production of neovascularization factors. In this case, abnormal neovascularization causes the vascular structure to become dense, and the blood vessels fail to grow sufficiently thick, resulting in abnormal symptoms such as increased pressure in the blood vessels and separation of the blood vessels from the ocular structure.

Ocular diseases related to the neovascularization include choroidal neovascularization, retinal neovascularization, subretinal neovascularization, corneal neovascularization, or iris neovascularization (rubeosis iridis). Additionally, the retinal neovascularization may cause diabetic retinopathy, retinopathy of prematurity, retinal vein occlusion, or the like. Furthermore, subretinal neovascularization may cause wet age-related macular degeneration (wet AMD).

The ocular disease for use in treating ocular diseases herein may mean ocular diseases related to the neovascularization described above.

### 2) Correlation between ocular disease and TRAP1

### TRAP1 expression in retinopathy model

To confirm a correlation between ocular diseases and TRAP1, the expression level of TRAP1 was confirmed using a retinopathy model.

Referring to the experiment results, increases in the TRAP1 expression are confirmed in both the oxygen-induced retinopathy model and streptozotocin (STZ)-induced diabetic retinopathy model (see FIGS. 18 and 19).

In other words, ocular diseases and increases in TRAP1 expression are confirmed to be correlated.

Additionally, in the oxygen-induced retinopathy model and the STZ-induced diabetic retinopathy model, increases in HIF1α, a marker of hypoxia, and VEGF-A, a downstream angiogenic factor, are confirmed, along with increases in TRAP1 (see FIGS. 18A and 19A). Furthermore, as a result of staining, TRAP1 is confirmed to be co-located with the staining of glutamine synthase (GS), a marker of Müller cells that is responsible for the production of various angiogenic factors, during the progression of the retinopathy diseases (see FIGS. 18C and 19C).

In other words, it was confirmed through the results that a hypoxic environment causes increases in TRAP1 expression in Müller cells.

### TRAP1 knockout in retinopathy model

Referring to the experiment results, neovascular and avascular areas are confirmed to be reduced in the case of an OIR model where TRAP1 is knocked out (see FIG. 20).

In other words, this indicates that pathological retinal angiogenesis may be improved when inhibiting TRAP1.

Additionally, referring to the HIF1α staining results in the retina, HIF1α is confirmed to be reduced when TRAP1 is knocked out in the STZ or OIR model (see FIG. 21).

In other words, TRAP1 inhibition is confirmed to be able to reduce HIF1α.

Furthermore, referring to the expression results of VEGF-A and ANGPTL4, the mRNA levels of VEGF-A and ANGPTL4 were elevated in STZ TRAP1+/+ but not elevated in STZ-TRAP1-/-, compared to those in Con TRAP1+/+. In other words, it is seen that when inhibiting TRAP1, the expression of VEGF-A and ANGPTL4 does not increase (see FIG. 22A).

Additionally, in the retina of the OIR model, the mRNA levels of VEGF-A and ANGPTL4 were decreased in TRAP1+/- and TRAP1-/- compared to that in TRAP1+/+. In other words, it is seen that when inhibiting TRAP1, the expression of VEGF-A and ANGPTL4 is reduced (see FIG. 22B).

In conclusion, it is safe to say that TRAP1 inhibition destabilizes HIF1α and thus reduces various angiogenic factors that cause retinopathy under hypoxic conditions.

### TRAP1 inhibitors and retinopathy

Treatment with a TRAP1 inhibitor may reduce avascular and neovascular areas in the retina. Referring to the experiment results, it is confirmed that when treated with MitoQ, both avascular and neovascular areas are significantly reduced in the retina of the OIR model. This confirms that MitoQ inhibits abnormal angiogenesis by inhibiting TRAP1 and inhibits the development of vascular diseases by stimulating normal angiogenesis. Such experiment results appeared to be the same when applying MitoQ through intravitreal injection and eye drop administration. This shows that even when applied through eye drop administration, it is effectively infiltrated into the tissue and thus exhibits effects (see FIGS. 23 and 24).

Additionally, to determine whether other TRAP1 inhibitor compounds were able to be applied to retinopathy treatment, whether these compounds were able to inhibit HIF1α was examined. As a result, all alkyl-TPPs, TPP-antioxidant conjugates (when comprising a linker with appropriate length), and other synthetic compounds, confirmed to inhibit TRAP1, were confirmed to have HIF1α inhibitory activity (see FIGS. 25 to 27 and Table 6 to 9).

**[Table 6] HIF1α inhibitory activity of alkyl-TPPs**

| IC₅₀ (µM), HIF1α inhibitory activity in MIO-M1 cells | | |
|---|---|---|
| Drug | IC₅₀ | Activity compared to that of SMX |
| TPP-1 | N.D. | N.D. |
| TPP-2 | N.D. | N.D. |
| TPP-4 | N.D. | N.D. |
| TPP-6 | 1.431 ± 5.39 | 0.33 |
| TPP-8 | 0.6427 ± 0.258 | 0.73 |
| TPP-10 | 0.3138 ± 0.0578 | 1.49 |
| TPP-12 | 0.1110 ± 0.0354 | 4.21 |
| TPP-14 | 0.0340 ± 0.0392 | 13.76 |
| TPP-16 | 0.0300 ± 0.0260 | 15.59 |
| SMX | 0.4678 ± 0.0151 | 1 |

**[Table 7] HIF1α inhibitory activity of TPP-antioxidant conjugates**

| IC₅₀ (µM), HIF1α inhibitory activity in MIO-M1 cells | | |
|---|---|---|
| Drug | IC₅₀ | Activity compared to that of SMX |
| SkQ1 | 0.1827 ± 0.0091 | 1.79 |
| Mito-CP | 0.2967 ± 0.0236 | 1.10 |
| Mito-VitE^{L} | 0.1424 ± 0.0088 | 2.30 |
| Mito-TEMPO | N.D. | N.D. |
| Mito-CP^{S} | N.D. | N.D. |
| Mito-VitE | N.D. | N.D. |
| SMX | 0.3269 ± 0.0229 | 1 |

**[Table 8] HIF1α inhibitory activity of other synthetic compounds**

| IC₅₀ (µM), HIF1α inhibitory activity in MIO-M1 cells | | |
|---|---|---|
| Drug | IC₅₀ | Activity compared to that of SMX |
| SB-U005 | 0.131 ± 0.0091 | 3.92 |
| SB-U009 | 0.1912 ± 0.0236 | 2.69 |
| SB-U011 | 0.0853 ± 0.0088 | 6.02 |
| SB-U012 | 0.1771 ± 0.0088 | 2.90 |
| SMX | 0.5137 ± 0.0552 | 1 |

**[Table 9] HIF1α inhibitory activity of other synthetic compounds**

| IC₅₀ (µM), HIF1α inhibitory activity in MIO-M1 cells | | |
|---|---|---|
| Drug | IC₅₀ | Activity compared to that of SMX |
| SB-U014 | 0.0694 ± 0.0031 | 4.02 |
| SB-U015 | 0.0887 ± 0.0058 | 3.14 |
| SB-K001 | 0.0555 ± 0.0033 | 5.03 |
| SB-B001 | 0.0947 ± 0.0132 | 2.95 |
| SB-B002 | 0.0223 ± 0.0010 | 12.51 |
| SMX-R | 0.2141 ± 0.0093 | 1.30 |
| SMX | 0.2789 ± 0.0170 | 1 |

### 3) Pharmaceutical composition for treating ocular diseases

Provided herein is a pharmaceutical composition comprising a TRAP1 inhibitor for treating ocular diseases. The TRAP1 inhibitor comprises the compound having the structure of Formula 1 or the pharmaceutically acceptable salt thereof. Additionally, provided herein is a use of the TRAP1 inhibitor to prepare the pharmaceutical composition for treating ocular diseases. Specifically, provided herein is a use of the compound having the structure of Formula 1 or the pharmaceutically acceptable salt thereof to prepare the pharmaceutical composition for treating ocular diseases. As described above, ocular diseases are related to TRAP1. In addition, it was confirmed through the experiment results that TRAP1 inhibition destabilized HIF1α and reduced various angiogenic factors that cause retinopathy under hypoxic conditions. Accordingly, ocular diseases may be treated through TRAP1 inhibition.

### 4. Treatment method

The compound provided herein or the pharmaceutically acceptable salt thereof may be administered to a subject in need thereof and used to treat a specific disease. That is, provided herein is a method of treating a specific disease, the method including administering a pharmaceutical composition comprising the compound or the pharmaceutically acceptable salt thereof to a subject in need thereof.

The disease includes all diseases capable of being treated through TRAP1 inhibition. In one example, the disease includes cancer but is not limited thereto and includes all diseases known to be associated with TRAP1. In this case, the cancer may include thyroid cancer, stomach cancer, colon cancer, lung cancer, breast cancer, liver cancer, prostate cancer, pancreatic cancer, gallbladder cancer, biliary tract cancer, and the like.

Additionally, as a specific example, provided is a method for treating ocular diseases, the method including administering a pharmaceutical composition comprising the compound provided herein or the pharmaceutically acceptable salt thereof to a subject in need thereof. In this case, the ocular diseases include all ocular diseases described in "3. Pharmaceutical composition 1) Ocular diseases".

The administration of the pharmaceutical composition to the subject in need thereof may be performed through various routes. For example, the administration includes oral administration (for example, drenches such as aqueous or nonaqueous solutions or suspensions, tablets, capsules (including sprinkle capsules and gelatin capsules), lumps, powders, granules, pastes for application to the tongue); absorption through the oral mucosa (for example, sublingual absorption); anal, rectal, or intravaginal administration (for example, pessaries, creams, or foams); parental administration (for example, sterile solutions or suspensions administered intramuscularly, intravenously, subcutaneously, or intrathecally); intranasal administration; intraperitoneal administration; subcutaneous administration; transdermal administration (for example, a patch applied to the skin); and topical administration (for example, creams, ointments, sprays applied to the skin, or eye drops). In one example, the pharmaceutical composition may be administered orally. In another example, the pharmaceutical composition may be administered topically using an eye drop. However, the administration method is not limited thereto.

The subject is a mammal, such as a human, or a non-human mammal. When administered to the subject, for example, a human, a composition or a compound is preferably administered, for example, as the pharmaceutical composition comprising the compound of the present application and a pharmaceutically acceptable carrier. The pharmaceutically acceptable carrier is well-known in the art and includes, for example, aqueous solutions such as water, physiologically buffered saline, oils such as glycol, glycerol, olive oil, and the like, other solvents such as injectable organic esters, or carriers.

The actual dosage of the pharmaceutical composition may vary depending on a particular patient, composition, and administration method to obtain an amount of active ingredient effective to achieve the desired therapeutic response without showing toxicity to the patient.

The dosage selected will depend on various factors, including a specific compound or combination of compounds used, the activity of esters, salts, or amides thereof, administration routes, administration times, excretion rates of the specific compound used, treatment durations, other drugs, compounds, and/or substances used in combination with the specific compound used, age, gender, weight, condition, general health, and medical history of the subject being treated, and other factors well-known in the medical field.

A doctor or veterinarian skilled in the art may easily determine and prescribe the required therapeutically effective amount of the pharmaceutical composition. For example, a physician or veterinarian may set the dosage of the pharmaceutical composition or the compound at a level lower than that necessary to achieve the desired therapeutic effect and slowly increase the dosage until the desired effect is achieved. "Therapeutically effective amount" means a concentration of the compound sufficient to derive the desired therapeutic effect.

It is typically understood that the effective amount of the compound may vary depending on the weight, gender, and medical history of the subject. Other factors that affect the effective amount include the severity of the condition of the subject, the disorder being a treatment target, the stability of the compound, and other types of therapeutics administered in combination with the compound of the present application if desired, but are not limited thereto. A large amount of the total dose may be delivered by administering a medicine several times. A method of determining efficacy and dose is known to those skilled in the art (Isselbacher et al. (1996) Harrison's Principles of Internal Medicine 13 ed., 1814-1882, incorporated herein by reference).

In a specific example, the compound provided herein may be administered alone or through conjoint administration with other types of therapeutics. As used herein, the term "conjoint administration" refers to any method of administering two or more different therapeutic compounds such that a second compound is administered while the previously administered therapeutic compound is still effective in the body (for example, the two compounds may be simultaneously effective on the subject while involving synergistic effects of the two compounds). For example, different therapeutic compounds in the same formulation or separate formulations may be administered concomitantly or sequentially. In a specific example, different therapeutic compounds may be administered at intervals within 1 hour, 12 hours, 24 hours, 36 hours, 48 hours, 72 hours, or 1 week from each other. Therefore, the subject receiving such treatment may benefit from the combined effects of different therapeutic compounds.

In a specific example, conjoint administration of the compound provided herein and one or more additional therapeutics (for example, one or more additional chemotherapeutics) provides improved efficacy compared to individual administration of the compound of the present application or one or more additional therapeutics. In such a specific example, conjoint administration provides an additive effect, where the additive effect herein means the sum of the respective effects of individual administrations of the compound herein and one or more additional therapeutics.

### III. Experiment example

### 1. Synthesis of compound

To confirm the binding capacity of compounds to TRAP1, the inventors of the present disclosure constructed SB-TM2, a fluorescent probe that binds to TRAP1, as well as additionally synthesized and purchased compounds for use in experiments.

Methyltriphenylphosphonium bromide (Alfa Aesar (Cat.# A15878)), ethyltriphenylphosphonium bromide (Alfa Aesar (Cat.# B23096)), butyltriphenylphosphonium bromide (Alfa Aesar (Cat.# A10504)), hexyltriphenylphosphonium bromide (Alfa Aesar (Cat.# A13826)), octyltriphenylphosphonium bromide (Alfa Aesar (Cat.# L02412)), decyltriphenylphosphonium bromide (Alfa Aesar (Cat.# A11021)), dodecyltriphenylphosphonium bromide (Alfa Aesar (Cat.# A14295)), tetradecyltriphenylphosphonium bromide (Alfa Aesar (Cat.# L04311)), hexadecyltriphenylphosphonium bromide (Alfa Aesar (Cat.# A15180)), MitoQ (BioVision, Cat #: B1309), SkQ1 (MedChemExpress Cat.#: HY-100474), and Mito-TEMPO (SIGMA Cat #: SML0737) were purchased and used in the experiments.

### 1-1. Synthesis of SB-TM2 probe

SB-TM2: 6-(11-oxo-2,3,5,6,7,11-hexahydro-1H-pyrano[2,3-f pyrido[3,2,1-ij] quinoline-10-carboxamido)hexyl)triphenylphosphonium methane sulfonate.

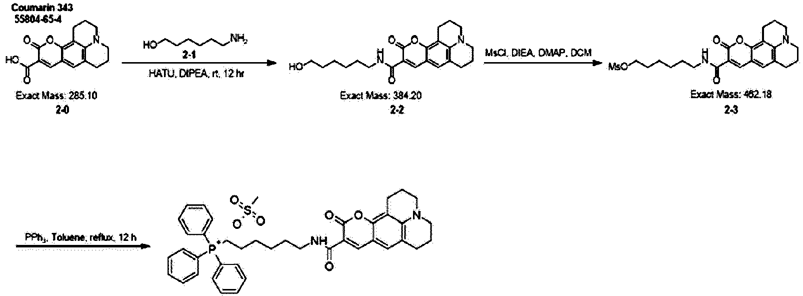

### Step A. N-(6-hydroxyhexyl)-20-oxo-27-oxa-23-azatetracycloheptadeca-(14),1(16),15(18),17(19)-tetraene-16-carboxamide

DIPEA (511.67 mg, 3.95 mmol) was added to a solution of compounds, 14-oxo-20-oxa-16-azatetracycloheptadeca-(8),1(10),9(12),11(13)-tetraene-10-carboxylic acid (376.5 mg, 1.31 mmol), 6-aminohexan-1-ol (170.12 mg, 1.38 mmol), and HATU (602.15 mg, 1.58 mmol), in DMF (30 mL) and then stirred at 25°C for 12 hours. LCMS showed that the starting material was consumed, and the desired product was formed. The reaction mixture was poured into 60 mL of H₂O and then extracted with ethyl acetate (50 mL x 2) . The combined ether acetate was washed with saturated brine (50 mL x 2) and dried over Na₂SO₄. The organic layer was evaporated to dryness to provide yellow foam as a crude product, which was purified by column chromatography (SiO2, petroleum ether/EtOAc = 1:0 to 1:4) to obtain yellow foam (465 mg, 1.17 mmol, 92.4% yield) as the desired product.

MS (ESI) : mass calcd. for C₂₂H₂₈N₂O₄, 384.2; m/z found, 385.2 [M+H]+.

1H NMR (400 MHz, CDCl3) δ 1.36 - 1.50 (m, 4 H), 1.51 - 1.72 (m, 4 H), 1.92 - 2.06 (m, 4 H), 2.78 (t, J = 6.0 Hz, 2 H), 2.90 (t, J = 6.3 Hz, 2 H), 3.28 - 3.39 (m, 4 H), 3.45 (q, J=6.8 Hz, 2 H), 3.65 (t, J = 6.5 Hz, 2 H), 7.02 (s, 1 H), 8.61 (s, 1 H), 8.88 (br s, 1 H).

### Step B. 6-[(21-Oxo-29-oxa-24-azatetracycloheptadeca-1(15),2(17),16(19),18(20)-tetraene-17-carbonyl)amino]hexyl methanesulfonate

N-(6-hydroxyhexyl)-20-oxo-27-oxa-23-azatetracycloheptadeca-(14),1(16),15(18),17(19)-tetraene-16-carboxamide (400 mg, 1.04 mmol) was dissolved in DCM (25 mL), followed by adding DMAP (1.27 mg, 10.40 umol) and DIEA (1.34 g, 10.40 mmol) thereto. Then, the resulting mixture was cooled to 0°C, followed by adding MsCl (106 mg, 9.25 mmol) dropwise thereto, and then stirred at 0°C for 2 hours. TLC (petroleum ether:EtOAc = 1:1, Rf = 0.43) showed that the starting alcohol was consumed, and one major new spot was formed. The resulting mixture was distributed in between dichloromethane (35 mL) and saturated aqueous NaHCO₃ (35 mL). The organic layer was collected, dried over Na₂SO₄, filtered, and concentrated in vacuo to obtain a yellow solid as a crude product. HNMR showed that the obtained solid was sufficiently pure for the next reaction.

1H NMR (400 MHz, CDCl3) δ 1.36 - 1.56 (m, 4 H), 1.60 - 1.85 (m, 4 H), 1.99 (m, 4 H), 2.78 (m, 2 H), 2.90 (m, 2 H), 3.01 (s, 3 H), 3.26 - 3.39 (m, 4 H), 3.44 (q, J = 6.6 Hz, 2 H), 4.23 (t, J = 6.5 Hz, 2 H), 7.02 (s, 1 H), 8.61 (s, 1 H), 8.88 (br d, J = 4.9 Hz, 1 H).

### Step C. (6-(11-Oxo-2,3,5,6,7,11-hexahydro-1H-pyrano[2,3-f]pyrido[3,2,1-ij]quinoline-10-carboxamido)hexyl)triphenylphosphonium methane sulfonate

6-[(21-Oxo-29-oxa-24-azatetracycloheptadeca-1(15),2(17),16(19),18(20)-tetraene-17-carbonyl)amino]hexyl methane sulfonate (97 mg, 203.41 umol) was thoroughly mixed with triphenylphosphane (266.76 mg, 1.02 mmol) in 5 mL of toluene (5 mL). Subsequently, the resulting solution was stirred at 130°C under N₂ for 18 hours. LCMS showed that the sulfonate was consumed, and the desired product was formed as a major component. The reaction mixture was cooled to room temperature and evaporated to dryness. TLC (DCM:MeOH = 10:1, Rf = 0.24) showed that one major new spot was formed under OPPh₃. The resulting crude product was purified by flash column chromatography on silica gel (first eluted with 50 to 100% EtOAc in petroleum for 20 minutes and held at 100% for 15 minutes to remove all impurities on the desired product, then switched to 0 to 10% MeOH in DCM for 20 minutes and held at 10% for 20 minutes) to obtain a triphenylphosphonium methanesulfonate salt as the desired product. The obtained product was additionally lyophilized to remove the residual solvent to obtain a yellow solid (106 mg, 145.55 umol, 23.85% yield, 98.24% purity).

MS (ESI): mass calcd. for C40H42N2O3P+, 629.29; m/z found, 629.5 [M+H]+.

1H NMR (400 MHz, MeOD) δ 1.45 (m, 2 H), 1.52 - 1.78 (m, 6 H), 1.90 - 2.04 (m, 4 H), 2.69 (s, 3 H), 2.74 - 2.87 (m, 4 H), 3.34 - 3.50 (m, 8 H), 7.08 (s, 1 H), 7.62 - 7.97 (m, 15 H), 8.44 (s, 1 H), 9.04 (br s, 1 H); 31P NMR (162 MHz, METHANOL-d4) δ ppm 23.81 (s, 1 P).

### 1-2. Synthesis of (10-(3-bromo-4,5,6-trimethoxy-2-methylphenyl)decyl)triphenylphosphonium bromide

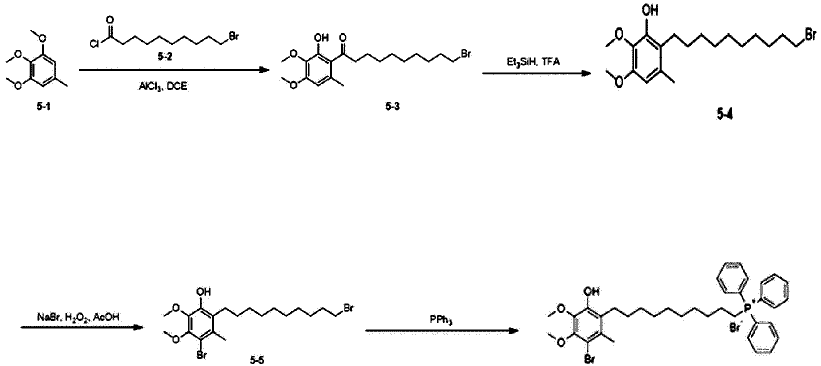

### Step A. 10-Bromo-1-(2-hydroxy-3,4-dimethoxy-6-methylphenyl)decan-1-one

Freshly powdered AlCl₃ (457.89 mg, 3.43 mmol) was added to 10-bromodecanoyl chloride (0.536 g, 1.89 mmol) and 1,2,3-trimethoxy-5-methyl-benzene (312.86 mg, 1.72 mmol) in dry DCE (10 mL) and stirred at 25°C for 40 hours. LCMS showed that the desired product was formed as a major component. The resulting mixture was poured into ice water and extracted with CH₂Cl₂ (50 mL * 2). The combined extract was washed with water, dried over Na₂SO₄, and concentrated to obtain an oil, which was purified by column chromatography (SiO2, 10:0 to 10:1 petroleum ether/EtOAc) to obtain a colorless oil (520 mg, 66.56% yield).

MS (ESI) : mass calcd. for C₁₉H₂₉BrO₄, 400.12; m/z found, 402.8 [M+H]+.

1H NMR (400 MHz, CDCl3) δ 1.21 - 1.55 (m, 10 H), 1.56 - 1.78 (m, 2 H), 1.85 (m, 2 H), 2.46 (s, 3 H), 2.89 (t, J = 7.4 Hz, 2 H), 3.41 (t, J = 6.8 Hz, 2 H), 3.88 (d, J = 12.3 Hz, 6 H), 6.31 (s, 1 H), 10.38 (s, 1 H).

### Step B. 2-(10-Bromodecyl)-5,6-dimethoxy-3-methyl-phenol

10-Bromo-1-(2-hydroxy-3,4-dimethoxy-6-methylphenyl)decan-1-one (520 mg, 1.14 mmol) was dissolved in TFA (10 mL), followed by adding Et3SiH (2 mL) thereto, and then stirred at 80°C for 12 hours. LCMS showed that the starting ketone was consumed, and a new peak was formed. The reaction mixture was evaporated to dryness and purified by column chromatography (SiO2, 5:0 to 5:1 petroleum ether/EtOAc) to obtain a colorless oil (410 mg, 82.34% yield).

MS (ESI) : mass calcd. for C₁₉H₃₁BrO₃, 386.15; m/z found, 388.9 [M+H]+.

1H NMR (400 MHz, CDCl3) δ 1.22 - 1.55 (m, 14 H), 1.86 (quin, J = 7.1 Hz, 2 H), 2.26 (s, 3 H), 2.51 - 2.65 (m, 2 H), 3.42 (t, J = 6.9 Hz, 2 H), 3.86 (m, 6 H), 5.82 (s, 1 H), 6.29 (s, 1 H) .

### Step C. 4-Bromo-2-(10-bromodecyl)-5,6-dimethoxy-3-methylphenol

2-(10-Bromodecyl)-5,6-dimethoxy-3-methyl-phenol (410 mg, 940.98 umol) and NaBr (145.23 mg, 1.41 mmol) were dissolved in AcOH (10 mL), followed by adding H₂O₂ (160.04 mg, 1.41 mmol, 30%) thereto at 25°C, and then stirred for 2 hours. LCMS showed that the starting material was consumed, and a new peak was formed. The reaction mixture was quenched with water (50 mL) and extracted with EtOAc (40 mL * 2). The combined organic layer was washed with saturated NaHCO₃ (40 mL) to a pH greater than 7, dried over Na₂SO₄, and concentrated into a colorless oil (300 mg, crude).

MS (ESI) : mass calcd. for C₁₉H₃₀Br₂O₃, 464.06; m/z found, 466.9 [M+H]+.

1H NMR (400 MHz, CDCl3) δ 1.22 - 1.55 (m, 14 H), 1.86 (quin, J = 7.1 Hz, 2 H), 2.26 (s, 3 H), 2.51 - 2.65 (m, 2 H), 3.42 (t, J = 6.9 Hz, 2 H), 3.85 (s, 3 H), 3.93 (s, 3 H), 5.77 (s, 1 H) .

### Step D. (10-(3-Bromo-4,5,6-trimethoxy-2-methylphenyl)decyl)triphenylphosphonium bromide

4-Bromo-2-(10-bromodecyl)-5,6-dimethoxy-3-methyl-phenol (300 mg, 597.11 umol) and PPh₃ (939.68 mg, 3.58 mmol) were dissolved in toluene (1 mL) and then stirred at 130°C under N₂ for 18 hours. TLC (DCM:MeOH = 10:1, Rf = 0.2) showed that one main peak was formed under OPPh₃. The reaction mixture was evaporated to obtain a brown residue, which was purified by Prep-HPLC (Column: 3_Phenomenex Luna C18 75 * 30 mm * 3 um; mobile phase: [water(0.2%FA)-ACN]; B%: 52%-82%, 6 minutes). After lyophilization, the desired product was obtained as a white solid (16 mg, 12.24% yield, 97.2% purity).

MS (ESI) : mass calcd. for C₃₇H₄₅BrO₃P+, 647.23; m/z found, 649.3 [M+H]+.

1H NMR (400 MHz, CHLOROFORM-d) δ 1.13 - 1.70 (m, 16 H), 2.34 (s, 3 H), 2.56 - 2.76 (m, 2 H), 3.65 - 3.79 (m, 2 H), 3.68 - 3.77 (m, 1 H), 3.83 (s, 3 H), 3.88 (s, 3 H), 7.61 - 7.93 (m, 15 H), 8.76 (s, 1 H); 31P NMR (162 MHz, CHLOROFORM-d) δ 24.47 (s, 1 P).

### 1-3. Synthesis of (10-(2-bromo-5-hydroxy-3,4- dimethoxy-6-methylphenyl)decyl)triphenylphosphonium formate

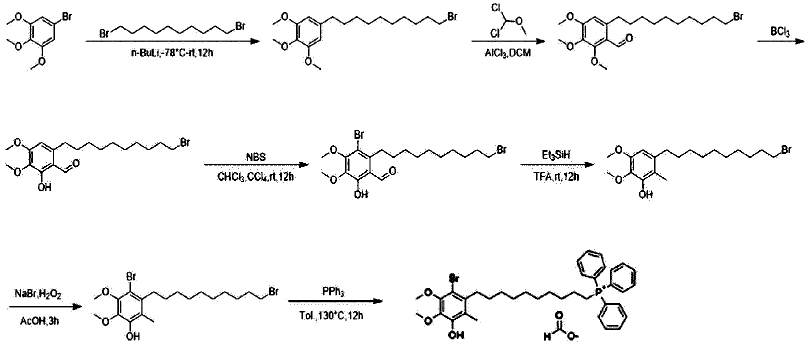

### Step A. 5-(10-Bromodecyl)-1,2,3-trimethoxy-benzene

To a solution of 5-bromo-1,2,3-trimethoxy-benzene (1.3 g, 5.26 mmol, 1 eq) in THF (20 mL), n-BuLi (2.5 M, 2.10 mL, 1 eq) was added dropwise at 78°C. After addition, the obtained mixture was stirred at the same temperature for 1 hour, followed by adding a solution of 1,10-dibromodecane (3.16 g, 10.52 mmol, 2 eq) in THF (10 mL) dropwise thereto at -78°C, and then stirred at 20°C for 11 hours. LCMS showed that 50.6% of the desired mass was detected. The residue was diluted with saturated NH₄Cl (10 mL) and extracted with EtOAc (50 mL * 3). The combined organic layer was dried over Na₂SO₄ and then filtered and concentrated under reduced pressure to obtain a residue. The residue was purified by column chromatography (SiO₂, petroleum ether/ethyl acetate = 100/0 to 95/5). A compound, 5-(10-bromodecyl)-1,2,3-trimethoxy-benzene (580 mg, 1.02 mmol, 19.35% yield, 68% purity), was obtained as a colorless oil.

MS (ESI) : mass calcd. for C₁₉H₃₁BrO₃, 387.4; m/z found, 387.1 [M+H]+.

1H NMR (400 MHz, CDCl3) δ = 6.40 (s, 2H), 3.86 (s, 6H), 3.83 (s, 3H), 3.42 (t, J = 6.8 Hz, 2H), 2.59 - 2.52 (m, 2H), 1.86 (quin, J = 7.2 Hz, 2H), 1.60 (br d, J = 5.5 Hz, 2H), 1.48 - 1.38 (m, 2H), 1.38 - 1.26 (m, 10H)

### Step B. 6-(10-Bromodecyl)-2,3,4-trimethoxy-benzaldehyde

A dry solution of 5-(10-bromodecyl)-1,2,3-trimethoxy-benzene (580 mg, 1.02 mmol, 1 eq) in CH₂Cl₂ (2 mL) was gradually added dropwise to a dry solution of AlCl₃ (239 mg, 1.79 mmol, 97.95 uL, 1.76 eq) in CH₂Cl₂ (8 mL) at 0°C. The obtained mixture was stirred at the same temperature for 45 minutes, followed by gradually adding a dry solution of dichloro(methoxy)methane (188.97 mg, 1.64 mmol, 145.36 uL, 1.61 eq, 68% purity) in CH₂Cl₂ (2 mL) dropwise thereto for 10 minutes. The obtained mixture was stirred at 0°C for 2 hours and 5 minutes. LCMS showed that the reaction was completed. The reaction mixture was poured into 30 mL of ice water, the methylene chloride phase was separated, and the aqueous phase was extracted twice with 50 mL of methylene chloride. The combined organic layer was dried over Na₂SO₄ and then filtered and concentrated under reduced pressure to obtain a residue. The resulting crude product was used in the next step without being additionally purified. A compound, 6-(10-bromodecyl)-2,3,4-trimethoxy-benzaldehyde (510 mg, 858.27 umol, 84.29% yield, 69.9% purity), was obtained as a colorless oil.

MS (ESI) : mass calcd. for C₂₀H₃₁BrO₄, 415.4; m/z found, 415. 1 [M+H]+.

1H NMR (400 MHz, CDCl3) δ = 10.41 (s, 1H), 6.53 (s, 1H), 4.00 (s, 3H), 3.95 (s, 3H), 3.89 (s, 3H), 3.43 (t, J = 6.9 Hz, 2H), 2.99 - 2.92 (m, 2H), 1.93 - 1.82 (m, 2H), 1.49 - 1.39 (m, 4H), 1.32 (br s, 10H)

### Step C. 6-(10-Bromodecyl)-2-hydroxy-3,4-dimethoxybenzaldehyde

BCl3 (1 M, 1.9 mL, 2.21 eq) was added dropwise to a solution of 6-(10-bromodecyl)-2,3,4-trimethoxy-benzaldehyde (510.00 mg, 858.27 umol, eq, 69.9% purity) in CH₂Cl₂ (10 mL) at 0°C. The obtained mixture was stirred at 0°C for 30 minutes and then stirred at 20°C for 30 minutes. LCMS showed that the reaction was completed. The resulting residue was poured into ice water (30 mL) and extracted with CH₂Cl₂ (50 mL * 3). The combined organic layer was dried over Na₂SO₄ and then filtered and concentrated under reduced pressure to obtain a residue. The residue was purified by column chromatography (SiO₂, petroleum ether/ethyl acetate = 100/0 to 95/5). A compound, 6- (10-bromodecyl)-2-hydroxy-3,4-dimethoxy-benzaldehyde (300 mg, 583.06 umol, 67.93% yield, 78% purity), was obtained as a colorless oil.

MS (ESI) : mass calcd. for C₁₉H₂₉BrO₄, 401. 3; m/z found, 401.1 [M+H]+.

1H NMR (400 MHz, CDCl3) δ = 12.30 - 12.20 (m, 1H), 10.24 - 10.03 (m, 1H), 6.34 (s, 1H), 3.96 (s, 3H), 3.89 (s, 3H), 3.43 (t, J = 6.9 Hz, 2H), 2.90 - 2.83 (m, 2H), 1.88 (quin, J = 7.1 Hz, 2H), 1.70 - 1.60 (m, 2H), 1.50 - 1.38 (m, 3H), 1.49 - 1.29 (m, 1H)

### Step D. 3-Bromo-2-(10-bromodecyl)-6-hydroxy-4,5-dimethoxy-benzaldehyde

NBS (133.04 mg, 747.51 umol, 1.2 eq) was added to a solution of 6-(10-bromodecyl)-2-hydroxy-3,4-dimethoxy-benzaldehyde (250 mg, 622.92 umol, 1 eq) in CCl₄ (2.5 mL) and CHCl₃ (2.5 mL) at 0°C. The obtained mixture was stirred at 0°C for 1 hour and then stirred at 20°C for 11 hours. LCMS showed that the reaction was completed. The resulting mixture was diluted with saturated NaHCO₃ (10 mL) and extracted with EtOAc (20 mL * 3) . The combined organic layer was dried over Na₂SO₄ and then filtered and concentrated under reduced pressure to obtain a residue. The residue was purified by prep-TLC (SiO2, petroleum ether/ethyl acetate = 4:1). A compound, 3-bromo-2-(10-bromodecyl)-6-hydroxy-4,5-dimethoxy-benzaldehyde (200 mg, 307.77 umol, 49.41% yield, 73.9% purity), was obtained as a yellow oil.

MS (ESI): mass calcd. for C₁₉H₂₈Br₂O₄, 480.2; m/z found, 481.0 [M+H]+.

### Step E. 5-(10-Bromodecyl)-2,3-dimethoxy-6-methyl-phenol

TFA (708.40 mg, 6.21 mmol, 460 uL, 21.25 eq) was added dropwise to a solution of Et₃SiH (169.99 mg, 1.46 mmol, 233.50 uL, 5 eq) and 3-bromo-2-(10-bromodecyl)-6-hydroxy-4,5-dimethoxy-benzaldehyde (190 mg, 292.38 umol, 1 eq, 73.9% purity) in CH₂Cl₂ (4 mL) at 0°C using an addition funnel over 5 minutes. The reaction mixture was stirred at 0°C for 2 hours. LCMS showed that the reaction was completed. The obtained mixture was slowly poured into saturated NaHCO3 (50 mL) and extracted with 100 mL of CH₂Cl₂ (100 mL * 3). The combined organic layer was dried over Na₂SO₄ and then filtered and concentrated under reduced pressure to obtain a residue. The residue was purified by prep-TLC (SiO2, petroleum ether:ethyl acetate = 4:1). A compound, 5-(10-bromodecyl)-2,3-dimethoxy-6-methyl-phenol (130 mg, 241.64 umol, 82.65% yield, 72% purity), was obtained as a colorless oil.

### Step F. 4-Bromo-5-(10-bromodecyl)-2,3-dimethoxy-6-methylphenol

H₂O₂ (41.09 mg, 362.46 umol, 34.82 uL, 30% purity, 1.5 eq) was added to a stirred solution of NaBr (37.29 mg, 362.46 umol, 11.65 uL, 1.5 eq) and 5-(10-bromodecyl)-2,3-dimethoxy-6-methylphenol (130 mg, 241.64 umol, 1 eq, 72% purity) in AcOH (5 mL) and then stirred at 20°C for 3 hours. LCMS showed that the reaction was completed. The residue was diluted with saturated NaHCO₃:Na₂S₂O₃ = 10:1 (30) mL and extracted with EtOAc (30 mL * 3). The combined organic layer was washed with brine (10 mL), dried over Na₂SO₄, and then filtered and concentrated under reduced pressure to obtain a residue. The resulting crude product was used in the next step without being additionally purified. A compound, 4-bromo-5-(10-bromodecyl)-2,3-dimethoxy-6-methyl-phenol (140 mg, 195.18 umol, 80.77% yield, 65% purity), was obtained as a yellow oil.

MS (ESI): mass calcd. for C₁₉H₃₀Br₂O₃, 466.3; m/z found, 466.9 [M+H]+.

1H NMR (400 MHz, CDCl3) δ = 5.73 (s, 1H), 3.86 (s, 3H), 3.78 (s, 3H), 3.34 (t, J = 6.9 Hz, 2H), 2.71 - 2.64 (m, 2H), 2.14 (s, 3H), 1.84 - 1.76 (m, 2H), 1.37 (br d, J = 4.1 Hz, 7H), 1.24 (br s, 7H)

### Step G. (10-(2-Bromo-5-hydroxy-3,4-dimethoxy-6-methylphenyl)decyl)triphenylphosphonium formate

A stirred solution of PPh₃ (255.96 mg, 975.88 umol, 5 eq) and 4-bromo-5-(10-bromodecyl)-2,3-dimethoxy-6-methyl-phenol (140 mg, 195.18 umol, 1 eq, 65% purity) in toluene (2 mL) was heated at 125°C under N₂ for 8 hours. LCMS showed that the reaction was completed. The solvent was removed in vacuo to obtain a residue. The residue was purified by column chromatography (SiO₂, petroleum ether/ethyl acetate = 100/0 to 0/100; ethyl acetate:MeOH = 100/0 to 92/8). The residue was purified by prep-HPLC (FA condition; column: Xtimate C18 100 * 30 mm * 3 um; mobile phase: [water(0.225% FA)-ACN]; B%: 40%-70%, 8 minutes). A compound, (10-(2-bromo-5-hydroxy-3,4-dimethoxy-6-methylphenyl)decyl)triphenylphosphonium formate (6 mg, 8.61 umol, 4.41% yield, 99.54% purity), was obtained as a colorless gum.

MS (ESI): mass calcd. for C₃₇H₄₅BrO₃P+, 648.6; m/z found, 649.2 [M+H]+.

1H NMR (400 MHz, CDCl₃) δ = 8.56 (br s, 1.309H), 7.78 - 7.59 (m, 15H), 3.84 (s, 3H), 3.76 (s, 3H), 3.44 (br s, 2H), 2.70 - 2.59 (m, 2H), 2.13 (s, 3H), 1.50 (br s, 4H), 1.40 - 1.12 (m, 12H)

31P NMR (162 MHz, CDCl3) δ = 24.17 (s, 1P)

### 1-4. Synthesis of (10-(3-bromo-4,5,6-trimethoxy-2-methylphenyl)decyl)triphenylphosphonium bromide

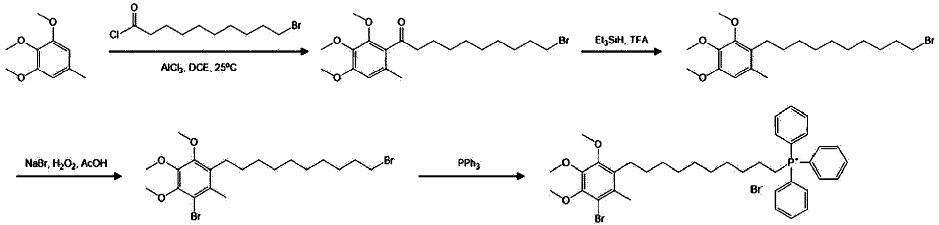

### Step A. 10-Bromo-1-(2,3,4-trimethoxy-6-methylphenyl)decan-1-one

AlCl₃ (206.41 mg, 1.55 mmol was added to a stirred solution of 10-bromodecanoyl chloride (536.94 mg, 1.89 mmol) and 4-bromo-1,2,3-trimethoxy-5-methyl-benzene (449.11 mg, 1.72 mmol) in DCE (10 mL) and then stirred at 25°C for 18 hours. LCMS showed that the desired product was formed as a major component. TLC (petroleum ether:EtOAc = 3:1, Rf = 0.4) showed that one major new spot was formed. The reaction mixture was poured into ice water, extracted with DCM (30 mL × 3), dried over Na₂SO₄, and concentrated to obtain a yellow oil, which was purified by flash column on silica gel (0 to 100% EtOAc in petroleum ether over 30 minutes) to obtain a colorless oil (215 mg, 29.1% yield).

MS (ESI) : mass calcd. for C₂₀H₃₁BrO₄, 414.14; m/z found, 416.8 [M+H]+.

1H NMR (400 MHz, CDCl3) δ 1.32 (m, 8 H), 1.38 - 1.49 (m, 2 H), 1.67 (m, 2 H), 1.86 (quin, J = 7.2 Hz, 2 H), 2.19 (s, 3 H), 2.75 (t, J = 7.4 Hz, 2 H), 3.41 (t, J = 6.9 Hz, 2 H), 3.77 - 3.92 (m, 9 H), 6.48 (s, 1 H) .

### Step B. 4-(10-Bromodecyl)-1,2,3-trimethoxy-5-methylbenzene

Et₃SiH (1.46 g, 12.52 mmol, 2 mL) was added to a stirred solution of 10-bromo-1-(2,3,4-trimethoxy-6-methyl-phenyl)decan-1-one (210 mg, 455.03 umol) in TFA (10 mL) at 25°C and then stirred at 80°C for 2 hours. LCMS showed that the desired product was produced as a major component. TLC (petroleum ether:EtOAc = 4:1, Rf = 0.45) showed that one major new spot was formed. The reaction mixture was evaporated to dryness in vacuo to obtain a colorless oil, which was additionally purified by flash column chromatography on silica gel (25 g, 0 to 50% EtOAc in petroleum ether over 30 minutes). The desired product, 4-(10-bromodecyl)-1,2,3-trimethoxy-5-methyl-benzene (118 mg, 250.93 umol, 55.15% yield), was obtained as a colorless oil.

MS (ESI) : mass calcd. for C20H33BrO3, 400.16; m/z found, 403.0 [M+H]+.

1H NMR (400 MHz, CDCl3) δ 1.20 - 1.54 (m, 14 H), 1.77 - 1.96 (m, 2 H), 2.27 (s, 3 H), 2.46 - 2.64 (m, 2 H), 3.42 (t, J = 6.8 Hz, 2 H), 3.76 - 3.97 (m, 9 H), 6.49 (s, 1 H).

### Step C. 1-Bromo-5-(10-bromodecyl)-2,3,4-trimethoxy-6-methyl-benzene

H₂O₂ (42.68 mg, 376.39 umol) was added to a stirred solution of NaBr (38.73 mg, 376.39 umol) and 4-(10-bromodecyl)-1,2,3-trimethoxy-5-methyl-benzene (118 mg, 250.93 umol) in AcOH (5 mL) and then stirred at 25°C for 2 hours. LCMS showed that the desired product was formed as a major component. The reaction mixture was distributed in between EtOAc/H₂O (80 mL/60 mL). The organic layer was washed with saturated aqueous NaHCO₃ (60 mL) to a pH greater than 7. The collected organic layer was dried over Na₂SO₄ and concentrated to obtain a yellow oil (140 mg, crude). HNMR showed that the obtained oil was consistent with the desired product with sufficient purity for the next step.

MS (ESI) : mass calcd. for C₂₀H₃₂Br₂O₃, 478.07; m/z found, 481.0 [M+H]+.

1H NMR (400 MHz, CDCl3) δ 1.20 - 1.52 (m, 14 H), 1.78 - 1.94 (m, 2 H), 2.36 (s, 3 H), 2.62 (m, 2 H), 3.42 (t, J = 6.9 Hz, 2 H), 3.81 - 3.98 (m, 9 H).

### Step D. (10-(3-Bromo-4,5,6-trimethoxy-2-methylphenyl)decyl)triphenylphosphonium bromide

A stirred solution of PPh₃ (366.06 mg, 1.40 mmol) and 1-bromo-5-(10-bromodecyl)-2,3,4-trimethoxy-6-methyl-benzene (140 mg, 279.13 umol) in toluene (1 mL) was heated at 130°C under N₂ for 18 hours. LCMS showed that the desired product was formed. TLC (DCM:MeOH = 10:1, Rf = 0.2) showed that one major new peak was formed under OPPh₃. The reaction mixture was evaporated to obtain a brown residue, which was purified by flash column chromatography on silica gel (25 g, 0-15% MeOH in DCM over 30 minutes). After lyophilization, the desired product was obtained as a white solid (108.5 mg, 51.41% yield, 98.2% purity).

MS (ESI) : mass calcd. for C₃₈H₄₃BrO₃P+, 661.24; m/z found, 663.3 [M+H]+.

1H NMR (400 MHz, CHLOROFORM-d) δ 1.12 - 1.50 (m, 12 H), 1.64 (m, 4 H), 2.34 (s, 3 H), 2.52 - 2.71 (m, 2 H), 3.77 - 3.97 (m, 11 H), 7.60 - 7.97 (m, 15 H); 31P NMR (162 MHz, CHLOROFORM-d) δ 24.53 (s, 1 P).

### 1-5. Synthesis of (10-(2-bromo-3,4,5-trimethoxy-6-methylphenyl)decyl)triphenylphosphonium bromide

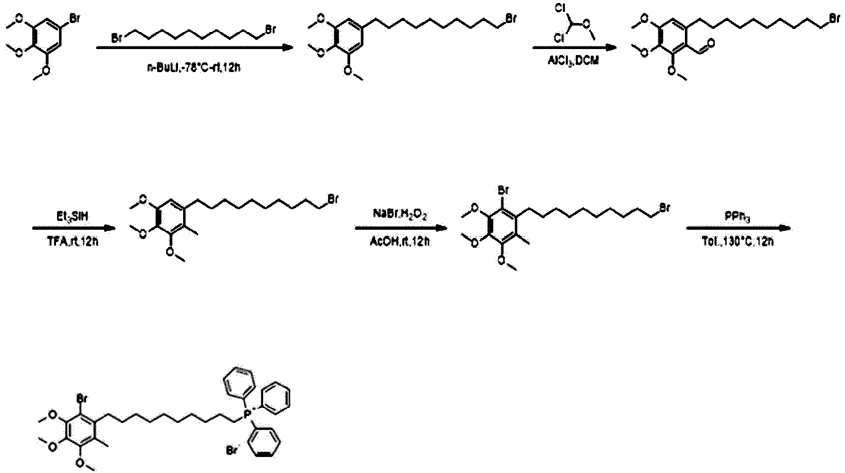

### Step A. 5-(10-Bromodecyl)-1,2,3-trimethoxy-benzene

To a solution of 5-bromo-1,2,3-trimethoxy-benzene (2 g, 8.09 mmol, 1 eq) in THF (30 mL), n-BuLi (2.5 M, 3.24 mL, 1 eq) was added dropwise at 78°C. After addition, the obtained mixture was stirred at the same temperature for 1 hour, followed by adding a solution of 1,10-dibromodecane (4.86 g, 16.19 mmol, 2 eq) in THF (10 mL) dropwise thereto at -78°C. The resulting mixture was stirred at 20°C for 11 hours. LCMS showed that 20% of the desired mass was detected. The residue was diluted with saturated NH4Cl (10 mL) and extracted with EtOAc (50 mL * 3) . The combined organic layer was dried over Na₂SO₄, filtered, and concentrated under reduced pressure to obtain a residue. The residue was purified by column chromatography (SiO2, petroleum ether/ethyl acetate = 100/0 to 95/5). A compound, 5-(10-bromodecyl)-1,2,3-trimethoxy-benzene (430 mg, 395.86 umol, 4.89% yield, 35.66% purity), was obtained as a colorless oil.

MS (ESI) : mass calcd. for C19H31BrO3, 387.4; m/z found, 389.1 [M+H]+.

### Step B. 6-(10-Bromodecyl)-2,3,4-trimethoxy-benzaldehyde

A dry solution of AlCl₃ (178 mg, 1.33 mmol, 72.95 uL, 3.37 eq) in CH₂Cl₂ (6 mL) was gradually added dropwise to a dry solution of 5-(10-bromodecyl)-1,2,3-trimethoxy-benzene (430 mg, 395.86 umol, 1 eq, 35.66% purity) in CH₂Cl₂ (2 mL) at 0°C. The obtained mixture was stirred at the same temperature for 45 minutes, followed by gradually adding a dry solution of dichloro(methoxy)methane (140 mg, 1.22 mmol, 107.69 uL, 3.08 eq) in CH₂Cl₂ (2 mL) dropwise thereto for 10 minutes. The obtained mixture was stirred at 0°C for 2 hours and 5 minutes. LCMS showed that the reaction was completed. The reaction mixture was poured into 30 mL of ice water, the methylene chloride phase was separated, and the aqueous phase was extracted twice with 50 mL of methylene chloride. The combined organic layer was dried over Na₂SO₄, filtered, and concentrated under reduced pressure to obtain a residue. The resulting crude product was used in the next step without being additionally purified. A compound, 6-(10-bromodecyl)-2,3,4-trimethoxy-benzaldehyde (410 mg, 384.97 umol, 97.25% yield, 39% purity), was obtained as a colorless oil.

MS (ESI): mass calcd. for C₂₀H₃₁BrO₄, 415.4; m/z found, 415.2 [M+H]+.

### Step C. 1-(10-Bromodecyl)-3,4,5-trimethoxy-2-methylbenzene

TFA (3 mL) was added to a mixture of 6-(10-bromodecyl)-2,3,4-trimethoxy-benzaldehyde (410 mg, 384.97 umol, 1 eq, 39% purity) and Et₃SiH (447.64 mg, 3.85 mmol, 614.89 uL, 10 eq). The obtained mixture was stirred at 20°C for 12 hours. LCMS showed that the reaction was completed. The obtained mixture was slowly poured into saturated NaHCO3 (50 mL) and extracted with CH2Cl2 (50 mL * 3). The combined organic layer was dried over Na₂SO₄, filtered, and concentrated under reduced pressure to obtain a residue. The residue was purified by prep-TLC (SiO2, petroleum ether/ethyl acetate = 4:1). A compound, 1-(10-bromodecyl)-3,4,5-trimethoxy-2-methyl-benzene (120 mg, 152.18 umol, 39.53% yield, 50.9% purity), was obtained as a colorless oil.

MS (ESI): mass calcd. for C20H33BrO3, 401.4; m/z found, 402.8 [M+H]+.

### Step D. 1-Bromo-2-(10-bromodecyl)-4,5,6-trimethoxy-3-methyl-benzene

H2O2 (17.25 mg, 152.18 umol, 14.62 uL, 30% purity, 1 eq) was added to a stirred solution of NaBr (15.66 mg, 152.18 umol, 4.89 uL, 1 eq) and 1-(10-bromodecyl)-3,4,5-trimethoxy-2-methylbenzene (120 mg, 152.18 umol, 1 eq, 50.9% purity) in AcOH (4 mL) and then stirred at 20°C for 12 hours. LCMS showed that the reaction was completed. The residue was diluted with saturated NaHCO₃:Na₂S₂O₃ = 10:1 (30) mL and extracted with EtOAc (30 mL * 3). The combined organic layer was washed with brine (10 mL), dried over Na₂SO₄, filtered, and concentrated under reduced pressure to obtain a residue. The resulting crude product was used in the next step without being additionally purified. A compound, 1-bromo-2-(10-bromodecyl)-4,5,6-trimethoxy-3-methyl-benzene (130 mg, crude), was obtained as a yellow oil.

MS (ESI): mass calcd. for C₂₀H₃₂Br₂O₃, 480.3; m/z found, 480.9 [M+H]+.

### Step E. 1-Bromo-2-(10-BLAHdecyl)-4,5,6-trimethoxy-3-methyl-benzene

A stirred solution of PPh₃ (212.99 mg, 812.04 umol, 5 eq) and 1-bromo-2-(10-bromodecyl)-4,5,6-trimethoxy-3-methylbenzene (130 mg, 162.41 umol, 1 eq, 60% purity) in toluene (2 mL) was heated at 125°C under N₂ for 12 hours. LCMS showed that the reaction was completed. The solvent was removed in vacuo to obtain a residue. The residue was purified by column chromatography (SiO2, petroleum ether/ethyl acetate = 100/0 to 0/100; ethyl acetate:MeOH = 100/0 to 92/8). A compound, 1-bromo-2-(10-BLAHdecyl)-4,5,6-trimethoxy-3-methyl-benzene (30 mg, 39.69 umol, 24.44% yield, 98.234% purity), was obtained as a colorless oil.

MS (ESI): mass calcd. for C₃₈H₄₇BrO₃P+, 662.7; m/z found, 663.2 [M+H]+.

1H NMR (400 MHz, CDCl₃) δ = 7.93 - 7.66 (m, 15H), 3.94 - 3.78 (m, 11H), 2.78 - 2.67 (m, 2H), 2.22 (s, 3H), 1.64 (br s, 4H), 1.50 - 1.34 (m, 4H), 1.25 (br d, J = 10.1 Hz, 8H) 31P NMR (162 MHz, CDCl3) δ = 24.54 (s, 1P)

### 1-6. Synthesis of formic acid and salt of triphenyl(10-(2,3,4,5-tetramethoxy-6-methylphenyl) decyl)phosphonium

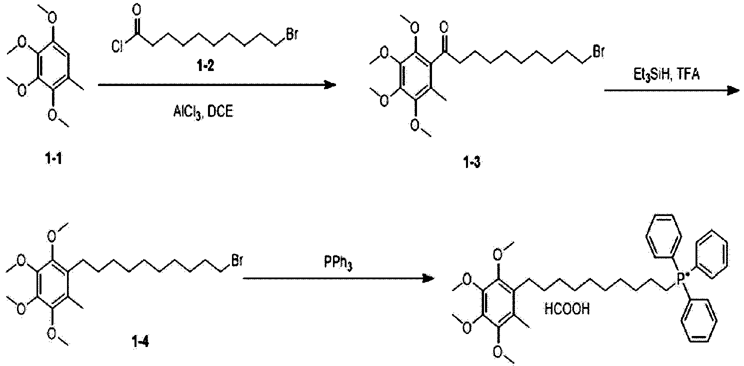

### Step A. 10-Bromo-1-(2,3,4,5-tetramethoxy-6-methylphenyl)decan-1-one

AlCl₃ (401.45 mg, 3.01 mmol, 164.53 uL, 0.9 eq) was added to a stirred solution of 10-bromodecanoyl chloride (992.09 mg, 3.68 mmol, 1.1 eq) and 1,2,3,4-tetramethoxy-5-methyl-benzene (710 mg, 3.35 mmol, 1 eq) in DCE (15 mL) and then stirred at 25°C for 18 hours. LCMS showed that the starting material was completely consumed. The reaction mixture was extracted into 10 mL of H₂O with DCM (15 mL * 3). The combined organic layer was then evaporated to dryness to obtain a product. The residue was purified by flash silica gel chromatography (ISCO^{®}; 20 g SepaFlash^{®} Silica Flash Column, Eluent of 15 to 20% ethyl acetate/petroleum ether gradient at 45 mL/min). A compound, 10-bromo-1-(2,3,4,5-tetramethoxy-6-methyl-phenyl)decan-1-one (800 mg, crude), was obtained as a yellow oil.

MS (ESI) : mass calcd. for C₂₁H₃₃BrO₅, 444.15; m/z found, 445.2 [M+H]+.

### Step B. 1-(10-Bromodecyl)-2,3,4,5-tetramethoxy-6-methylbenzene

Triethylsilane (2.55 g, 21.91 mmol, 3.50 mL, 27.89 eq) was added to a stirred solution of 10-bromo-1-(2,3,4,5-tetramethoxy-6-methyl-phenyl)decan-1-one (350 mg, 785.83 umol, 1 eq) in TFA (15 mL) at 25°C and then stirred at 80°C for 2 hours. LCMS showed that the starting material was completely consumed. The reaction mixture was concentrated in vacuo to obtain a crude product. The residue was purified by flash silica gel chromatography (ISCO^{®}; 40 g SepaFlash^{®} Silica Flash Column, Eluent of 15 to 20% ethyl acetate/petroleum ether gradient at 45 mL/min) . A compound, 1-(10-bromodecyl)-2,3,4,5-tetramethoxy-6-methyl-benzene (150 mg, 347.70 umol, 44.25% yield), was obtained as a colorless oil.

MS (ESI) : mass calcd. for C₂₁H₃₅BrO₄, 430.17; m/z found, 433.2 [M+3]+.

### Step C. Formic acid and salt of triphenyl(10-(2,3,4,5-tetramethoxy-6-methylphenyl) decyl)phosphonium

A stirred solution of PPh3 (344.51 mg, 1.31 mmol, 4.36 eq) and 1-(10-bromodecyl)-2,3,4,5-tetramethoxy-6-methyl-benzene (130 mg, 301.34 umol, 1 eq) in toluene (1 mL) was heated at 130°C under N₂ for 18 hours. LCMS showed that the starting material was completely consumed. The reaction mixture was concentrated in vacuo to obtain a crude product. The residue was purified by prep-HPLC ((FA condition), column: Xtimate C18 100 * 30 mm * 10 um; mobile phase: [water(0.225% FA)-ACN];B%: 40% to 70%, 10 minutes). A compound, triphenyl-[10-(2,3,4,5-tetramethoxy-6-methyl-phenyl)decyl]phosphonium (64.7 mg, 103.30 umol, 34.28% yield, 98% purity), was obtained as a yellow oil.

MS (ESI) : mass calcd. for C₃₉H₅₀O₄P+, 613.34; m/z found, 613.6 [M+H]+.

1H NMR (400 MHz, DMSO-d6) δ ppm 1.17 - 1.37 (m, 12 H) 1.40 - 1.59 (m, 4 H) 2.04 - 2.11 (m, 3 H) 2.49 (br s, 2 H) 3.55 - 3.60 (m, 2 H) 3.65 - 3.68 (m, 3H) 3.69 - 3.72 (m, 3 H) 3.76 - 3.81 (m, 6 H) 7.72 - 7.84 (m, 12 H) 7.87 - 7.97 (m, 3 H) 8.21 - 8.43 (m, 1 H)

1-7. Synthesis of formic acid and salt of (10-(4,5-dimethoxy-2-methylphenyl)decyl)triphenylphosphonium

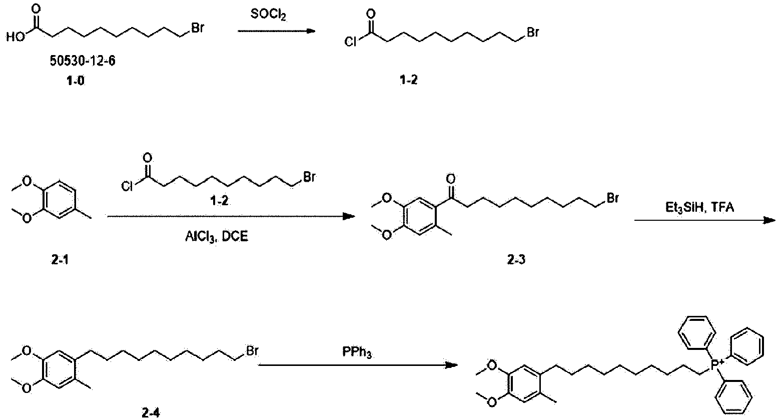

### Step A. 10-Bromodetanoyl chloride

SOCl₂ (947.36 mg, 7.96 mmol, 577.66 uL, 4 eq) was added to a mixture of 10-bromodecanoic acid (500 mg, 1.99 mmol, 1 eq) in DCM (4 mL). The reaction mixture was stirred at 25°C for 2 hours. TLC showed that the starting material was completely consumed. The reaction mixture was concentrated in vacuo. The resulting crude product was not purified. A compound, 10-bromodecanoyl chloride (500 mg, crude), was obtained as an orange oil.

### Step B. 10-Bromo-1-(4,5-dimethoxy-2-methylphenyl)decan-1-one

AlCl₃ (197.13 mg, 1.48 mmol, 80.79 uL, 0.9 eq) was added to a solution of 10-bromodecanoyl chloride (487.17 mg, 1.81 mmol, 1.1 eq) and 1,2-dimethoxy-4-methyl-benzene (250 mg, 1.64 mmol, 1 eq) in DCE (10 mL). The obtained mixture was stirred at 25°C for 16 hours. LCMS showed that the starting material was completely consumed. The reaction mixture was quenched with H₂O (10 mL) and then filtered. The reaction filtrate was extracted with DCM (20 mL * 3). The organic layer was separated, dried over Na₂SO₄, filtered, and concentrated under reduced pressure to obtain a residue. The residue was purified by flash silica gel chromatography (ISCO^{®}; 12 g SepaFlash^{®} Silica Flash Column, Eluent of 0 to 20% ethyl acetate/petroleum ether gradient at 80 mL/minute). A compound, 10-bromo-1-(4,5-dimethoxy-2-methyl-phenyl)decan-1-one (300 mg, 737.59 umol, 44.90% yield, 94.740% purity), was obtained as a white solid.

MS (ESI): mass calcd. for C₁₉H₂₉BrO₃, 384.13; m/z found, 387.0 [M+3]+.

1H NMR (400 MHz, CHLOROFORM-d) δ ppm 1.32 - 1.38 (m, 2 H) 1.52 - 1.69 (m, 4 H) 1.78 (quin, J = 7.13 Hz, 4 H) 2.28 (t, J=7.50 Hz, 2 H) 2.42 (s, 3 H) 2.79 (t, J = 7.38 Hz, 2 H) 3.33 (t, J = 6.82 Hz, 2 H) 3.84 (d, J = 4.25 Hz, 6 H) 6.59 - 6.70 (m, 1 H) 7.10 - 7.17 (m, 1 H)

### Step C. 1-(10-Bromodecyl)-4,5-dimethoxy-2-methylbenzene

Et₃SiH (1.82 g, 15.65 mmol, 2.50 mL, 24.13 eq) was added to a solution of 10-bromo-1-(4,5-dimethoxy-2-methylphenyl)decan-1-one (250 mg, 648.79 umol, 1 eq) in TFA (10 mL). The obtained mixture was stirred at 80°C for 2 hours. LCMS showed that the starting material was completely consumed. The reaction mixture was concentrated in vacuo to obtain a crude product. The residue was purified by flash silica gel chromatography (ISCO^{®}; 20 g SepaFlash ^{®} Silica Flash Column, Eluent of 0 to 15% ethyl acetate/petroleum ether gradient at 80 mL/min). A compound, 1-(10-bromodecyl)-4,5-dimethoxy-2-methylbenzene (130 mg, 350.07 umol, 53.96% yield), was obtained as a colorless oil.

MS (ESI) : mass calcd. for C₁₉H₃₁BrO₂, 370.15; m/z found, 371.2 [M+H]+.

1H NMR (400 MHz, CHLOROFORM-d) δ ppm 1.14 - 1.35 (m, 12 H) 1.41 (dt, J = 15.10, 7.65 Hz, 2 H) 1.73 (quin, J = 7.16 Hz, 2 H) 2.09 - 2.19 (m, 3 H) 2.36 - 2.43 (m, 2 H) 3.28 (t, J = 6.82 Hz, 2 H) 3.72 (d, J = 3.13 Hz, 6 H) 6.43 - 6.62 (m, 2 H)

### Step D. Formic acid and salt of (10-(4,5-dimethoxy-2-methylphenyl)decyl)triphenylphosphonium

PPh₃ (353.16 mg, 1.35 mmol, 5 eq) was added to a solution of 1-(10-bromodecyl)-4,5-dimethoxy-2-methyl-benzene (100 mg, 269.29 umol, 1 eq) in toluene (5 mL). The obtained mixture was stirred at 130°C for 24 hours. LCMS showed that the starting material was completely consumed. The reaction mixture was concentrated in vacuo to obtain a crude product. The residue was purified by prep-HPLC (FA condition: column: Phenomenex Luna C18 75 * 30 mm * 3 um; mobile phase: [water(0.225%FA)-ACN]; B%: 35% to 70%, 35 minutes) to obtain the desired product (40 mg, purity 93.747%) as a white solid, which was additionally separated by prep-HPLC (condition: column: Phenomenex Luna C18 75 * 30 mm * 3 um; mobile phase: [water(0.225%FA)-ACN]; B%: 35% to 70%, 35 minutes). A compound, 10-(4,5-dimethoxy-2-methylphenyl)decyl-triphenyl-phosphonium (17 mg, 27.41 umol, 10.18% yield, 96.703% purity, FA), was obtained as a colorless oil.

MS (ESI) : mass calcd. for C₃₇H₄₆O₂P+, 553.32; m/z found, 553.5 [M+H]+.

1H NMR (400 MHz, DMSO-d6) δ ppm 1.16 - 1.31 (m, 10 H) 1.38 - 1.60 (m, 6 H) 2.16 (s, 3 H) 2.40 - 2.48 (m, 2 H) 3.56 - 3.60 (m, 2 H) 3.69 (d, J = 2.75 Hz, 6 H) 6.68 (s, 1 H) 6.72 (s, 1 H) 7.74 - 7.85 (m, 12 H) 7.87 - 7.94 (m, 3 H) 8.44 (s, 1 H)

### 1-8. Synthesis of (10-(3-methyl-1,4-dioxo-1,4-dihydronaphthalen-2-yl)decyl)triphenylphosphonium

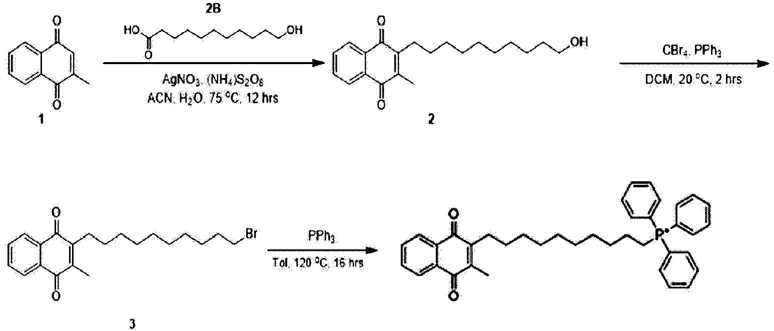

### Preparation procedure of Cpd.2

Cpd.1 (10.0 g, 58.0 mmol, 1.00 eq) and Cpd.2B (12.9 g, 63.8 mmol, 1.10 eq) were added, and then AgNO₃ (9.87 g, 58.0 mmol, 1.00 eq) was put into a round-bottom flask filled with ACN (100 mL) and H₂O (100 mL). A solution of (NH)₄S₂O₈ (15.9 g, 69.6 mmol, 15.1 mL, 1.20 eq) in H₂O (100 mL) was added dropwise to the obtained mixture. The mixture was stirred at 75°C in the dark for 4 hours. LCMS (ET36187-5-P1L, Cpd.2: RT = 1.431 minutes) showed that Cpd.1 was partially consumed, and Cpd.2 was formed. The mixture was cooled to 20°C and then extracted with EtOAc (100.0 mL × 3). The organic layer was washed with NaHCO3 (50.0 mL) and brine (50.0 mL). The organic layer was concentrated in vacuo. The residue was purified by column chromatography (SiO2, petroleum ether/ethyl acetate = 1/0 to 0/1). Cpd.2 (5.91 g, 17.9 mmol, 30.9% yield) was obtained as a white solid.

### Preparation procedure of Cpd.3

Cpd.2 (2.00 g, 6.09 mmol, 1.00 eq) was added to a three-necked round-bottom flask filled with DCM (200.0 mL). CBr4 (2.42 g, 7.31 mmol, 1.20 eq) and PPh3 (1.92 g, 7.31 mmol, 1.20 eq) were added to the obtained mixture. The resulting product was stirred at 20°C for 2 hours. TLC (petroleum ether/ethyl acetate = 15/1 Rf = 0.53) showed that Cpd.2 was consumed, and Cpd.3 was formed. LCMS (ET36249-7-p1a, Cpd.3:RT = 1.678 minutes) showed that Cpd.3 was formed. Then, concentration in vacuo was performed. The residue was purified by column chromatography (SiO2, petroleum ether/ethyl acetate = 2/1 to 15/1). Cpd.3 (1.10 g, 2.81 mmol, 46.1% yield) was obtained as a yellow oil.

1H NMR: ET36249-7-P1a (400 MHz, CDCl3).

δ 8.07~8.09 (m, 2H), 7.68∼7.70 (m, 2H), 3.41 (t, J = 4 Hz, 2H), 2.63 (t, J = 8 Hz, 2H), 2.20 (s, 3H), 1.48 to 1.87 (m, 2H), 1.30 to 1.46 (m, 15H).

### Preparation procedure of target compound

Cpd.3 (0.20 g, 511 umol, 1.00 eq) was added to a one-necked round-bottom flask filled with toluene (1.40 mL). PPh3 (160 mg, 613 umol, 1.20 eq) was added to the obtained mixture. Degassing with N2 was performed three times. The resulting product was stirred at 12°C for 16 hours. TLC (dichloromethane/methanol = 10/1, Rf = 0.50) showed that Cpd.3 was consumed, and the reaction was completed. HPLC (ET36249-13-p1e) showed that the target had a purity of 91.2%. Then, concentration in vacuo was performed to remove toluene (1.40 mL). The residue was filled with MeOH (4.00 mL) and purified by prep-TLC (dichloromethane/methanol = 10/1). The target (12.0 mg, 18.3 umol, 3.59% yield) was obtained as an orange gum.

1H NMR: ET36249-13-P1d (400 MHz, MeOD-d4).

δ 7.88~8.04 (m, 2H), 7.75∼7.87 (m, 15H), 3.42∼3.35 (m, 2H), 2.63 (t, J = 8 Hz, 2H), 2.16 (s, 3H), 1.28∼1.69 (m, 16H).

### 1-9. Synthesis of triphenyl(10-phenyldecyl)phosphonium chloride

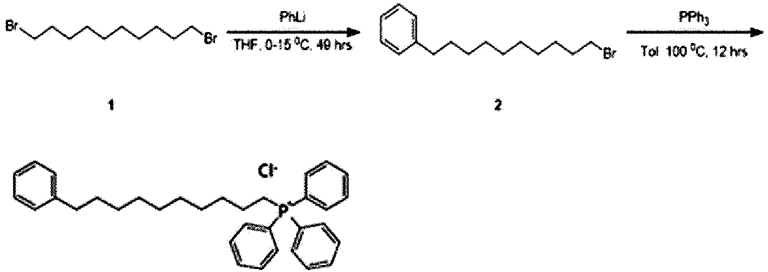

### Preparation procedure of Cpd.2

Cpd.1 (3.00 g, 10.0 mmol, 1.00 eq) was added to THF (7.50 mL) at 0°C. The obtained mixture was degassed three times with N2. PhLi (1.80 M, 1.83 mL, 0.33 eq) was added dropwise to the resulting solution at 0°C, stirred at 0°C for 1 hour, heated to 15°C, and stirred at 15°C for 48 hours. TLC (petroleum ether:ethyl acetate = 1:0, Rf (Cpd.2) = 0.60) showed that Cpd.1 was consumed, and the reaction was completed. Then, concentration in vacuo was performed. Additional purification was not performed. Cpd.2 (2.30 g, crude) was obtained as a colorless oil.

1H NMR: ET41362-1-P1C1 (400 MHz, CDCl3)

δ 7.27-7.26 (m, 2H), 7.19-7.17 (d, J = 8 Hz, 2H), 1.89-1.82 (m, 15H), 1.59-1.52 (m, 9H), 1.30 (s, 32H), 0.94-0.90 (t, J = 8 Hz, 12H).

### Preparation procedure of triphenyl(10-phenyldecyl)phosphonium chloride

Cpd.2 (0.70 M, 11.0 mL, 1.00 eq) was added to toluene (27.0 mL) at 15°C. PPh3 (4.06 g, 15.4 mmol, 2.00 eq) was added to the resulting solution. The obtained mixture was degassed with N2 three times, heated to 100°C, and stirred at 100°C for 12 hours. TLC (dichloromethane:methanol = 20:1, Rf (target 1) = 0.30) showed that Cpd.2 was consumed, and the reaction was completed. Then, concentration in vacuo was performed. The resulting crude product was purified by silica gel chromatography (dichloromethane:methanol = 100:0 to 20:1). Target 1 (27.0 mg, 54.8 umol, 7.09e-1% yield, 97.5% LCMS (ET41362-3-P1J1) purity) was obtained as a yellow oil.

1H NMR: ET41362-3-P1J1 (400 MHz, CDCl3)

δ 7.79-7.71 (d, J = 32 Hz, 15H), 7.28-7.16 (m, 6H), 3.95-3.74 (m, 4H), 2.59-2.55 (t, J = 8 Hz, 2H), 1.62-1.56 (m, 4H), 1.25-1.20 (d, J = 20 Hz, 10H).

### 1-10. Synthesis of (10-cyclohexyldecyl)triphenylphosphonium chloride

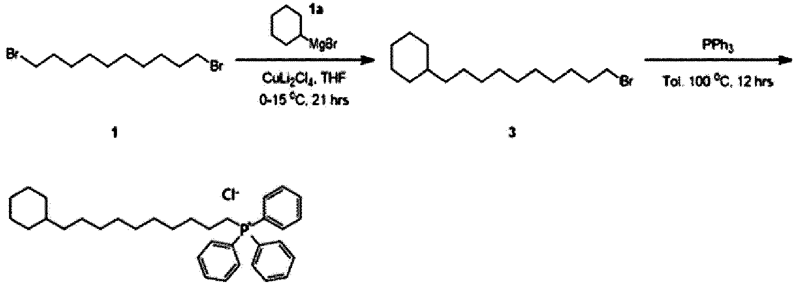

### Preparation procedure of Cpd.3

Cpd.1 (3.00 g, 10.0 mmol, 1.00 eq) was added to THF (3.00 mL) at 0°C. The obtained mixture was degassed three times with N₂. CuLi₂Cₗ₄ (0.10 M, 999 uL, 0.01 eq) was added to the resulting solution. Cpd.1A (1.00 M, 12.0 mL, 1.20 eq) was added dropwise to the resulting solution at 0°C. Then, the obtained solution was stirred at 0°C for 1 hour, heated to 15°C, and stirred at 15°C for 20 hours. TLC (petroleum ether:ethyl acetate = 1:0, Rf (Cpd.3) = 0.80) showed that Cpd.1 was consumed, and the reaction was completed. Then, concentration in vacuo was performed. Additional purification was not performed. Cpd.3 (3.90 g, crude) was obtained as a colorless oil.

1H NMR: ET41362-2-P1C1 (400 MHz, CDCl3)

δ 3.43-3.40 (t, J = 8 Hz, 1H), 1.70-1.67 (m, 15H), 1.20-1.12 (m, 14H), 0.90-0.84 (m, 8H).

### Preparation procedure of (10-cyclohexyldecyl)triphenylphosphonium chloride

Cpd.3 (0.70 M, 18.3 mL, 1.00 eq) was added to toluene (16.0 mL) at 15°C. PPh₃ (6.74 g, 25.7 mmol, 2.00 eq) was added to the resulting solution. The obtained mixture was degassed three times with N₂, heated to 100°C, and stirred at 100°C for 12 hours. TLC (dichloromethane:methanol = 20:1, Rf (target 2) = 0.20) showed that Cpd.3 was consumed, and the reaction was completed. Then, concentration in vacuo was performed. The resulting crude product was purified by silica gel chromatography (dichloromethane:methanol = 100:0 to 20:1). Target 2 (0.02 g, 40.4 umol, 3.14e-1% yield, 98.1% LCMS (ET41362-4-P1J1) purity) was obtained as a yellow oil.

1H NMR: ET41362-4-P1C1 (400 MHz, CDCl3)

δ 7.89-7.70 (m, 15H), 3.83 (s, 2H), 2.60 (s, 2H), 1.68-1.62 (m, 9H), 1.18 (s, 18H), 0.87-0.79 (m, 2H).

### 1-11. Preparation of (10-(3,4-dimethylphenyl)decyl)triphenylphosphonium bromide

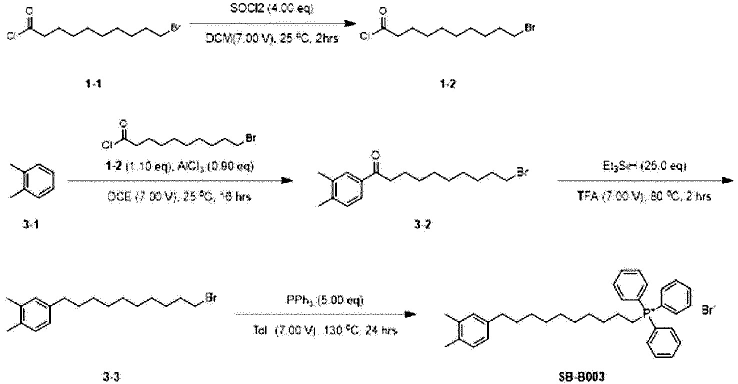

### Step A. 10-Bromodecanoyl chloride

SOCl₂ (56.8 g, 478 mmol, 34.7 mL, 4.00 eq) was added to a solution of 10-bromodecanoic acid (30.0 g, 119 mmol, 1.00 eq) in DCM (210 mL). The obtained mixture was stirred at 25°C for 1 hour. TLC1 (petroleum ether:ethyl acetate = 5:1, Rf (start material) = 0.30, Rf (product) = 0.52) showed that the starting material was completely consumed. The obtained mixture was concentrated in vacuo. As a yellow oil, 10-bromodecanoyl chloride (30.0 g, crude) was obtained.

### Step B. 10-Bromo-1-(3,4-dimethylphenyl)decan-1-one

AlCl₃ (5.65 g, 42.4 mmol, 2.32 mL, 0.90 eq) was added to a solution of 10-bromodecanoyl chloride (14.0 g, 51.8 mmol, 1.10 eq) and o-xylene (5.00 g, 47.1 mmol, 5.69 mL, 1.00 eq) in DCE (35.0 mL). The obtained mixture was stirred at 25°C for 16 hours. TLC (petroleum ether:ethyl acetate = 5:1, starting material Rf = 0.70, Rf (product) = 0.88) showed that the starting material was completely consumed. The obtained mixture was poured into ice water (50.0 mL) and extracted with DCM (50.0 mL). The organic phase was separated and concentrated in vacuo. The residue was purified by column chromatography (SiO2, petroleum ether: ethyl acetate = 1:0 to 1:1). As a yellow solid, 10-bromo-1-(3,4-dimethylphenyl)decan-1-one (6.00 g, 13.4 mmol, 28.3% yield, 75.5% purity) was obtained, which was confirmed by HNMR (ET47086-1-P1A2) and LCMS (ET47086-1-P1A1, T = 0.996, M + 1 = 339.2) .

1H NMR (400 MHz, CHLOROFORM-d) δ = 7.74 (s, 1H) 7.70 (dd, J = 7.60, 1.64 Hz, 1H) 7.21 (d, J = 7.60 Hz, 1H) 3.41 (t, J = 6.80 Hz, 2H) 2.93 (t, J = 7.60 Hz, 2H) 2.32 (s, 6H) 1.86 (quin, J = 7.20 Hz, 2H) 1.73 (quin, J = 7.20 Hz, 2H) 1.26 - 1.49 (m, 10H)

### Step C. 4-(10-Bromodecyl)-1,2-dimethyl-benzene

Et₃SiH (8.57 g, 73.7 mmol, 11.8 mL, 25.0 eq) was added to a solution of 10-bromo-1-(3,4-dimethylphenyl)decan-1-one (1.00 g, 2.95 mmol, 1.00 eq) in TFA (7.00 mL). The obtained mixture was stirred at 80°C for 2 hours. TLC (petroleum ether:ethyl acetate = 5:1, Rf (start material) = 0.7, Rf (product) = 0.6) showed that the starting material was completely consumed. The mixture was poured into water (10.0 mL) and extracted with EtOAc (5.00 mL × 3). Then, the combined organic layer was washed with brine (10.0 mL). The residue was purified by column chromatography (SiO2, petroleum ether:ethyl acetate = 1:1 to 0:1). As a colorless oil, 4-(10-bromodecyl)-1,2-dimethylbenzene (0.20 g, 615 umol, 20.8% yield) was obtained.

### Step D: Salt of 10-(3,4-dimethylphenyl)decyltriphenylphosphonium bromide

PPh₃ (161 mg, 615 umol, 1.10 eq) was added to a solution of 4-(10-bromodecyl)-1,2-dimethoxy-benzene (200 mg, 559 umol, 1.00 eq) in toluene (7.00 mL). The obtained mixture was stirred at 130°C for 18 hours. TLC1 (dichloromethane:methanol = 5:1, Rf (product) = 0.4) and TLC2 (petroleum ether:ethyl acetate = 5:1, Rf (start material) = 0.8) showed that the starting material was completely consumed. The obtained mixture was concentrated in vacuo. The residue was ground with PE:MTBE = 2:1 (1 mL) at 25°C for 30 minutes. As a white solid, a salt of 10-(3,4-dimethylphenyl)decyltriphenylphosphonium bromide (0.05 g, 84.2 umol, 13.6% yield, 98.9% purity, Br-) was obtained as a white solid, which was confirmed by HNMR (ET46959-5-P1B1), LCMS (ET46959-5-P1B1, T = 2.958, M+ = 507.2, and HPLC (ET46959-5-P1A3, T = 4.121, Purity = 98.9%).

1H NMR (400 MHz, DMSO-d6)

δ = 7.93 - 7.86 (m, 3H), 7.84 - 7.73 (m, 12H), 7.00 (d, J = 7.60 Hz, 1H), 6.92 (s, 1H), 6.86 (br d, J = 7.60 Hz, 1H), 3.56 (br t, J = 14.4 Hz, 2H), 2.48 - 2.43 (m, 2H), 2.16 (d, J = 5.20 Hz, 6H), 1.57 - 1.38 (m, 6H), 1.20 (br d, J = 15.2 Hz, 10H)

### 1-12. Preparation of 10-(2,5-dimethoxy-3,4-dimethylphenyl)decyl-triphenyl-phosphonium

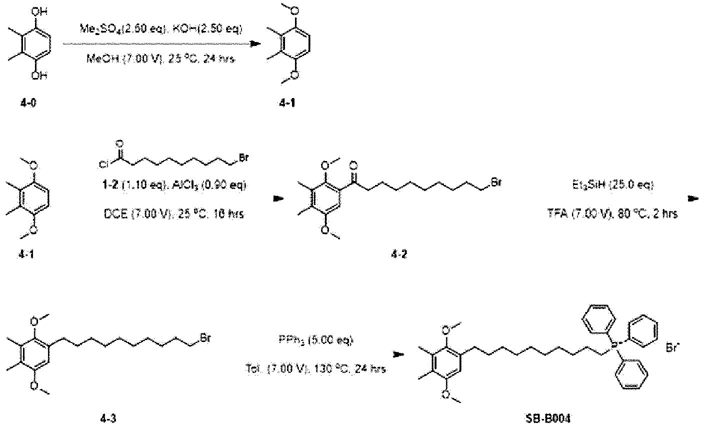

### Step A. 10-Bromo-1-(2,5-dimethoxy-3,4-dimethylphenyl)decan-1-one

KOH (11.2 g, 199 mmol, 2.50 eq) and dimethyl sulfate (25.1 g, 199 mmol, 18.9 mL, 2.50 eq) were added to a solution of 2,3-dimethylbenzene-1,4-diol (11.0 g, 79.6 mmol, 1.00 eq) in EtOH (70.0 mL). The obtained mixture was stirred at 0°C for 3.5 hours. TLC1 (petrileum ether:ethyl acetate = 5:1, Rf (start material) = 0.10, Rf (product) = 0.70) showed that the starting material was completely consumed. The mixture was poured into 3 M HCl (100 mL) and extracted with PE (50.0 mL × 3). Then, the combined organic layer was washed with 1 M HCl (50.0 mL), water (50.0 mL), and brine (50.0 mL). The residue was purified by column chromatography (SiO2, petroleum ether:ethyl acetate = 1:0 to 0:1). As a brown solid, 10-bromo-1-(2,5-dimethoxy-3,4-dimethyl-phenyl)decan-1-one (6.00 g, 36.1 mmol, 45.3% yield) was obtained, which was confirmed by LCMS (ET46959-3-P1A1, T = 0.795, M + H = 167.3) and HNMR (ET46959-3-P1A1).

1H NMR (ET46959-3-P1A1, 400 MHz, DMSO-d6)

δ = 6.71 (s, 2H), 3.70 (s, 6H), 2.06 (s, 6H)

### Step B. 10-Bromo-1-(2,5-dimethoxy-3,4-dimethylphenyl)decan-1-one

AlCl₃ (722 mg, 5.41 mmol, 296 uL, 0.90 eq) was added to a solution of 10-bromodecanoyl chloride (1.78 g, 6.62 mmol, 1.10 eq) and 1,4-dimethoxy-2,3-dimethylbenzene (1.00 g, 6.02 mmol, 1.14 mL, 1.00 eq) in DCE (7.00 mL). The obtained mixture was stirred at 25°C for 2 hours. TLC (petroleum ether:ethyl acetate = 5:1, Rf (start material) = 0.83, Rf (product) = 0.72) showed that the starting material was completely consumed. The reaction mixture was quenched with H₂O (10.0 mL), and the mixture was filtered. The reaction filtrate was extracted with DCM (20.0 mL × 3). The organic phase was separated, dried over Na₂SO₄, filtered, and concentrated under reduced pressure to obtain a residue. The residue was purified by column chromatography (SiO2, petroleum ether:ethyl acetate = 1:0 to 0:1). As a yellow solid, 10-bromo-1-(2,5-dimethoxy-3,4-dimethyl-phenyl)decan-1-one (500 mg, 1.25 mmol, 20.8% yield) was obtained, which was confirmed by HNMR (ET46959-6-P1A1) and LCMS (ET46985-6-P1A2, T = 1.011, M + H = 399.3).

1H NMR: (ET46959-6-P1A1, 400 MHz, DMSO-d6)

δ = 6.89 (s, 1H), 3.76 (s, 3H), 3.59 (s, 3H), 2.93 (t, J = 7.20 Hz, 2H), 2.16 (s, 3H), 2.11 (s, 3H), 1.39 - 1.24 (m, 16H)

### Step C. 1-(10-Bromodecyl)-2,5-dimethoxy-3,4-dimethylbenzene

Et₃SiH (3.64 g, 31.3 mmol, 5.00 mL, 25.0 eq) was added to a solution of 10-bromo-1-(2,5-dimethoxy-3,4-dimethylphenyl)decan-1-one (500 mg, 1.25 mmol, 1.00 eq) in TFA (5.00 mL). The obtained mixture was stirred at 80°C for 2 hours. TLC (petroleum ether:ethyl acetate = 5:1, Rf (start material) = 0.7, Rf (product) = 0.6) showed that the starting material was completely consumed. The mixture was poured into water (10.0 mL) and extracted with EtOAc (5.00 mL × 3). Then, the combined organic layer was washed with brine (10.0 mL). The residue was purified by column chromatography (SiO2, petroleum ether:ethyl acetate = 1:0 to 0:1). As a pale yellow oil, 1-(10-bromodecyl)-2,5-dimethoxy-3,4-dimethyl-benzene (0.20 g, 519 umol, 41.4% yield) was obtained, which was confirmed by HNMR (ET46959-8-P1A) and LCMS (ET46959-8-P1A, T = 1.055. M + H = 385.3)

1H NMR (ET46959-8-P1A, 400 MHz, DMSO-d6)

δ = 6.63 - 6.56 (m, 1H), 3.70 (s, 3H), 3.54 (s, 3H), 2.10 (s, 3H), 2.02 (s, 3H), 1.77 (q, J = 7.20 Hz, 2H), 1.59 - 1.48 (m, 2H), 1.41 - 1.21 (m, 14H))

### Step D. Formic acid and salt of 10-(2,5-dimethoxy-3,4-dimethyl-phenyl)decyl-triphenyl-phosphonium

PPh₃ (161 mg, 615 umol, 1.10 eq) was added to a solution of 4-(10-bromodecyl)-1,2-dimethoxy-benzene (200 mg, 559 umol, 1.00 eq) in toluene (3.50 mL). The obtained mixture was stirred at 130°C for 18 hours. TLC1 (dichloromethane:methanol = 5:1, Rf (product) = 0.4) and TLC2 (petroleum ether:ethyl acetate = 5:1, Rf (start material) = 0.8) showed that the starting material was completely consumed. The mixture was concentrated in vacuo. The residue was purified by prep-HPLC (column: Phenomene × Luna C18 75 × 30 mm × 3 um; mobile phase: [water (FA) - ACN]; B%: 40% to 75%, 8 minutes). Formic acid and salt of 10-(2,5-dimethoxy-3,4-dimethyl-phenyl) decyl-triphenyl-phosphonium (30.0 mg, 52.5 umol, 10.1% yield, 99.3% purity) were obtained as a yellow gum, which was confirmed by HNMR (ET46959-9-P1A), LCMS (ET46959-9-P1A, T = 2.938. M+ = 567.2), and HPLC (ET46959-9-P1B, T = 4.052, Purity = 99.3%).

1H NMR (ET46959-9-P1A, 400 MHz, DMSO-d6)

δ = 8.52 (s, 1H), 7.92 - 7.86 (m, 3H), 7.83 - 7.74 (m, 12H), 6.61 - 6.56 (m, 1H), 3.70 (s, 3H), 3.54 (s, 5H), 2.10 (s, 3H), 2.02 (s, 3H), 1.56 - 1.38 (m, 7H), 1.30 - 1.15 (m, 11H)

### 1-13. Preparation of (10-(3,4-dimethoxyphenyl) decyl) triphenylphosphonium bromide

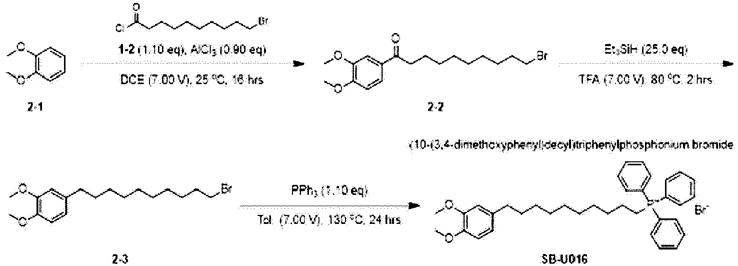

### Step 1: 10-Bromo-1-(3,4-dimethoxyphenyl)decan-1-one

AlCl₃ (4.34 g, 32.6 mmol, 1.78 mL, 0.90 eq) was added to a solution of 10-bromodecanoyl chloride (10.7 g, 39.8 mmol, 1.10 eq) and 1,2-dimethoxybenzene (5.00 g, 36.2 mmol, 4.63 mL, 1.00 eq) in DCE (35.0 mL). The obtained mixture was stirred at 25°C for 16 hours. The mixture was poured into ice water (50.0 mL) and extracted with DCM (50.0 mL). The organic phase was separated and concentrated in vacuo. The residue was purified by column chromatography (SiO2, petroleum ether:ethyl acetate = 1:0 to 1:1). As a white solid, 10-bromo-1-(3,4-dimethoxyphenyl)decan-1-one(3.00 g, 7.92 mmol, 21.8% yield, 98.0% purity) was obtained.

MS (ESI) : mass calcd. for C18H27BrO3, 370.11; m/z found, 371.2 [M + H]+.

1H NMR (400 MHz, CDCl3-d)

δ = 7.59 (dd, J = 8.32, 1.96 Hz, 1H), 7.54 (d, J = 1.96 Hz, 1H), 6.89 (d, J = 8.44 Hz, 1H), 3.95 (d, J = 3.32 Hz, 6H), 3.41 (t, J = 6.84 Hz, 2H), 2.92 (t, J = 7.40 Hz, 2H), 1.85 (quin, J = 7.16 Hz, 2H), 1.66 - 1.78 (m, 2H), 1.27 - 1.50 (m, 10H)

### Step 2: 4-(10-Bromodecyl)-1,2-dimethoxybenzene

Et₃SiH (15.7 g, 134 mmol, 21.5 mL, 25.0 eq) was added to a solution of 10-bromo-1-(3,4-dimethoxyphenyl)decan-1-one (2.00 g, 5.39 mmol, 1.00 eq) in TFA (15.0 mL). The obtained mixture was stirred at 80°C for 1 hour and concentrated in vacuo. The residue was purified by column chromatography (SiO2, petroleum It was purified as ether:ethyl acetate = 1:0 to 10:1). As a pale yellow oil, 4-(10-bromodecyl)-1,2-dimethoxy-benzene (1.00 g, 2.79 mmol, 51.7% yield, 99.5% purity) was obtained.

MS (ESI) : mass calcd. for C18H29BrO2, 356.14; m/z found, 357.2 [M + H]+.

### Step 3: (10-(3,4-Dimethoxyphenyl)decyl)triphenylphosphonium bromide

PPh₃ (161 mg, 615 umol, 1.10 eq) was added to a solution of 4-(10-bromodecyl)-1,2-dimethoxy-benzene (200 mg, 559 umol, 1.00 eq) in toluene (1.40 mL). The obtained mixture was stirred at 130°C for 12 hours and concentrated in vacuo. The resulting crude product was ground with MeCN (2.00 mL) at 20°C for 1 hour and then purified under reverse phase (neutral conditions). As a yellow gum, 10-(3,4-dimethoxyphenyl)decyl-triphenyl-phosphonium (50.0 mg, 92.3 umol, 16.4% yield, 99.6% purity) was obtained.

MS (ESI): mass calcd. for C₃₆H₄₄BrO₂P, 618.23; m/z found, 539.2 [M]+.

1H NMR (400 MHz, DMSO-d6)

δ = 7.85 - 7.95 (m, 3H), 7.73 - 7.84 (m, 12H), 6.82 (d, J = 8.16 Hz, 1H), 6.75 (d, J = 1.84 Hz, 1H), 6.66 (dd, J = 8.08, 1.83 Hz, 1H), 3.70 (d, J = 7.96 Hz, 6H), 3.48 - 3.63 (m, 2H), 2.47 (br s, 2H), 2.07 (s, 1H), 1.37 - 1.59 (m, 6H), 1.10 - 1.32 (m, 10H)

### 1-14. Preparation of 2,2,5,5-tetramethyl-3-(((10-(triphenylphosphonio)decyl)oxy)carbonyl)pyrrolidin-1-olate (Mito-CP) and 2,2,5,5-tetramethyl-3-((2-(triphenylphosphonio)ethoxy)carbonyl)pyrrolidine-1-olate (Mito-CP^{S})

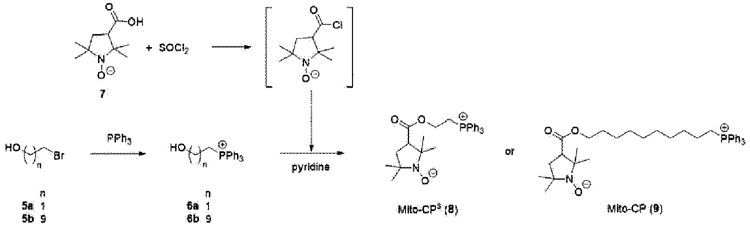

### (2-Hydroxyethyl)triphenylphosphonium (6A).

A mixture of 2-bromoethanol (1.5 mmol), PPh₃ (1.0 mmol), and acetonitrile (20 mL) was refluxed for 24 hours. After being cooled to room temperature, the solvent was removed by rotary evaporation. The obtained residue of a pale yellow oil was washed twice with ethyl ether to obtain phosphonium salt 6A. Yield: 77%; 1H NMR (400 MHz, CDCl3) δ 7.95 - 7.56 (m, 15H), 5.21 (s, 1H), 4.14 (d, J = 16.6 Hz, 2H), 3.83 (s, 2H).

### (10-Hydroxydecyl)triphenylphosphonium (6B).

The synthetic procedure of 6A was applied, along with 10-bromodecanol (1.5 mmol), to obtain a product as a brown oil. Yield: 85%; 1H NMR (400 MHz, CDCl3) δ 7.92 - 7.83 (m, 6H), 7.79 (dd, J = 7.7, 5.8 Hz, 3H), 7.70 (m, 6H), 3.91 - 3.81 (m, 2H), 3.63 (t, J = 6.6 Hz, 2H), 1.69 - 1.50 (m, 12H), 1.31 (m, 4H).

2,2,5,5-Tetramethyl-3-((2-(triphenylphosphonio)ethoxy)carbonyl)pyrrolidine-1-oleate (Mito-CPs; 8).

Pyridine (1.0 mmol) was added to a solution of 3-carboxy-2,2,5,5-tetramethylpyrrolidine-1-oleate (7, 1.0 mmol) in benzene. A flask was kept cool in an ice bath, followed by adding thionyl chloride (2.0 mmol) dropwise thereto for 1 hour. The solvent was removed by vacuum evaporation. The resulting residue and (2-hydroxyethyl)triphenylphosphonium (6A, 1.0 mmol) were dissolved in dichloromethane (10 mL). Pyridine (1.0 mmol) was added dropwise to the resulting solution under the ice bath and stirred at room temperature for 6 hours. The reaction mixture was quenched with a saturated aqueous NaHCO₃ solution and extracted with ethyl acetate (60 ml × 3). The combined organic layer was washed with brine, dried over Na₂SO₄, filtered, and evaporated in vacuo. The resulting crude product was purified by MPLC (MeOH 5% in DCM) to obtain a compound (21%); HRMS (ESI, m/z) calculated for C₂₉H₃₄NO₃P [M]+ 475.2276, found 475.2260.

2,2,5,5-Tetramethyl-3-(((10-(triphenylphosphonio)decyl)oxy)carbonyl)pyrrolidine-1-oleate (Mito-CP; 9).

The synthetic procedure of 8 was applied, along with (10-hydroxydecyl)triphenylphosphonium (6B, 1.0 mmol), to obtain 9 as a brown oil. Yield: 24%; HRMS (ESI, m/z) calculated for C₃₇H₅₀NO₃P [M]+ 587.3528, found 587.3524.

### 1-15. MitoQ^{S}: Preparation of ((4,5-dimethoxy-2-methyl-3,6-dioxocyclohexa-1,4-dien-1-yl)methyl)triphenylphosphonium bromide

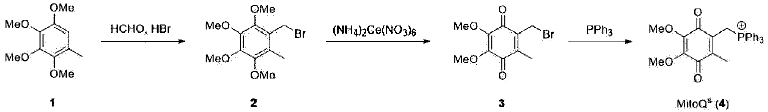

1-(Bromomethyl)-2,3,4,5-tetramethoxy-6-methylbenzene (2).

Paraformaldehyde (0.763 g, 25.4 mmol) and 1,2,3,4-tetramethoxy-5-methylbenzene (2.721 g, 12.7 mmol) were dissolved in 47% HBr (10 mL). The obtained mixture was then stirred at 40°C for 2 hours and left at room temperature. After completion of the reaction, the resulting product was extracted with hexane (40 mL), and the combined organic layer was dried over sodium sulfate. The residual solvent was removed in vacuo to prepare light yellow oil 2 (yield: 88%); 1H NMR (400 MHz, CDCl3) δ 4.61 (s, 2H), 3.95 (s, 3H), 3.93 (s, 3H), 3.89 (s, 3H), 3.79 (s, 3H), 2.27 (s, 3H); 13C NMR (100 MHz, CDCl3) δ 148.5, 148.0, 147.8, 144.7, 126.6, 124.9, 61.3, 61.1, 61.1, 60.8, 26.6, 11.1.

2-(Bromomethyl)-5,6-dimethoxy-3-methylcyclohexa-2,5-diene-1,4-dione (3).

Intermediate product 2 (211 mg, 0.687 mmol) was dissolved in THF (10 mL). Subsequently, ammonium cerium (IV) nitrate (1.5 g, 2.74 mmol) dissolved in water (10 mL) was added to the reaction mixture. The obtained mixture was stirred at room temperature for 2 hours. After completion of the reaction, the resulting mixture was extracted with DCM (20 mL), and the extract was washed with brine until it became neutral. The combined organic layer was then dried over sodium sulfate, and the residual solvent was removed in vacuo. The residue was purified by silica gel chromatography to obtain yellow oil 3 (yield: 45%); 1H NMR (400 MHz, CDCl3) δ 5.39 (s, 2H), 4.03 (s, 3H), 4.02 (s, 3H), 2.17 (s, 3H); 13C NMR (100 MHz, CDCl3) δ 183.9, 181.6, 145.0, 144.5, 141.7, 137.6, 61.4, 61.3, 21.5, 12.0; MS (ESI, m/z) calculated for C₁₀H₁₂BrO₄ [M+H]+ 274.99, found 275.00.

((4,5-Dimethoxy-2-methyl-3,6-dioxocyclohexa-1,4-dien-1-yl)methyl)triphenylphosphonium (MitoQs; 4).

Triphenylphosphine (57.1 mg, 0.218 mmol) and 3 (22.31 mg, 0.0726 mmol) were dissolved in ACN (10 mL), and the reaction mixture was refluxed overnight. After being cooled to room temperature, the residual solvent was removed in vacuo. The residue was purified by silica gel chromatography to obtain white solid 4 (yield: 18%); 1H NMR (400 MHz, CD3OD) δ 7.88-7.81 (m, 3H), 7.70-7.64 (m, 6H), 7.62-7.55 (m, 6H), 3.80 (s, 3H), 3.52 (s, 3H), 3.34 (s, 2H), 1.81 (d, J = 1.5 Hz, 3H); 13C NMR (101 MHz, MeOD) δ 182.74 (d, J = 3.3 Hz), 182.65 (d, J = 2.3 Hz), 145.80, 145.70, 145.21, 143.96, 135.27 (d, J = 3.0 Hz), 134.30 (d, J = 10.1 Hz), 130.37 (d, J = 12.8 Hz), 118.14 (d, J = 85.9 Hz), 61.31, 61.26, 24.81 (d, J = 49.8 Hz), 14.68 (d, J = 2.7 Hz); HRMS (ESI, m/z) calculated for C₂₈H₂₆O₄P [M]+ 457.1563, found 457.1566.

### 1-16. Mito-VitE: Preparation of 2-(6-hydroxy-2,5,7,8-tetramethylchroman-2-yl)ethyl)triphenylphosphonium

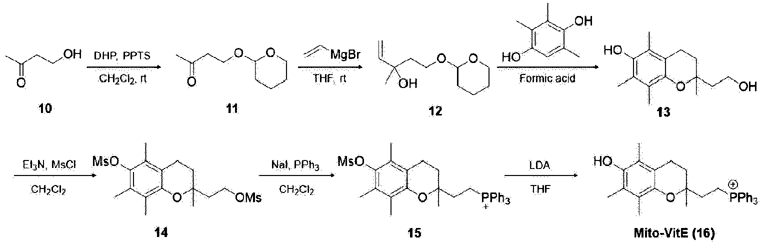

### 4-(Tetrahydro-2H-pyran-2-yl)butan-2-one (11).

Pyridinium p-toluenesulfonate (1.135 mmol, 0.1 eq) was added to a solution of 3,4-dihydro-2H-pyran (15.89 mmol) and 4-hydroxybutan-2-one (11.35 mmol) in CH₂Cl₂ (15 mL) and stirred at room temperature for 4 hours. Subsequently, the reaction mixture was concentrated in vacuo, and the residue was dissolved in Et2O (50 mL). The resulting product was washed with saturated aqueous NaCl (40 mL) and H₂O (10 mL), dried over anhydrous MgSO₄, filtered, and concentrated in vacuo to obtain Cpd.11. Yield: 64%; 1H NMR (400 MHz, CDCl3) δ 4.60 (t, J = 3.4 Hz, 1H), 4.00 (dt, J = 10.2, 6.2 Hz, 1H), 3.89 - 3.80 (m, 1H), 3.69 (dt, J = 10.1, 6.3 Hz, 1H), 3.55 - 3.45 (m, 1H), 2.71 (t, J = 6.2 Hz, 2H), 2.20 (s, 3H), 1.83 - 1.64 (m, 3H), 1.60 - 1.54 (m, 3H).

### 3-Methyl-5-((tetrahydro-2H-pyran-2-yl)oxy)pent-1-en-3-ol (12) .

Vinylmagnesium bromide (13.35 mmol) was added to a solution of 11 (5.81 mmol) in THF (25 mL) at -78°C. The resulting product was stirred for 2 hours, heated to room temperature for 30 minutes, followed by adding saturated aqueous NH₄Cl (50 mL) dropwise thereto, and extracted with Et₂O. The combined organic phase was washed with saturated aqueous NaCl, dried over anhydrous MgSO₄, filtered, and concentrated to obtain a pale yellow oil. The resulting crude product was purified by MPLC. Yield: 93%; 1H NMR (400 MHz, CDCl3) δ 5.90 (ddd, J = 16.7, 10.7, 5.8 Hz, 1H), 5.38 - 5.27 (m, 2H), 5.10 (ddd, J = 10.3, 8.7, 1.4 Hz, 1H), 4.67 - 4.53 (m, 1H), 3.97 (dt, J = 9.7, 4.2 Hz, 1H), 3.83 (td, J = 11.5, 5.6 Hz, 1H), 3.59 - 3.45 (m, 3H), 1.97 (m, 1H), 1.80 - 1.72 (m, 2H), 1.59 - 1.49 (m, 3H), 1.29 (t, J = 5.0 Hz, 3H).

### 2-(2-Hydroxyethyl)-2,5,7,8-tetramethylchroman-6-ol (13).

A solution of 12 (5.39 mmol) and freshly prepared 2,3,5-trimethylbenzene-1,4-diol (4.49 mmol) in formic acid (10 mL) was refluxed under a nitrogen environment for 3.5 hours. The reaction mixture was poured into crushed ice, and the organic material was extracted with Et₂O. The combined organic phase was washed with H₂O, dried over anhydrous MgSO₄, and concentrated in vacuo. The brown oily residue was dissolved in MeOH and concentrated HCl and then refluxed under argon for 30 minutes. After removing the solvent in vacuo, the residue was dissolved in Et₂O, washed with H₂O, saturated aqueous NaHCO₂, and H₂O under argon again, dried over anhydrous MgSO₄, filtered, and concentrated to obtain a brown oil. The resulting crude product was purified by MPLC to obtain a compound. Yield: 35%; 1H NMR (400 MHz, CDCl3) δ 5.30 (s, 1H), 4.23 (s, 1H), 3.97 - 3.86 (m, 2H), 2.66 (dd, J = 9.8, 6.2 Hz, 2H), 2.17 (s, 3H), 2.12 (s, 3H), 2.09 (s, 3H), 1.97 (m, 2H), 1.93 - 1.82 (m, 2H), 1.29 (s, 3H).

### 2,5,7,8-Tetramethyl-2-(2-((methylsulfonyl)oxy)ethyl)chroman-6-ylmethanesulfonate (14).

Methanesulfonyl chloride (1.055 mmol) was added to a solution of 13 (0.479 mmol) and triethylamine (2.88 mmol) in CH₂Cl₂ (2 mL) and then stirred at room temperature for 1 hour. The reaction mixture was diluted with CH₂Cl₂ (20 mL), washed with H₂O, dried over anhydrous MgSO₄, filtered, and concentrated in vacuo to obtain a white solid. The obtained solid was recrystallized from EtOH to obtain a product. Yield: 43%; 1H NMR (400 MHz, CDCl3) δ 4.56 - 4.45 (m, 2H), 4.41 (dd, J = 14.8, 8.7 Hz, 2H), 3.25 (s, 3H), 3.00 (s, 3H), 2.63 (t, J = 7.0 Hz, 2H), 2.23 (d, J = 13.2 Hz, 6H), 2.09 (s, 3H), 1.86 (t, J = 6.8 Hz, 2H), 1.31 (s, 3H).

### Triphenyl(2-(2,5,7,8-tetramethyl-6-((methylsulfonyl)oxy)chroman-2-yl)ethyl)phosphonium (15).

A mixture of 14 (0.256 mmol), sodium iodide (192 mg, 1.279 mmol), and triphenylphosphine (1.279 mmol) was flushed with argon in a Kimax tube and then sealed. Then, the reaction proceeded at 90°C for 48 hours to be stirred into a molten material. The resulting crude product was dissolved in CH₂Cl₂ and precipitated three times from petroleum ether. The resulting product was dissolved in methanol and passed through an anion exchange column loaded with -Oms. The residual solvent was removed in vacuo to obtain pure product 15. Yield: 39%; 1H NMR (400 MHz, CDCl3) δ 7.87 - 7.72 (m, 9H), 7.66 (td, J = 7.9, 3.4 Hz, 6H), 4.12 (m, 2H), 3.28 (s, 3H), 2.61 - 2.49 (m, 2H), 2.25 (s, 3H), 2.15 (s, 3H), 2.05 (s, 2H), 2.03 (s, 3H), 2.00 (d, J = 6.5 Hz, 2H), 1.49 (s, 3H).

### (2-(6-Hydroxy-2,5,7,8-tetramethylchroman-2-yl)ethyl)triphenylphosphonium (Mito-VitE; 16).

Lithium diisopropylamide (0.119 mmol, 1 M solution in THF) was added to a solution of 15 (0.100 mmol) in THF (2 mL) at 0°C. After 30 minutes, the solution was heated to room temperature, followed by adding aqueous saturated NH₄OMs (10 mL). The aqueous layer was extracted three times with CH₂Cl₂. Then, the organic phase was combined, dried over anhydrous MgSO₄, filtered, and concentrated in vacuo. The residue was purified by MPLC to obtain product 16. Yield: 9%; 1H NMR (400 MHz, CDCl3) δ 7.85 - 7.73 (m, 4H), 7.71 - 7.58 (m, 11H), 3.77 - 3.64 (m, 2H), 3.41 - 3.28 (m, 2H), 2.17 (s, 3H), 2.07 (d, J = 11.7 Hz, 6H), 1.94 (m, 4H), 1.34 (d, J = 7.6 Hz, 3H); HRMS (ESI, m/z) calculated for C₃₃H₃₆O₂P [M]+ 495.2447, found 495.2445.

### 1-17. Mito-VitE^{L}: Preparation of (10-(6-hydroxy-2,5,7,8-tetramethylchroman-2-yl)decyl)triphenylphosphonium iodide

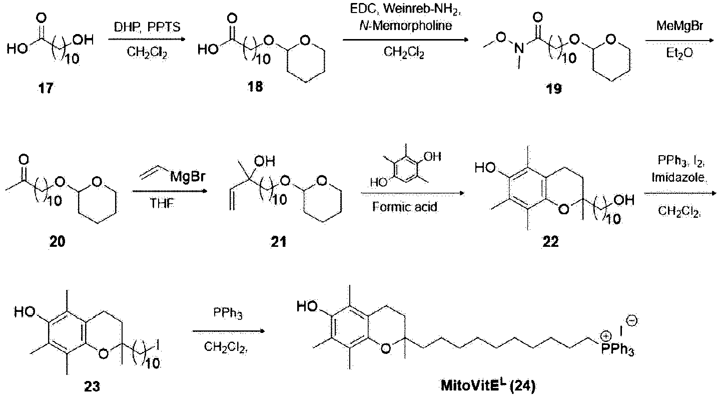

### 11-((Tetrahydro-2H-pyran-2-yl)oxy)undecylic acid (18).

A solution of pyridinium p-toluenesulfonate (1.135 mmol), 3,4-dihydro-2H-pyran (15.89 mmol), and 11-hydroxyundecylic acid (17, 11.35 mmol) in CH₂Cl₂ (15 mL) was stirred at room temperature for 4 hours. The reaction mixture was concentrated in vacuo. Then, the residue was dissolved in Et2O (50 mL), washed with saturated aqueous NaCl (40 mL) and H₂O (10 mL), dried over anhydrous MgSO₄, filtered, and concentrated in vacuo to obtain 18. Yield: 46%; 1H NMR (400 MHz, CDCl3) δ 4.63 - 4.48 (m, 1H), 3.88 (ddd, J = 11.1, 7.6, 3.3 Hz, 1H), 3.75 - 3.64 (m, 1H), 3.55 - 3.48 (m, 1H), 3.38 (dt, J = 9.6, 6.7 Hz, 1H), 2.34 (t, J = 7.5 Hz, 2H), 1.91 - 1.44 (m, 14H), 1.32 (d, J = 24.9 Hz, 8H).

### N-methoxy-N-methyl-11-((tetrahydro-2H-pyran-2-yl)oxy)undecanamide (19).

N,O-dimethylhydroxylamine hydrochloride (5.75 mmol) and N-methylmorpholine (5.75 mmol) were added continuously to a solution of 18 (5.23 mmol) in CH₂Cl₂ (10 mL) at -15°C. After 10 minutes, N-(3-dimethylaminopropyl)-N'-decylcarbodiimide hydrochloride (1.62 g, 5.75 mmol) was added thereto several times for 15 minutes and stirred at -15°C for 3 hours. The reaction was quenched by adding 1 M HCl (5 mL), and the organic compound was extracted with CH₂Cl₂ (3 × 50 mL). The organic extract was combined, washed with a saturated NaHCO₃ solution (50 mL), dried over MgSO₄, filtered, and evaporated to obtain the desired amide 19 (yield: 80%), which was used in the next step without being additionally purified. 1H NMR (400 MHz, CDCl3) δ 4.60 (dd, J = 20.5, 17.9 Hz, 1H), 3.87 (ddd, J = 11.1, 7.4, 3.5 Hz, 1H), 3.79 - 3.65 (m, 4H), 3.50 (dt, J = 5.1, 4.5 Hz, 1H), 3.38 (dt, J = 9.6, 6.7 Hz, 1H), 3.18 (s, 3H), 2.41 (t, J = 7.6 Hz, 2H), 1.90 - 1.78 (m, 1H), 1.76 - 1.47 (m, 14H), 1.29 (s, 8H).

### 12-((Tetrahydro-2H-pyran-2-yl)oxy)dodecane-2-one (20).

MeMgI (10.45 mmol) was added to a solution of 19 (4.18 mmol) in Et₂O (13 mL) at 0°C and stirred at the same temperature for 3 hours. Then, a saturated aqueous NH₄Cl solution (30 mL) was added to the reaction mixture. The organic layer was separated, and the aqueous layer was extracted with tBuOMe (3 × 30 mL). The combined organic layer was dried over Na₂SO₄ and concentrated under reduced pressure. The residue was purified by flash chromatography to obtain Cpd.20. Yield: 80%; 1H NMR (400 MHz, CDCl3) δ 4.61 - 4.54 (m, 1H), 3.87 (ddd, J = 11.1, 7.4, 3.4 Hz, 1H), 3.77 - 3.66 (m, 1H), 3.55 - 3.46 (m, 1H), 3.38 (dt, J = 9.6, 6.7 Hz, 1H), 2.41 (t, J = 7.5 Hz, 2H), 2.13 (s, 3H), 1.90 - 1.76 (m, 1H), 1.72 (ddd, J = 11.9, 6.0, 3.2 Hz, 1H), 1.63 - 1.47 (m, 14H), 1.37 - 1.20 (m, 6H).

### 3-Methyl-13-((tetrahydro-2H-pyran-2-yl)oxy)tridec-1-en-3-ol (21).

Vinylmagnesium bromide (8.3 mmol, 2.5 eq) was added to a solution of 20 (3.32 mmol) in THF (15 mL) at -78°C. The resulting solution was stirred for 2 hours and then heated to room temperature for 30 minutes. Saturated aqueous NH₄Cl (50 mL) was added dropwise thereto, and the organic compound was extracted with Et₂O. The combined organic layer was washed with saturated aqueous NaCl, dried over anhydrous MgSO₄, filtered, concentrated, and purified by MPLC to obtain Cpd.21. Yield: 93%; 1H NMR (400 MHz, CDCl3) δ 5.91 (dd, J = 17.4, 10.8 Hz, 1H), 5.20 (dd, J = 17.4, 1.2 Hz, 1H), 5.04 (dd, J = 10.8, 1.2 Hz, 1H), 4.64 - 4.51 (m, 2H), 3.87 (ddd, J = 11.0, 7.4, 3.3 Hz, 2H), 3.77 - 3.65 (m, 2H), 3.56 - 3.46 (m, 2H), 3.38 (dt, J = 9.5, 6.7 Hz, 2H), 1.90 - 1.77 (m, 2H), 1.71 (tt, J = 16.3, 7.0 Hz, 2H), 1.64 - 1.46 (m, 10H), 1.27 (s, 8H).

### 2-(10-Hydroxydecyl)-2,5,7,8-tetramethylchroman-6-ol (22).

A solution of freshly prepared 2,3,5-trimethylbenzene-1,4-diol (2.56 mmol) and 21 (3.08 mmol) in formic acid (10 mL) was refluxed for 3.5 hours under a nitrogen environment. The reaction mixture was poured into crushed ice, and the organic compound was extracted with Et₂O under argon. The combined organic phase was washed with H₂O, dried over anhydrous MgSO₄, and concentrated in vacuo. The obtained brown residue was dissolved in MeOH and concentrated HCl, and the reaction mixture was additionally refluxed for 30 minutes under argon. The solvent was removed in vacuo. Then, the residue was dissolved in Et₂O, washed with H₂O, saturated aqueous NaHCO₃, and H₂O under argon again, dried over anhydrous MgSO₄, filtered, and concentrated to obtain a brown oil. The obtained oil was purified by MPLC to obtain Cpd.22. Yield: 35%; 1H NMR (400 MHz, CDCl3) δ 4.38 (s, 1H), 3.62 (t, J = 6.6 Hz, 2H), 2.59 (t, J = 6.8 Hz, 2H), 2.15 (s, 3H), 2.10 (s, 6H), 1.76 (qq, J = 13.9, 7.1 Hz, 2H), 1.64 - 1.24 (m, 17H), 1.22 (s, 3H).

### 2-(10-Iododecyl)-2,5,7,8-tetramethylchroman-6-ol (23).

A solution of 22 (0.165 mmol) in CH₂Cl₂ (2 mL) was added to a solution of 1H-imidazole (0.348 mmol), iodine (0.182 mmol), and triphenylphosphine (0.348 mmol) in CH₂Cl₂ (2 mL) through a syringe at 0°C. The reaction mixture was additionally stirred at room temperature for 12 hours. The obtained mixture was washed with Na₂SO₃, H₂O, and a brine solution, dried over Na₂SO₄, filtered, and concentrated under reduced pressure. The resulting residue was purified by MPLC to obtain Cpd.23. 1H NMR (400 MHz, CDCl3) δ 4.19 (d, J = 2.7 Hz, 1H), 3.19 (t, J = 4.0 Hz, 2H), 2.60 (t, J = 6.8 Hz, 2H), 2.16 (m, 3H), 2.11 (s, 6H), 1.90 - 1.69 (m, 5H), 1.63 - 1.47 (m, 4H), 1.44 - 1.21 (m, 10H), 0.99 - 0.95 (m, 1H), 0.86-0.83 (m, 3H).

### (10-(6-Hydroxy-2,5,7,8-tetramethylchroman-2-yl)decyl)triphenylphosphonium iodide (Mito-VitE; 24).

A mixture of 23 (0.25 mmol) and triphenylphosphine (327 mg, 1.25 mmol) was flushed with argon in a Kimax tube, sealed, and stirred as a molten material at 90°C for 48 hours. The resulting crude product was dissolved in CH₂Cl₂ and precipitated three times from petroleum ether. The residual solvent was removed in vacuo to obtain 24. Yield: 12%; 1H NMR (400 MHz, CDCl3) δ 7.86 - 7.79 (m, 9H), 7.73 - 7.68 (m, 6H), 3.76-3.71 (m, 2H), 2.59 (t, J = 6.8 Hz, 2H), 2.15 (s, 3H), 2.10 (s, 3H), 2.09 (s, 3H), 1.81-1.72 (m, 2H), 1.63-1.62 (m, 4H), 1.37-1.36 (m, 2H), 1.21-1.18 (m, 15H); HRMS (ESI, m/z) calculated for C41H52O2P [M]+ 607.3699, found 607.2697.

### 1-18. SB-U141: Preparation of triphenyl(8-(2,3,4,5-tetramethoxy-6-methylphenyl) octyl)phosphonium

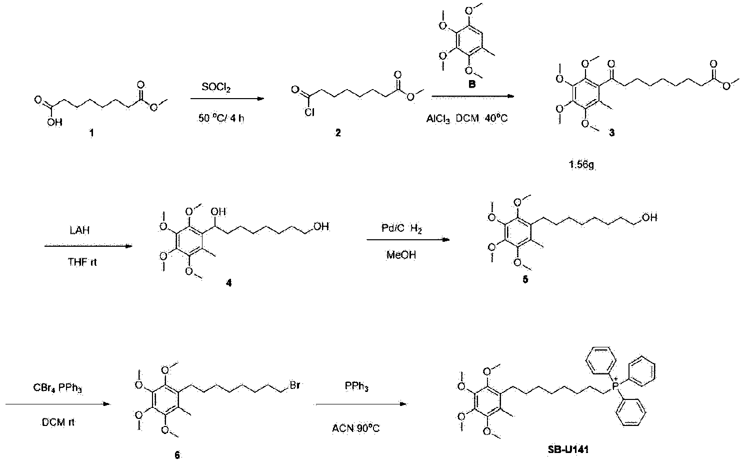

Methyl-8-chloro-8-oxootanoate (2) was obtained by reacting 8-methoxy-8-oxooctanoic acid (1) with SOCl₂ at 50°C for 4 hours. Methyl 9-oxo-9-(2,3,4,5-tetramethoxy-6-methylphenyl)nonanoate (3) was synthesized by reacting 2 and 1,2,3,4-tetramethoxy-5-methylbenzene with AlCl₃ and dichloromethane (DCM) at 40°C. Then, 1-(2,3,4,5-tetramethoxy-6-methylphenyl)octane-1,8-diol (4) was synthesized by reacting 3 with LAH in THF at room temperature. Next, 8-(2,3,4,5-tetramethoxy-6-methylphenyl)octan-1-ol (5) was synthesized by reacting 4 with Pd/C and H2 in MeOH. Subsequently, 1-(8-bromooctyl)-2,3,4,5-tetramethoxy-6-methylbenzene (6) was obtained by reacting 5 with CBr₄PPh₃ in DCM. Triphenyl(8-(2,3,4,5-tetramethoxy-6-methylphenyl)octyl)phosphonium (SB-U141) was obtained by reacting 6 with PPh₃ in ACN at 90°C.

### 1-19. SB-U142: Preparation of triphenyl(12-(2,3,4,5-tetramethoxy-6-methylphenyl) dodecyl)phosphonium

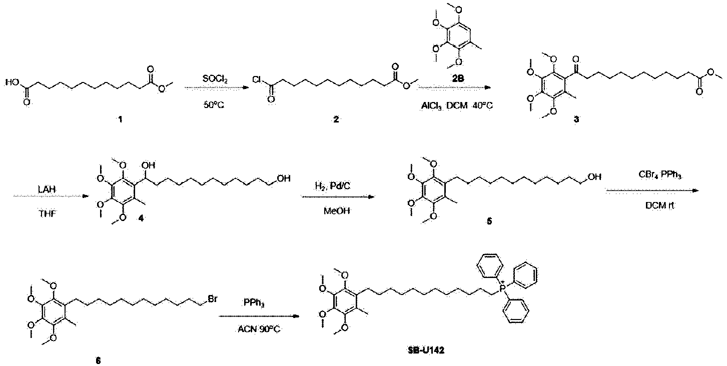

Methyl 12-chloro-12-oxododecanoate (2) was synthesized by reacting 12-methoxy-12-oxododecanoic acid (1) with SOCl₂ at 50°C for 4 hours. Methyl 12-oxo-12-(2,3,4,5-tetramethoxy-6-methylphenyl)dodecanoate (3) was obtained by reacting 2 with 1,2,3,4-tetramethoxy-5-methylbenzene and AlCl₃ in DCM at 40°C. Then, 1-(2,3,4,5-tetramethoxy-6-methylphenyl)dodecane-1,12-diol (4) was obtained by reacting 3 with LAH in THF. Next, 12-(2,3,4,5-tetramethoxy-6-methylphenyl)dodecane-1-ol (5) was obtained by reacting 4 with H₂ and Pd/C in MeOH. Subsequently, 1-(12-bromododecyl)-2,3,4,5-tetramethoxy-6-methylbenzene (6) was synthesized by reacting 5 with CBr₄ and PPh₃ in DCM at room temperature. Triphenyl(12-(2,3,4,5-tetramethoxy-6-methylphenyl)dodecyl)phosphonium (SB-U142) was synthesized by reacting 6 with PPh₃ in ACN at 90°C.

### 1-20. SB-U151: Preparation of (8-(4,5-dimethoxy-2-methylphenyl)octyl)triphenylphosphonium

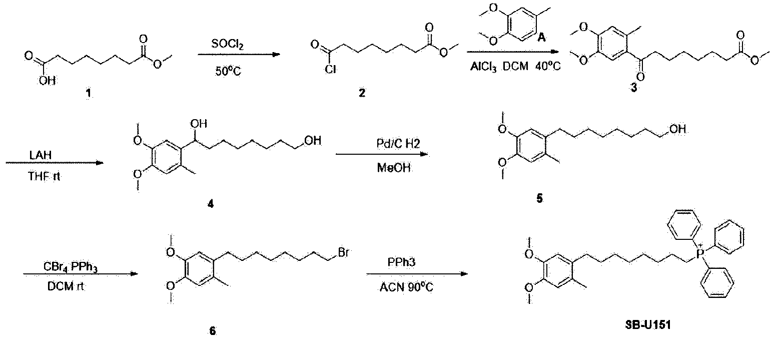

Methyl 8-chloro-8-oxooctanoate (2) was obtained by reacting 8-methoxy-8-oxooctanoic acid with SOCl₂ at 50°C. Methyl 8-(4,5-dimethoxy-2-methylphenyl)-8-oxooctanoate (3) was obtained by reacting 2 with AlCl₃ in DCM at 40°C. Then, 1-(4,5-dimethoxy-2-methylphenyl)octane-1,8-diol (4) was obtained by reacting 3 with LAH in THF at room temperature. Next, 8-(4,5-dimethoxy-2-methylphenyl)octan-1-ol (5) was obtained by reacting 4 with Pd/C and H₂ in MeOH. Subsequently, 1-(8-bromooctyl)-4,5-dimethoxy-2-methylbenzene (6) was obtained by reacting 5 with CBr₄ and PPh₃ in DCM at room temperature. Lastly, (8-(4,5-dimethoxy-2-methylphenyl)octyl)triphenylphosphonium (SB-U151) was obtained by reacting 6 with PPh₃ in ACN at 90°C.

### 1-21. SB-U152: Preparation of (12-(4,5-dimethoxy-2-methylphenyl)dodecyl)triphenylphosphonium

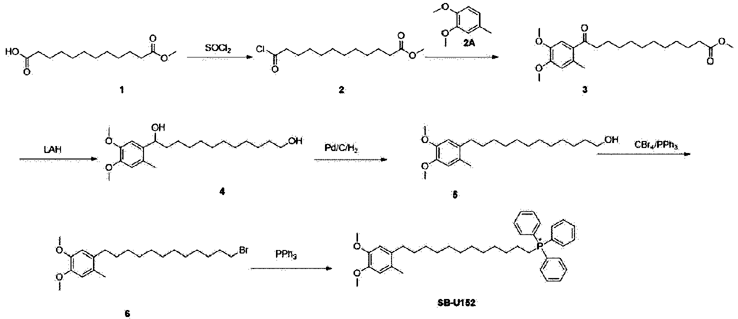

Methyl 12-chloro-12-oxododecanoate (2) was synthesized by reacting 12-methoxy-12-oxododecanoic acid with SOCl₂ at 50°C for 4 hours. Methyl 12-(4,5-dimethoxy-2-methylphenyl)-12-oxododecanoate (3) was synthesized by reacting 2 with 1,2-dimethoxy-4-methylbenzene. Then, 1-(4,5-dimethoxy-2-methylphenyl)dodecane-1,12-diol (4) was synthesized by reacting 3 with LAH. Next, 12-(4,5-dimethoxy-2-methylphenyl)dodecane-1-ol (5) was synthesized by reacting 4 with Pd/C and H₂. Subsequently, 1-(12-bromododecyl)-4,5-dimethoxy-2-methylbenzene (6) was synthesized by reacting 5 with CBr₄ and PPh₃. Lastly, (12-(4,5-dimethoxy-2-methylphenyl)dodecyl)triphenylphosphonium (SB-U152) was synthesized by reacting 6 with PPh₃.

### Example 2. Preparation of recombinant protein

The genes encoding zTRAP1 and hTRAP1 were cloned into a modified pET-Duet vector with an N-terminal hexahistidine tag followed by a TEV protease cleavage site and then expressed in *E. coli* BL21 (DE3) cells. Fifteen hours after induction with 0.4 mM IPTG at 20°C, cells were obtained and lysed by ultrasonication. The soluble fraction of the lysate was applied to a Ni2+ affinity chromatography column (GE Healthcare). The hexahistidine tag was cleaved by TEV protease, and the protein was additionally purified by gel-filtration chromatography in a buffer solution containing 25 mM Tris-HCl pH 7.5, 150 mM NaCl, and 5 pmM beta-mercaptoethanol (beta-ME).

### Example 3. Structural analysis

The inventors of the present disclosure analyzed the binding structure of TRAP1 and MitoQ to derive the structure of the compound that binds to TRAP1.

Purified zTPAP1 was mixed with AMPPNP at a molar ratio of 1:1.5. Crystallization was performed as described in Lavery et al., 2014. After the crystals grew, 0.1 mM MitoQ was added to the crystallization drops and incubated for 24 hours. For X-ray diffraction experiments, the crystals were transferred to a well solution containing 20% glycerol and then quickly frozen in liquid nitrogen. Diffraction data were collected at beamline 5C of Pohang Accelerator Laboratory (PAL) and processed using the HKL-2000 software (Otwinowski and Minor, 1997). The electron density of MitoQ was calculated using the difference Fourier method. Model building and refinement were performed by the Coot and Phenix programs (Adams et al., 2010; Emsley et al., 2010), respectively.

As a result of structural analysis, the distance between the two protomers of TRAP1 was about 25 Å, confirming that binding to CBS of TRAP1 was enabled when the distance between the Ub moiety and the TPP moiety was appropriate. Based on the result, a compound structure with an appropriate length was confirmed to be essential in binding to TRAP1 (see FIGS. 1 and 2) .

### Example 4. Binding capacity analysis

### Fluorescence polarization (FP) analysis

### Example 4-1. Using SB-TM2 probe

The substance to be analyzed (at a final volume of 100 µL) was added to FP buffer (35 mM NaCl, 2.7 mM KCl, 4.3 mM Na2HPO4, 1.4 mM KH2PO4 (pH 7.3), 1 mM DTT, 2 mM MgCl2, and 0.1 mg/mL BSA) containing human full-length TRAP1 protein (400 nM) and SB-TM2 probe (100 nM) and then reacted at room temperature for 1 hour (96-well plate). Using a SYNERGY NEO microplate reader, FP was measured at an excitation wavelength set to 440 nm and an emission wavelength set to 500 nm.

As a result, competitive binding with SB-TM2 was confirmed to be enabled starting from TPP-8. Additionally, when the alkyl chain had a length equal to or greater than the size of C8, binding to the CBS of TRAP1 was confirmed to be enabled. Furthermore, it was confirmed that the longer the alkyl chain, the stronger the binding capacity. Moreover, compared to the binding capacity of SMX (MitoQ), the binding capacity of TPP-12, TPP-14, and TPP-16 was confirmed to be superior to that of SMX (MitoQ). In particular, the binding capacity of TPP-16, having the longest alkyl chain, was confirmed to be twice as strong as that of SMX (see FIG. 4).

Additionally, among the antioxidants bound to TPP, when the connection distance between TPP and the antioxidant was sufficient, binding to the CBS of TRAP1 was confirmed to be enabled (see FIG. 6).

Additionally, it was confirmed that other synthetic compounds linked through TPP and hydrocarbons had binding capacity to the CBS of TRAP1 (see FIG. 8).

### Example 4-2. Using PU-H71-FITC

For FP analysis, purified recombinant TRAP1 (400 nM) was incubated with PU-H71-FITC (10 nM), a fluorescent probe synthesized as described by Taldone et al., 2013 in the presence of an inhibitor at increasing concentrations for 2 hours. Fluorescence polarization was measured using a microplate reader (Synergy NEO, BioTek) at room temperature.

As a result, it was confirmed that alkyl-TPPs did not compete with PU-H71-FITC (an Hsp90/TRAP1 inhibitor targeting the ATP-binding site) and did not bind to the ATP-binding site (see FIG. 9B).

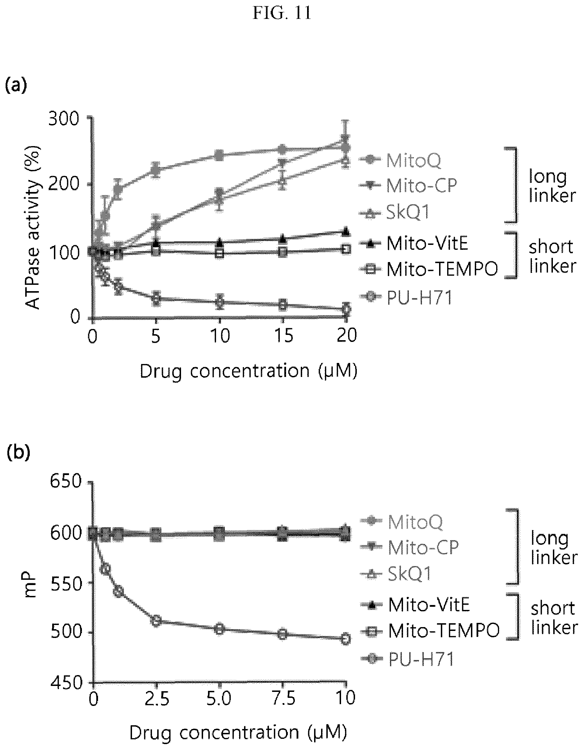

Additionally, it was confirmed that the TPP-antioxidant conjugates with a long linker also did not compete with PU-H71-FITC (Hsp90/TRAP1 inhibitor targeting the ATP-binding site) and did not bind to the ATP-binding site (see FIG. 11B).

### Example 5. ATPase activity assay

The inventors of the present disclosure performed an assay to determine whether the compounds disclosed herein bound to the ATP-binding site in TRAP1 and affected ATPase activity.

The ATPase activity of TRAP1 was measured using the PiColorLock Gold Phosphate Detection Kit (Abcam). Then, 0.5 µM TRAP1 (wild-type or mutation) was pre-incubated along with inhibitors at various concentrations for 30 minutes and then incubated with 0.2 mM ATP at 37°C for 3 hours in ATPase activity assay buffer containing 50 mM Tris-HCl, 20 mM KCl, and 6 mM MgCl (pH 7.4). Next, 20 uL of a mixture of a PiColorLock Gold reagent and an accelerator (100:1) was added to each sample (100 µL). After 5 minutes of incubation, 10 µL of a stabilizer was added to stop color development. Absorbance was measured at 620 nm using a microplate reader (Synergy NEO, BioTek). The background signal was normalized by subtracting the absorbance of unreacted samples.

As a result, unlike PU-H71, where the ATPase activity decreased with the increasing concentration, alkyl-TPPs did not show a tendency that the ATPase activity decreased in a concentration-dependent manner (see FIG. 9A). This means that the alkyl-TPPs act in a different manner from PU-H71, which binds to the ATP-binding site and inhibits ATPase activity.

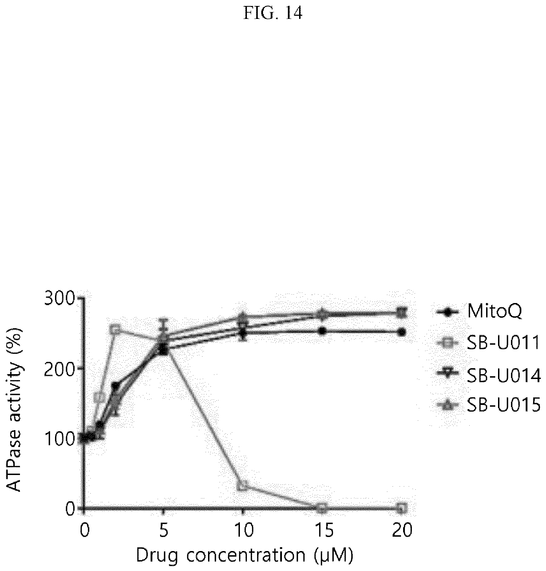

Additionally, TPP-antioxidant conjugates and other synthetic compounds (SB-U011, U014, and U015) did not show a tendency that the ATPase activity decreased in a concentration-dependent manner (see FIGS. 11A and 14).

### Example 6. Protein expression analysis

The inventors of the present disclosure performed protein expression analysis to determine whether the compounds disclosed herein inhibited TRAP1 or cytoplasmic Hsp90.

### Cell incubation and treatment

Human cancer cell line 22Rv1 was purchased from the American Type Culture Collection (ATCC) and maintained as recommended by the supplier. Briefly, cancer cells were incubated in a DMEM or RPMI medium (GIBCO) containing 10% fetal bovine serum (FBS; ATCC) and 1% penicillin/streptomycin (GIBCO) at 37°C in a humidified atmosphere of 5% CO2. The cells were not incubated for more than 6 months.

The 22Rv1 cells were treated with 5 µM of each compound, cultured for 2 hours, and then analyzed by western blot.

### Antibody

Anti-Phospho-AMPKα, anti-Cdk4, anti-CHOP, and anti-SIRT3 antibodies were purchased from Cell Signaling Technology. Anti-Akt and anti-AMPK were purchased from Santa Cruz Biotechnology. Anti-Hsp70 was purchased from BD Biosciences. Anti-β-actin antibodies were purchased from MP Biomedicals. Anti-SDHB was purchased from Abcam.

### Western blot

Cell lysates were separated by SDS-PAGE and transferred to a PVDF membrane. The membrane was blocked with 10% skim milk in TBST (TBS containing 0.05% Tween-20) for 1 hour at room temperature and incubated with primary antibodies overnight at 4°C. The membrane was washed with TBST three times for 1 hour and incubated with secondary antibodies (1:5000) diluted in 10% skim milk in TBST for 1 hour. The membrane was washed with TBST three times and visualized using an enhanced chemiluminescence detection kit (BioRad).

As a result of the experiment, TPP-10 to 16 were confirmed to inhibit TRAP1, thereby reducing SDHB and SIRT3, and among these, TPP-14 and 16 were confirmed to have strong effects. However, none of the alkyl-TPPs caused changes in the expression of Akt and Cdk4 proteins, or Hsp70, used as a marker of Hsp90 inhibition (see FIG. 10). This confirms that alkyl-TPPs with a predetermined length inhibit TRAP1 and do not affect cytoplasmic Hsp90

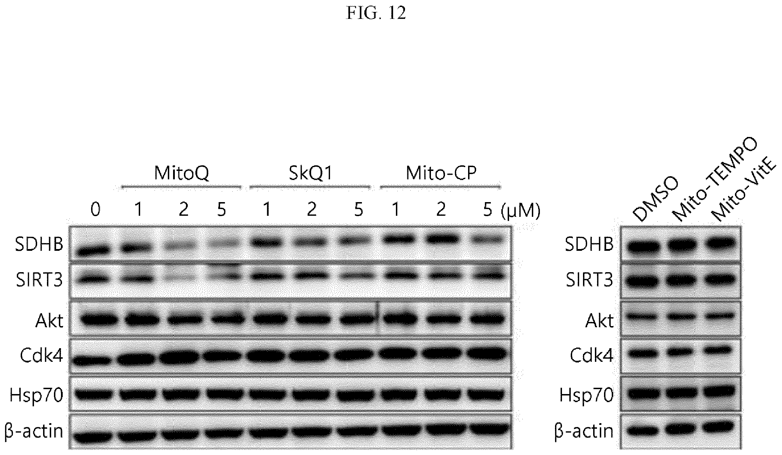

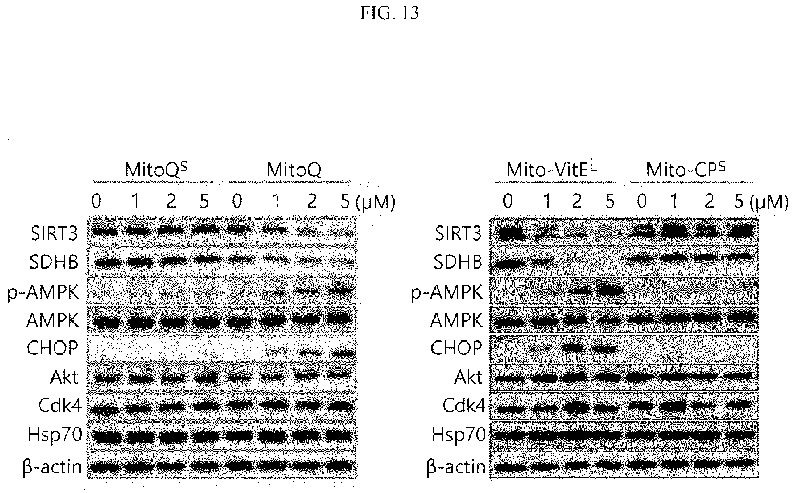

Additionally, in the case of TPP-antioxidant conjugates, the conjugates with a long linker exhibited decreases in SDHB and SIRT3 according to TRAP1 inhibition without Hsp90 inhibition, and the conjugates with a short linker did not affect protein expression (see FIG. 12). This confirms that only the TPP-conjugates with an appropriate length inhibit TRAP1. Additionally, unlike compounds with short linkers, it is confirmed that in the case of involving linkers with an appropriate length, AMPK (p-AMPK), a marker of TRAP1 inhibition, is activated, and the mitochondrial unfolded protein response was induced (CHOP) (FIG. 13).

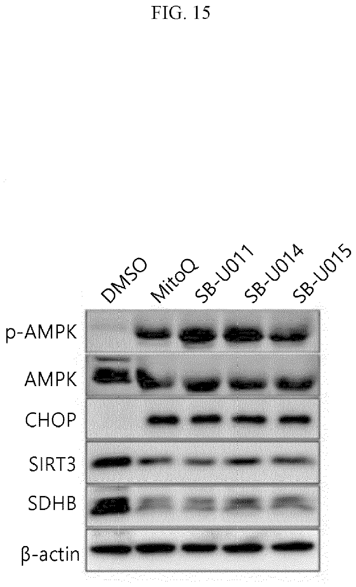

Furthermore, it was confirmed through the western blot results that other synthetic compounds also inhibited TRAP1 (see FIG. 15).

### Example 7. In vivo experiment to confirm effect of compound on cancer

### In vivo mouse xenograft

Immunodeficient athymic nude mice (male, 8 weeks old) were purchased from OrientBio. The mice were maintained in a pathogen-free facility (12-hour dark/light cycle) at the UNIST In Vivo Research Center and supplied with standard diet and water. All animal experiments were approved by UNIST (UNISTIACUC-19-11) . 22Rv1 cells (1 × 10⁷) were injected subcutaneously into both sides of the nude mice. When the tumor size reached approximately 100 mm³, 3 mg/Kg drugs (MitoQ, SB-U014, and SB-U015) and vehicle (DMSO) dissolved in 20% cremophor EL (Sigma) in PBS were administered intraperitoneally daily. The tumor volume was measured daily using an electronic caliper and calculated using the following equation: V = 1/2 X (width)2 X length. When the experiment was completed, animals were euthanized, and the tumor was collected for histology and western blot. Band intensities were quantified using ImageJ software (National Institute of Health, USA). In this case, western blot was performed in the same manner as described above.

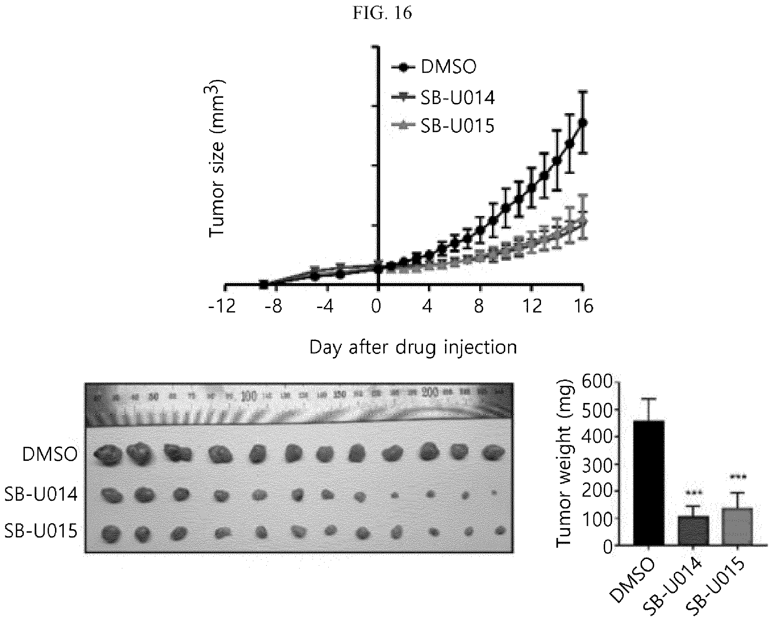

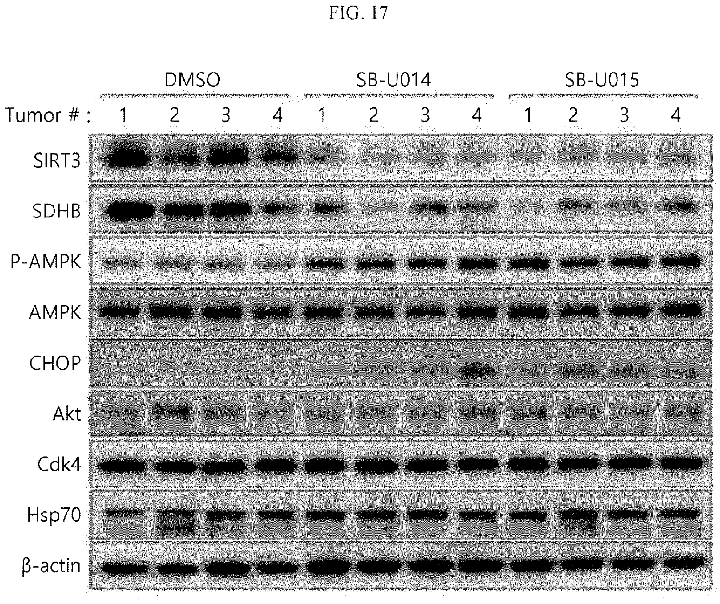

As a result of the experiment, it was confirmed that when treated with SB-U014 and U015, the tumor size became smaller and the tumor weight decreased, compared to when treated with DMSO (see FIG. 16). Additionally, the western blot results showed that TRAP1 inhibition lead to decreases in SDHB and SIRT3, activated AMPK (p-AMPK), and induced the mitochondrial unfolded protein response (CHOP) without causing changes in Akt, Cdk4, and Hsp70 (see FIG. 17).

Through this, it was confirmed that the compounds disclosed herein inhibited cancer cell growth and reduced the size of cancer cells through TRAP1 inhibition.

### Example 8. In vivo experiment to confirm effect of compound on ocular diseases

8-1. Oxygen-induced retinopathy (OIR) induction experiment in TRAP1 knockout mice.

As TRAP1+/+ (wild-type), TRAP1+/- (hetero), and TRAP1-/- (knockout) experimental mice, Black6 J strain mice were used. On the 7th day after birth, a baby mouse and a mother mouse were placed in an in vivo chamber (Coy-lab) and supplied with high oxygen (75% O₂). On the 12th day after birth, the baby mouse was taken out of the in vivo chamber. Then, a surrogate mother was put thereinto, and normal oxygen (21% O₂) was supplied. Then, the eyeball of the baby mouse was removed on the 17th day after birth and used for experiments.

The experiment was performed on the TRAP1 knockout mice by breeding hetero-female mice with male mice and comparing a litter of baby mice. Genotyping was performed on the day of eye enucleation.

### 8-2. Eye drop treatment

In the process of forming the in vivo oxygen-induced retinopathy (OIR) model, the baby mouse was treated with eye drops for 6 days, from the day when being taken out to normal oxygen after being supplied with high oxygen until the day their eyes were collected (on the 12th day to 17th day after birth). Treatment was performed three times a day at intervals of 4 hours. As a drug treatment method, Mito Q was diluted to a concentration of 1 mM in Liposic and dropped 10 uL into the eyes of the baby mouse. Then, the eyes were allowed to blink for 1 minute. After wiping the residual amount outside the eyeball with a cotton swab, the mouse was returned to the care of the mother mouse.

### 8-3. Intravitreal injection of drugs

In the process of forming an in vivo oxygen-induced retinopathy (OIR) model, baby mice were treated with intravitreal injection on the day when being taken out to normal oxygen (the 12th day after birth) after being supplied with high oxygen. The baby mice were anesthetized through intraperitoneal injection of an anesthetic (2.5% Avertin, 1X phosphate-buffered saline (PBS) as a solvent). Then, the eyeball of the mouse was poked with a pin holder to make a hole. The drug was injected into the hole at a volume of 1 uL at 0.1 uL/sec using a fine glass tube. MitoQ was diluted in tertiary distilled water (0.1% DMSO + 99.9% tertiary distilled water) to a concentration of 0.1 mg/mL and used.

### 8-4. Whole-mount staining of tissue and microscope image observation

After forming the in vivo oxygen-induced retinopathy model, the eyes were collected on the 17th day after birth. As a negative control, the eyes of a mouse raised in normal oxygen were collected on the 17th day after birth. The baby mouse was anesthetized through intraperitoneal injection of an anesthetic (2.5% Avertin, 1X phosphate-buffered saline (PBS) as a solvent). Then, 10 mL of 1X PBS was injected into the left ventricle to remove all blood. The eyeball was removed and fixed in 4% paraformalin at 4°C for 24 hours. The retina was separated and then cut into a four-pronged radial form. After performing blocking for 1 hour at room temperature (1× PBS + 0.5% BSA + 0.1% Tryton-X-100), primary antibodies (CD31, 1:100) were treated at 4°C for 24 hours. On the next day, a cleaning solution (1X PBS + 0.1% Tryton X-100) was used for washing for 20 minutes four times. Then, secondary antibodies (Alexa fluor-594, 1:500, Invitrogen) were diluted in the blocking solution and then treated at 4°C for 24 hours. On the next day, a cleaning solution (1X PBS + 0.1% Tryton X-100) was used for washing for 20 minutes five times. Then, a mounting solution (vector lab, H-1700) was used to mount the tissue on a slide.

Images were obtained using a stereo fluorescence microscope (Axion xoom, Zeiss) to observe the entire tissue image. Then, magnified images of specific sections were observed using a confocal scanning microscope (multi-photon confocal microscope, LSM 780. Zeiss).

### 8-5. In vitro streptozotocin (STZ)-induced diabetic retinopathy model

The experiment was performed using the TRAP1 wild-type, hetero, and knockout mice. Streptozotocin (Sigma) was diluted in a solution of 0.1 M sodium citrate (pH 5.0) and injected intraperitoneally into 8-week-old male mice to a concentration of 75 mg/kg once a day for 5 days. After one week, blood sugar was measured, and whether diabetes occurred was confirmed by measuring the blood sugar over 350 mg/dL. From 8 weeks after STZ injection until 16 weeks, baby food was provided by adding feed to water three times a week. The eyeball was removed at 16 weeks of age after STZ injection and analyzed.

### 8-6. Immunofluorescence staining

The removed eyeball was fixed in 4% paraformalin under conditions at 4°C for 24 hours. After tissue treatment, a paraffin block was made to cut the tissue to a thickness of 10 um, and the tissue was attached to a slide to prepare tissue fragments.

Paraffin was dissolved with xylan, and the xylan was removed with 100%, 80%, 70%, and 50% ethanol. After supplying moisture with tertiary distilled water, the slide was placed in a solution of 10 mM sodium citrate (pH 6.0) and heated in a pressure cooker for 10 minutes to restore constant temperature. After treated with a solution of 1× PBS + 1% Tryton X-100 at room temperature for 1 hour to increase permeability, blocking (1× PBS + 5% BSA + 5% FBS + 0.3% Tryton-X-100) was performed at room temperature for 1 hour. After being treated with primary antibodies (TRAP1, Thermo, 1:100/ Glutamine synthase, Millipore, 1:100/ GFAP, Abcam, 1:50/ HIF1α, Novus, 1:20) at 4°C for 24 hours, a cleaning solution (1X PBS + 0.3% Tryton X-100) was used three times for five minutes on the next day for washing. After being treated with secondary antibodies (Alelx fluor-488, 546, 633, 1:500) at room temperature for 1 hour and washed, DAPI (Thremo, 300 nM) was used to stain the nuclei. The slide was fixed with a mounting solution.

Images were observed using a confocal scanning microscope (Multi-photon confocal microscopy, LSM 780, Zeiss).

### 8-7. Western blot analysis

The mouse eyeball was removed, and then the retina was separated. Whole-cell lysates were prepared using RIPA lysis buffer to perform electrophoresis. After being transferred to a PVDF membrane, blocking (10% skim milk powder) was performed at room temperature for 1 hour. Then, primary antibodies (TRAP1, Abcam/ HIF1 ex, Novus/ VEGF-A, Abcam/ GFAP, Millipore/ β-actin, Millipore, all diluted to 1:500) were treated at 4°C for 18 hours. On the next day, secondary antibodies (anti-mouse or rabbit-HRP. KPL.) were treated at room temperature for 1 hour, and protein expression was analyzed using a western blotting detection reagent (Bio-rad).

For quantitative analysis, the area fraction was calculated using Image J and normalized using β-actin.

### 8-8. Real-time polymerase chain reaction (real-time PCR)

After removing the mouse eyeball, the RNA layer was separated using Trizol and chloroform. Then, RNA was extracted using an RNA extraction kit (Qiagen) . Next, cDNA was synthesized using a cDNA synthesis kit (NEB), and quantitative analysis of cDNA was performed by real-time polymerase chain reaction using SYBR real-time polymerase chain reaction master mix (Enzynomics). The amplification reaction was performed using LightCycler (Roche) equipment. The analysis was performed using comparative CT analysis. Normalization was performed using β-actin. Then, compared to the average expression levels of target genes (TRAP1, VEGF-A, and ANGPTL4) in the control group, an increase or decrease in the experimental group was analyzed.

### 8-9. Experiment results

Referring to FIGS. 18 and 19, TRAP1 expression is confirmed to be increased in both the oxygen-induced retinopathy model and the streptozotocin (STZ)-induced diabetic retinopathy model. Additionally, in these models, increases in HIF1α, a marker of hypoxia, and VEGF-A, a downstream angiogenic factor, are confirmed, along with increases in TRAP1. Furthermore, referring to the staining results, TRAP1 is confirmed to be co-located with the staining of glutamine synthase (GS), a marker of Müller cells that is responsible for the production of various angiogenic factors, during the progression of retinopathy diseases.

Referring to FIG. 20, neovascular and avascular areas are confirmed to be reduced in the case of the OIR model where TRAP1 is knocked out.

Additionally, referring to the HIF1α staining results of the retina in FIG. 21, HIF1α is confirmed to be reduced when TRAP1 is knocked out in the STZ or OIR model.

Referring to the expression results of VEGF-A and ANGPTL4 in FIG. 22A, the mRNA levels of VEGF-A and ANGPTL4 were elevated in STZ TRAP1+/+ but not elevated in STZ-TRAP1-/-, compared to those in Con TRAP1+/+. Furthermore, referring to FIG. 22B, the mRNA levels of VEGF-A and ANGPTL4 were decreased in TRAP1+/- and TRAP1-/- compared to TRAP1+/+ in the retina of the OIR model.

Lastly, referring to the results in FIGS. 23 and 24, it is confirmed that treatment with MitoQ significantly reduces both avascular and neovascular areas in the retina of the OIR model.

### Example 9. Drug activity assay using MIO-M1 HRE cell line

After transfection (jetPRIME kit) of MIO-M1 Müller cells with 5HRE/GFP plasmid (Addgene, #46926), the cells transfected with the plasmid were selected using 1 mg/mL of G418 (Neomycin), a selectable marker. A stable cell line was constructed by selecting cells, growing in a colony form, from single cells. The prepared MIO-M1-HRE/GFP stable cell line was dispensed into a 96-well plate and treated with drugs at different concentrations the next day. After being exposed to a hypoxic environment (1% O₂) for 24 hours, the GFP (Ex/Em: 488/507) fluorescence signal was measured using a SYNERGY NEO microplate reader (BioTek Instrument). The relative fluorescence value was calculated on the basis of 100% of the negative control group.

As a result, referring to FIGS. 25, 26, and 27, all alkyl-TPPs, TPP-antioxidant conjugates (when containing a linker with appropriate length), and other synthetic compounds, confirmed to inhibit TRAP1, were confirmed to have HIF1α inhibitory activity.

## Claims

1. A compound represented by following Formula 1 or a pharmaceutically acceptable salt thereof: 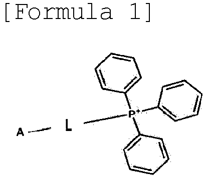 Wherein,
L comprises (CH₂)ₙ;
n is an integer of 7 or more and 40 or less; and
A is selected from methyl, unsubstituted or substituted aryl, unsubstituted or substituted cycloalkyl, and unsubstituted or substituted heterocyclyl.

2. The compound or the pharmaceutically acceptable salt thereof of claim 1, wherein A is selected from aryl, cycloalkyl, and heterocyclyl that are unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

3. The compound or the pharmaceutically acceptable salt thereof of claim 2, wherein A is unsubstituted or substituted aryl, and
wherein the unsubstituted or substituted aryl is phenyl unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

4. The compound or the pharmaceutically acceptable salt thereof of claim 3, wherein A is selected from the group consisting of

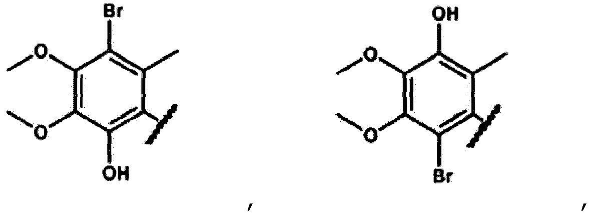

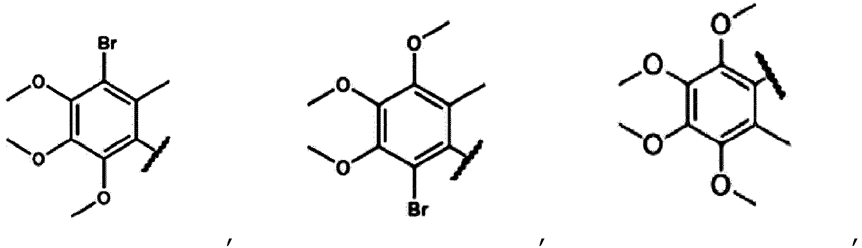

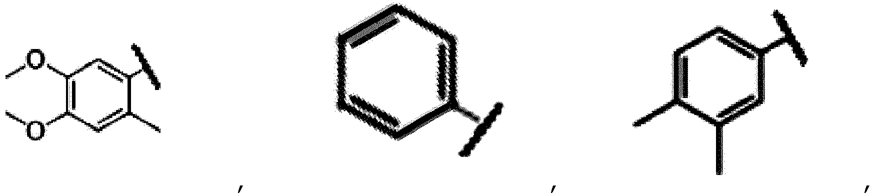

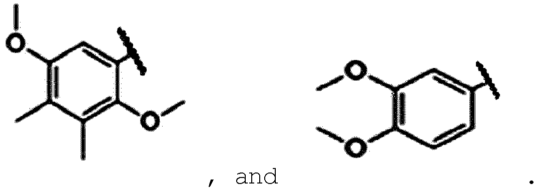

5. The compound or the pharmaceutically acceptable salt thereof of claim 2, wherein A is unsubstituted or substituted aryl, and
wherein the unsubstituted or substituted aryl is naphthalene unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

6. The compound or the pharmaceutically acceptable salt thereof of claim 5, wherein A is

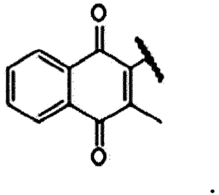

7. The compound or the pharmaceutically acceptable salt thereof of claim 2, wherein A is unsubstituted or substituted aryl, and
wherein the unsubstituted or substituted aryl is benzodioxole unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

8. The compound or the pharmaceutically acceptable salt thereof of claim 7, wherein A is 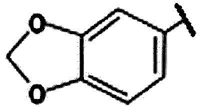 or

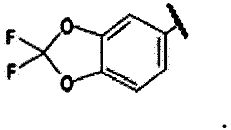

9. The compound or the pharmaceutically acceptable salt thereof of claim 2, wherein A is unsubstituted or substituted cycloalkyl, and
wherein the unsubstituted or substituted cycloalkyl is cyclohexyl unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

10. The compound or the pharmaceutically acceptable salt thereof of claim 9, wherein A is unsubstituted cyclohexyl.

11. The compound or the pharmaceutically acceptable salt thereof of claim 2, wherein A is unsubstituted or substituted heterocyclyl, and
wherein the unsubstituted or substituted heterocyclyl is pyrrolidine or chromane unsubstituted or substituted with one or more selected from halogen, =O, hydroxyl, oxycarbonyl, hydroxy C1-5 alkyl, C1-5 alkyl, C1-5 alkenyl, C1-5 alkynyl, and C1-5 alkoxy.

12. The compound or the pharmaceutically acceptable salt thereof of claim 11, wherein A is pyrrolidine substituted with one or more selected from oxycarbonyl, C1-5 alkyl, and =O.

13. The compound or the pharmaceutically acceptable salt thereof of claim 12, wherein A is

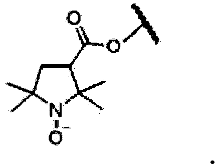

14. The compound or the pharmaceutically acceptable salt thereof of claim 11, wherein A is chroman-2-yl substituted with one or more selected from C1-5 alkyl and hydroxyl.

15. The compound or the pharmaceutically acceptable salt thereof of claim 14, wherein A is

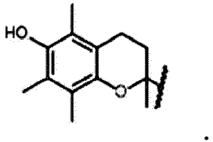

16. The compound or the pharmaceutically acceptable salt thereof of claim 1, wherein n is an integer of 9 or more.

17. The compound or the pharmaceutically acceptable salt thereof of claim 1, wherein the compound binds to TRAP1 and competes with SB-TM2.

18. A TRAP1 inhibitor comprising the compound or the pharmaceutically acceptable salt thereof of claim 1.

19. The inhibitor of claim 18, wherein the TRAP1 inhibitor binds to a CBS.

20. A TRAP1-client protein binding inhibitor comprising the compound or the pharmaceutically acceptable salt thereof of claim 1.

21. A pharmaceutical composition for treating cancer comprising the compound or the pharmaceutically acceptable salt thereof of claim 1.

22. A pharmaceutical composition for treating ocular disease comprising the compound or the pharmaceutically acceptable salt thereof of claim 1.

23. The pharmaceutical composition of claim 22, wherein the pharmaceutical composition is administered orally or administered topically using an eye drop.

24. A method of treating cancer or ocular disease, the method comprising administering the compound or the pharmaceutically acceptable salt thereof of claim 1 to a subject in need thereof.
